(19) Europäisches Patentamt
European Patent Office
Office européen des brevets

(11) Veröffentlichungsnummer: 0 059 683
A2

(12) # EUROPÄISCHE PATENTANMELDUNG

(21) Anmeldenummer: 82810079.2

(22) Anmeldetag: 19.02.82

(51) Int. Cl.[3]: C 07 D 501/20
C 07 D 501/57, C 07 D 501/59
A 61 K 31/545, C 07 D 277/48
C 07 D 285/08

(30) Priorität: 27.02.81 CH 1335/81
18.06.81 CH 4036/81
21.01.82 CH 368/82

(43) Veröffentlichungstag der Anmeldung:
08.09.82 Patentblatt 82/36

(84) Benannte Vertragsstaaten:
AT BE CH DE FR IT LI LU NL SE

(71) Anmelder: CIBA-GEIGY AG
Patentabteilung Postfach
CH-4002 Basel(CH)

(72) Erfinder: Wehrli, Hansuli, Dr.
Binningerstrasse 41
CH-4153 Reinach(CH)

(72) Erfinder: Wiederkehr, René, Dr.
Grenzweg 9
CH-4148 Pfeffingen(CH)

(72) Erfinder: Kocsis, Karoly, Dr.
Gartenstrasse 63
CH-4052 Basel(CH)

(54) **7-Acylamido-3-cephem-4-carbonsäureverbindungen, Verfahren zu ihrer Herstellung, pharmazeutische Präparate, welche diese Verbindungen enthalten, und Verwendung von letzteren.**

(57) 7β-Acylamido-3-cephem-4-carbonsäureverbindungen der Formel

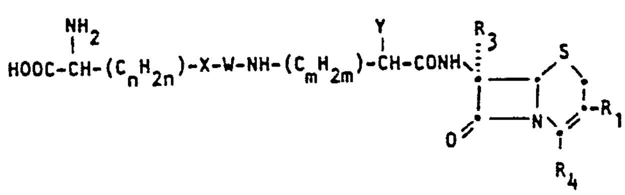

worin insbesondere m 0 (direkte Bindung) oder 3 bedeutet und die Gruppe $-(C_nH_{2n})-$ Methylen oder Aethylen, X Sauerstoff, W die Gruppe –CO– oder X–W zusammen die Gruppe –CO–, Y Wasserstoff oder einen unsubstituierten oder substituierten organischen Rest, z.B. Niederalkyl, Hydroxyniederalkyl, Cycloalkenyl, Phenyl oder Heterocyclyl, $R_1$ Wasserstoff, Niederalkyl, Niederalkoxy, Halogen oder eine Gruppe der Formel $-CH_2-R_2$, worin $R_2$ eine veresterte Hydroxy- oder Mercaptogruppe, z.B. Niederalkanoyloxy oder Heterocyclylthio bedeutet und $R_3$ Wasserstoff oder Methoxy darstellen, Hydrate und Salze von diesen Verbindungen besitzen antibiotische Eigenschaften und sind gegen grampositive und gramnegative Mikroorganismen wirksam. Die neuen Verbindungen können z.B. in Form von antibiotisch wirksamen Präparaten zur Behandlung von Infektionen verwendet werden. Die neuen Verbindungen werden in an sich bekannter Weise hergestellt. Ebenfalls umfasst sind Zwischenprodukte.

EP 0 059 683 A2

CIBA-GEIGY AG
Basel (Schweiz)

4-13310/1-3

# 7-Acylamido-3-cephem-4-carbonsäureverbindungen, Verfahren zu ihrer Herstellung, pharmazeutische Präparate, welche diese Verbindungen enthalten, und Verwendung von letzteren.

Die vorliegende Erfindung betrifft neue 7β-Acylamido-3-cephem-4-carbonsäure-Verbindungen, Verfahren zu ihrer Herstellung, pharmazeutische Präparate, welche solche Verbindungen enthalten, und ihre Verwendung zur Herstellung von pharmazeutischen Präparaten oder als pharmakologisch wirksame Verbindungen.

Die vorliegende Erfindung betrifft 7β-Acylamido-3-cephem-4-carbonsäure-Verbindungen der Formel

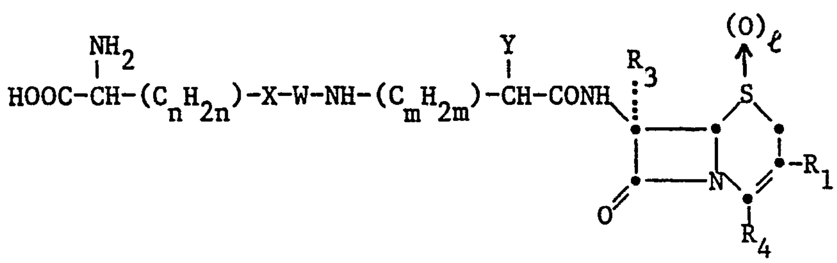

(I),

worin

$\ell$ eine ganze Zahl von 0 bis 2,

m eine ganze Zahl von 0 (direkte Bindung) bis 4 ,

n eine ganze Zahl von 1 bis 4,

X Sauerstoff, Schwefel oder die Gruppe -NH-,

W eine Gruppe -CO-, $-CONHSO_2-$ oder $-SO_2NHCO-$ oder X und W zusammen eine Gruppe -CO- oder $-CONHSO_2-$,

Y Wasserstoff oder einen unsubstituierten oder substituierten organischen Rest,

$R_1$ Wasserstoff, Niederalkyl, Niederalkoxy, Halogen oder eine Gruppe der Formel $-CH_2-R_2$, worin

$R_2$ eine freie, veresterte oder verätherte Hydroxyl- oder Mercaptogruppe oder eine quaternäre Ammoniumgruppe darstellt,

$R_3$ Wasserstoff oder Niederalkoxy und

$R_4$ Carboxyl oder geschütztes Carboxyl bedeuten,

Hydrate und Salze von Verbindungen der Formel I, Verfahren zur Herstellung von Verbindungen der Formel I, pharmazeutische Präparate, welche Verbindungen der Formel I enthalten und die Verwendung von Verbindungen der Formel I zur Herstellung von pharmazeutischen Präparaten oder als pharmakologisch wirksame Verbindungen.

In der Beschreibung der vorliegenden Erfindung bedeutet der im Zusammenhang mit Gruppen, z.B. Niederalkyl, Niederalkylen, Niederalkoxy, Niederalkanoyl etc., verwendete Ausdruck "Nieder", dass die entsprechenden Gruppen, falls nicht ausdrücklich anders definiert, bis zu 7 und bevorzugt bis zu 4 Kohlenstoffatome enthalten.

In der obigen Formel I bedeutet der Index $\ell$ in erster Linie 0. Falls $\ell$ den Wert 1 hat, liegt die entsprechende 1-Oxidverbindung der Formel I in der $\alpha$- oder $\beta$-Stellung oder im Gemisch von Verbindungen mit beiden Stellungen vor.

Wenn der Index m 0 bedeutet, liegt eine direkt Bindung vor. Wenn m eine ganze Zahl von 1 bis 4 bedeutet, stellt die Gruppe $-(C_mH_{2m})-$ eine unverzweigte oder verzweigte Alkylenkette dar und bedeutet beispielsweise Methylen, 1,2-Aethylen, 1,3-Propylen oder 1,4-Butylen.

Eine Gruppe-$(C_nH_{2n})$- ist eine unverzweigte oder verzweigte Alkylenkette und ist beispielsweise Methylen, 1,2-Aethylen, 1,3-Propylen oder 1,4-Butylen, ferner 1,1-Aethylen, 1,1-Propylen, 1,2-Propylen, 1,1-Butylen oder 1,1-Isobutylen.

Ein unsubstituierter oder substituierter organischer Rest Y hat bis zu 18 Kohlenstoffatome und ist ein unsubstituierter oder durch eine oder mehrere funktionelle Gruppen substituierter niederaliphatischer Kohlenwasserstoffrest oder ist ein cycloaliphatischer, cycloaliphatisch-aliphatischer, aromatischer, aromatisch-aliphatischer Kohlenwasserstoffrest oder ist ein unsubstituierter oder substituierter heterocyclischer oder heterocyclisch-aliphatischer Rest.

Ein niederaliphatischer Kohlenwasserstoffrest Y ist beispielsweise Niederalkyl mit 1-7, bevorzugt 1-4, Kohlenstoffatomen, z.B. Methyl, Aethyl, Propyl oder Butyl, oder ist Niederalkenyl mit 2-5 Kohlenstoffatomen, z.B. Vinyl oder Allyl.

Ein durch ein oder mehrere funktionelle Gruppen substituierter niederaliphatischer Rest Y ist beispielsweise durch Hydroxy, veräthertes Hydroxyl, beispielsweise Niederalkoxy, z.B. Methoxy oder Aethoxy, durch verestertes Hydroxyl, beispielsweise Niederalkanoyloxy, z.B. Acetyloxy, durch Halogen, z.B. Chlor, Carboxyl, verestertes Carboxyl, beispielsweise Niederalkoxycarbonyl, z.B. Methoxycarbonyl, durch Sulfo, amidiertes Sulfo, Amino, Mono- oder Dialkylamino, z.B. Methylamino oder Aethylamino, oder durch Acylamino, beispielsweise Niederalkanoylamino, z.B. Acetylamino, substituiertes Niederalkyl, z.B. Hydroxymethyl, 2-Hydroxyäthyl, Methoxymethyl, 2-Acetoxyäthyl, 2-Chloräthyl, Carboxymethyl, 2-Carboxyäthyl, Aethoxycarbonylmethyl, 2-Aethoxycarbonyläthyl, Sulfomethyl, 2-Sulfoäthyl, Sulfamoylmethyl, 2-Sulfamoyläthyl, 2-Aminoäthyl, 2-Dimethylaminoäthyl oder 2-Acetylaminoäthyl.

Ein cycloaliphatischer Kohlenwasserstoffrest Y ist beispielsweise Cycloalkyl mit 3-8, insbesondere 3-6, Kohlenstoffatomen, z.B. Cyclopropyl, Cyclopentyl oder Cyclohexenyl, oder ist Cycloalkenyl mit 3-8, insbesondere 3-6, Kohlenstoffatomen, z.B. 1-Cyclohexenyl oder 1,4-Cyclohexadienyl.

Ein cycloaliphatisch-aliphatischer Kohlenwasserstoffrest Y ist beispielsweise einer der genannten aliphatischen Reste, der durch einen der genannten cycloaliphatischen Reste substituiert ist, z.B. Cyclopropylmethyl oder -äthyl oder Cyclohexylmethyl oder -äthyl.

Ein aromatischer Kohlenwasserstoffrest Y ist beispielsweise Phenyl.

Ein aromatisch-aliphatischer Kohlenwasserstoffrest Y ist beispielsweise einer der genannten aliphatischen Reste, der beispielsweise durch Phenyl substituiert ist, z.B. Benzyl oder Phenäthyl.

Ein heterocyclischer Rest Y ist beispielsweise ein monocyclischer aza-, thia-, oxa-, thiaza-, thiadiaza-, diaza- oder tetraazacyclischer Rest aromatischen Charakters.

Ein heterocyclischer Rest Y ist in erster Linie ein aromatischer monoaza-, monothia- oder monooxacyclischer Rest, beispielsweise Pyridinium, Thienyl, z.B. 2- oder 3-Thienyl, oder Furyl, z.B. 2- oder 3-Furyl, ein monocyclischer thiaza-, thiadiaza-, diaza- oder tetraazacyclischer Rest, z.B. Thiazolyl, z.B. 2-Thiazolyl, Isothiazolyl, z.B. 2- oder 4-Isothiazolyl, Thiadiazolyl, z.B. 1,2,4-Thiadiazol-3-yl oder 1,3,4-Thiadiazol-2-yl, Imidazolyl, z.B. 2-Imidazolyl, oder Tetrazolyl, z.B. 1- oder 5-Tetrazolyl.

Substituenten des genannten heterocyclischen Restes Y sind beispielsweise Niederalkyl, insbesondere Methyl, sowie Aethyl, n-Propyl, Isopropyl oder geradkettiges oder verzweigtes Butyl, oder Niederalkyl, welches durch Hydroxy, verestertes Hydroxy, z.B. Niederalkanoyloxy, Halogen, z.B. Chlor, in Salzform vorliegendes Phosphono, welches gegebenenfalls durch ein oder zwei Niederalkylgruppen verestert ist, Carboxy, verestertes Carboxy, z.B. Niederalkoxycarbonyl, Sulfo, amidiertes Sulfo, Amino, Mono- oder Diniederalkylamino, Acylamino, z.B. Niederalkanoylamino, oder durch Carboxy oder Halogen substituiertes Niederalkanoylamino substituiert ist, beispielsweise 2-Hydroxyäthyl, 2-Acetoxyäthyl, 2-Chloräthyl, Carboxymethyl, 2-Carboxyäthyl, Aethoxycarbonylmethyl, 2-Aethoxycarbonyläthyl, Sulfomethyl, 2-Sulfoäthyl, Sulfamylmethyl, 2-Sulfamyläthyl, 2-Aminoäthyl, 2-Dimethylaminoäthyl, 2-Acetylaminoäthyl, 3-Carboxypropionylamino der 3-Chlorpropionylamino.

Funktionelle Gruppen oder abgewandelte, z.B. geschützte, funktionelle Gruppen, z.B. Halogen, z.B. Fluor, Chlor oder Brom, unsubstituiertes oder substituiertes Amino, z.B. unsubstituiertes oder durch Niederalkyl mono- oder disubstituiertes Amino, z.B. Amino, Methylamino oder Dimethylamino, Acylamino, z.B. Niederalkanoylamino oder Niederalkylsulfonylamino, oder durch Halogen oder Carboxy substituiertes Niederalkanoylamino, z.B. Acetylamino, Methylsulfonylamino oder Aethylsulfonylamino, 3-Chlorpropionylamino oder 3-Carboxypropionylamino, Nitro, Hydroxy, Niederalkoxy, z.B. Methoxy oder Aethoxy, Carboxy, verestertes Carboxy, z.B. Niederalkoxycarbonyl, z.B. Methoxycarbonyl oder Aethoxycarbonyl, gegebenenfalls substituiertes, z.B. mono- oder diniederalkyliertes Carbamoyl, z.B. Methylcarbamoyl oder Dimethylcarbamoyl, oder Cyan, sowie Oxo oder Oxido sind ebenfalls Substituenten des heterocyclischen Restes Y.

Bevorzugte unsubstituierte oder substituierte Heterocyclylreste Y sind beispielsweise Thienyl, z.B. 2- oder 3-Thienyl, Furyl, z.B. 2- oder 3-Furyl, Tetrazolyl, z.B. 1-Tetrazolyl, Aminothiazolyl, z.B. 2-Amino-4-thiazolyl, Aminothiadiazolyl, z.B. 5-Amino-1,2,4-thiadiazol-3-yl, und N-Niederalkylaminothiadiazolyl, z.B. 5-N-Methylamino-1,2,4-thiadiazol-3-yl.

Ein heterocyclisch-aliphatischer Rest Y ist beispielsweise einer der genannten aliphatischen Reste, z.B. Methyl, der durch einen der genannten heterocyclischen Reste, z.B. Tetrazolyl, substituiert ist, z.B. Tetrazol-5-ylmethyl.

Eine Niederalkylgruppe $R_1$ enthält 1-4 Kohlenstoffatome und ist beispielsweise Aethyl, Propyl, Butyl oder insbesondere Methyl.

Eine Niederalkoxygruppe $R_1$ enthält 1-4 C-Atome und ist beispielsweise Methoxy, Aethoxy, Propoxy oder Butoxy.

$R_1$ mit der Bedeutung Halogen ist Fluor, Brom oder Jod und bevorzugt Chlor.

Eine veresterte Hydroxy- oder Mercaptogruppe $R_2$ is durch eine niederaliphatische Carbonsäure oder durch eine gegebenenfalls N-substituierte Carbaminsäure verestert.

Eine durch eine niederaliphatische Carbonsäure veresterte Hydroxygruppe $R_2$ ist insbesondere Niederalkanoyloxy, z.B. Acetyloxy, ferner Formyloxy, Propionyloxy, Valeryloxy, Hexanoyloxy, Heptanoyloxy, Pivaloyloxy oder Acetoacetoxy.

Eine durch eine niederaliphatische Carbonsäure veresterte Mercaptogruppe $R_2$ ist insbesondere Niederalkanoylthio, z.B. Acetylthio, Formylthio, Propionylthio, Valeroylthio, Hexanoylthio, Heptanoylthio oder Pivaloylthio.

In einer durch eine gegebenenfalls N-substituierte Carbaminsäure veresterten Hydroxy- oder Mercaptogruppe $R_2$ sind N-Substituenten unsubstituiertes oder durch Halogen, z.B. Chlor, oder durch Niederalkanoyloxy, z.B. Acetoxy oder Propionyloxy, substituiertes Niederalkyl, z.B. Methyl, Aethyl, 2-Chloräthyl oder 2-Acetoxyäthyl.

Eine durch eine gegebenenfalls N-substituierte Carbaminsäure veresterte Hydroxygruppe $R_2$ ist z.B. Carbamoyloxy, N-Methylcarbamoyloxy, N-Aethylcarbamoyloxy, N-(2-Chloräthyl)-carbamoyloxy oder N-(2-Acetoxyäthyl)-carbamoyloxy.

Eine durch eine gegebenenfalls N-substituierte Carbaminsäure veresterte Mercaptogruppe ist z.B. Carbamoylthio, N-Methylcarbamoylthio, N-Aethylcarbamoylthio, N-(2-Chloräthyl)-carbamoylthio oder N-(2-Acetoxyäthyl)-carbamoylthio.

Eine verätherte Hydroxy- oder Mercaptogruppe $R_2$ ist beispielsweise durch einen aliphatischen Kohlenwasserstoffrest veräthert, und ist insbesondere Niederalkoxy mit 1-4 C-Atomen, in erster Linie Methoxy, sowie Aethoxy, n-Propyloxy oder Isopropyloxy, ferner geradkettiges oder verzweigtes Butyloxy, oder ist Niederalkylthio mit 1-4 C-Atomen, in erster Linie Methylthio, sowie Aethylthio, n-Propylthio oder Isopropylthio, ferner geradgettiges oder verzweigtes Butylthio.

Eine verätherte Mercaptogruppe $R_2$ ist insbesondere durch einen unsubstituierten oder substituierten heterocyclischen Rest veräthert, welcher 1 bis 4 Stickstoffheteroatome und gegebenenfalls ein zusätzliches Sauerstoff- oder Schwefelheteroatom besitzt und welcher über ein Ringkohlenstoffatom mit dem Schwefelatom der Mercaptogruppe verbunden ist.

Ein solcher heterocyclischer Rest ist insbesondere ein unsubstituierter oder substituierter monocyclischer, fünfgliedriger und sechsgliedriger diaza-, triaza-, tetraaza-, thiaza-, thiadiaza-, thiatriaza-, oxaza- oder oxadiazacyclischer Rest mit aromatischem Charakter.

Substituenten eines solchen heterocyclischen Restes sind die gleichen, weiter vorn für den heterocyclischen Rest Y genannten Substituenten.

Eine Heterocyclylthiogruppe $R_2$, worin der heterocyclische Rest einen monocyclischen fünfgliedrigen Rest darstellt, ist u.a. Imidazolylthio, z.B. 2-Imidazolylthio, unsubstituiertes oder durch Niederalkyl und/ oder Phenyl substituiertes Triazolylthio, z.B. 1H-1,2,3-Triazol-5-ylthio, 1-Methyl-1H-1,2,3-triazol-4-ylthio, 1H-1,2,4-Triazol-3-ylthio, 5-Methyl-1H-1,2,4-triazol-3-ylthio oder 4,5-Dimethyl-4H-1,2,4-triazol-3-ylthio, unsubstituiertes oder wie angegeben substituiertes Tetrazolylthio, z.B. 1H-Tetrazol-5-ylthio, 1-Methyl-1H-tetrazol-5-ylthio, in Salzform, z.B. als Natriumsalz, vorliegendes oder gegebenenfalls durch ein oder zwei Niederalkyl-, z.B. Aethylgruppen, verestertes 1-Phosphonomethyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio oder 1-(2-Dimethylaminoäthyl)-1H-tetrazol-5-ylthio, unsubstituiertes oder durch Niederalkyl substituiertes Thiazolylthio oder Isothiazolylthio, z.B. 2-Thiazolylthio, 4,5-Dimethyl-2-thiazolylthio, 3-Isothiazolylthio, 4-Isothiazolylthio oder 5-Isothiazolylthio, insbesondere unsubstituiertes oder wie angegeben substituiertes Thiadiazolylthio, z.B. 1,2,3-Thiadiazol-4-ylthio, 1,2,3-Thiadiazol-5-ylthio, 1,3,4-Thiadiazol-2-ylthio, 2-Methyl-1,3,4-thiadiazol-5-ylthio, 1,2,4-Thiadiazol-5-ylthio oder 1,2,5-Thiadiazol-3-ylthio, Thiatriazolylthio, z.B. 1,2,3,4-Thiatriazolyl-5-ylthio, unsubstituiertes oder wie angegeben substituiertes Oxazolylthio oder Isoxazolylthio, z.B. 5-Oxazolylthio, 4-Methyl-5-oxazolylthio, 2-Oxazolylthio oder 3-Methyl-5-isoxazolylthio und unsubstituiertes oder wie angegeben substituiertes Oxadiazolylthio, z.B. 1,2,4-Oxadiazol-5-ylthio oder 2-Methyl-1,3,4-oxadiazol-5-ylthio.

Eine Heterocyclylthiogruppe $R_2$, worin der heterocyclische Rest einen monocyclischen sechsgliedrigen Rest darstellt, enthält 1-3 Stickstoffatome und ist beispielsweise unsubstituiertes oder durch Niederalkyl, z.B. Methyl, Carboxyniederalkyl, z.B. Carboxymethyl, oder durch Sulfoniederalkyl, z.B. Sulfomethyl, substituiertes 5,6-Dioxotetrahydro-as-triazinylthio, z.B. 1- oder 2-Methyl-5,6-dioxo-1,2,5,6-tetrahydro-as-triazin-3-ylthio, 4-Methyl-5,6-dioxo-1,4,5,6-tetrahydro-as-triazin-3-ylthio, 1- oder 2-Carboxymethyl-5,6-dioxo-1,2,5,6-tetrahydro-as-triazin-3-ylthio, 4-Carboxymethyl-5,6-dioxo-1,4,5,6-tetrahydro-as-triazin-3-ylthio, 1- oder 2-Sulfomethyl-5,6-dioxo-1,2,5,6-tetrahydro-as-triazin-3-ylthio oder 4-Sulfomethyl-5,6-dioxo-1,4,5,6-tetrahydro-as-triazin-3-ylthio.

Eine quaternäre Ammoniumgruppe $R_2$ ist von einer tertiären organischen Base, beispielsweise von einem aliphatischen Amin oder vorzugsweise von einer unsubstituierten oder substituierten heterocyclischen Stickstoffbase, abgeleitet und ist über das Stickstoffatom an die in 3-Stellung des Cephemgerüsts befindliche Methylengruppe gebunden. Die positive Ladung am quaternären Stickstoffatom wird beispielsweise durch die in 4-Stellung des Cephemgerüsts befindliche, negativ geladene Carboxylatgruppe kompensiert.

Eine quaternäre Ammoniumgruppe $R_2$, welche von einem aliphatischen Amin abgeleitet ist, ist beispielsweise Triniederalkylammonium, z.B. Trimethyl- oder Triäthylammonium.

Eine quaternäre Ammoniumgruppe $R_2$, welche von einer unsubstituierten oder substituierten heterocyclischen Stickstoffbase abgeleitet ist, ist beispielsweise unsubstituiertes oder durch Niederalkyl, z.B. Methyl oder Aethyl, Niederalkenyl, z.B. Vinyl oder Allyl, Carboxyniederalkyl, z.B. Carboxymethyl, Niederalkoxycarbonylniederalkyl, z.B. Methoxycarbonylmethyl, Sulfoniederalkyl, z.B. Sulfomethyl, Aminoniederalkyl, z.B. 2-Aminoäthyl, oder Diniederalkylaminoniederalkyl, z.B. 2-Dimethylaminoäthyl, in 2-Stellung substituiertes 1-Pyrazolium, z.B. 2-Methyl- oder 2-Aethyl-1-pyrazolium, 2-Allyl- oder 2-Vinyl-1-pyrazolium, 2-Carboxymethyl-1-pyrazolium, 2-Methoxycarbonylmethyl-1-pyrazolium, 2-Sulfomethyl-1-pyrazolium, 2-(2-Aminoäthyl)-1-pyrazolium oder 2-(2-Dimethylaminoäthyl)-1-pyrazolium.

Eine quaternäre Ammoniumgruppe $R_2$, welche von einer unsubstituierten oder substituierten heterocyclischen Stickstoffbase abgeleitet ist, ist ebenfalls beispielsweise unsubstituiertes oder durch Niederalkyl, z.B. Methyl oder Aethyl, Carboxyniederalkyl, z.B. Carboxymethyl oder Diniederalkylaminoniederalkyl, z.B. 2-Dimethylaminoäthyl in 3-Stellung substituiertes 1-Triazolium, z.B. 3-Methyl-1-triazolium, 3-Carboxymethyl-1-triazolium oder 3-(2-Dimethylaminoäthyl)-1-triazolium.

Eine quaternäre Ammoniumgruppe $R_2$, welche von einer unsubstituierten oder substituierten heterocyclischen Stickstoffbase abgeleitet ist, ist ebenfalls beispielsweise unsubstituiertes oder durch Niederalkyl, z.B. Methyl, Carbamoyl, Niederalkylcarbamoyl, z.B. Methylcarbamoyl, Hydroxyniederalkyl, z.B. Hydroxymethyl, Niederalkoxyniederalkyl, z.B. Methoxymethyl, Cyanniederalkyl, z.B. Cyanmethyl, Carboxyniederalkyl, z.B. Carboxymethyl, Sulfoniederalkyl, z.B. 2-Sulfoäthyl, Carboxyniederalkyl, z.B. 2-Carboxyvinyl, Carboxyniederalkylthio, z.B. Carboxymethylthio, Thiocarbamoyl, Halogen, z.B. Brom oder Chlor, Carboxy, Sulfo oder Cyan mono- oder disubstituiertes Pyridinium, z.B. Niederalkylpyridinium, z.B. 2-, 3- oder 4- Methylpyridinium oder 2-, 3- oder 4-Aethylpyridinium, Carbamoylpyridinium, z.B. 3- oder 4-Carbamoylpyridinium, Niederalkylcarbamoylpyridinium, z.B. 3- oder 4-Methylcarbamoylpyridinium, z.B. 3- oder 4-Methylcarbamoylpyridinium, Diniederalkylcarbamoylpyridinium, z.B. 3- oder 4-Dimethylcarbamoylpyridinium, Hydroxyniederalkylpyridinium, z.B. 3- oder 4-Hydroxymethylpyridinium, Niederalkoxyniederalkylpyridinium, z.B. 4-Methoxymethylpyridinium, Cyanoniederalkylpyridinium, z.B. 3-Cyanomethylpyridinium, Carboxyniederalkylpyridinium, z.B. 3-Carboxymethylpyridinium, Sulfoniederalkylpyridinium, z.B. 4-(2-Sulfoäthylpyridinium), Carboxyniederalkenylpyridinium, z.B. 3-(2-Carboxyvinyl)-pyridinium, Carboxyniederalkylthiopyridinium, z.B. 4-Carboxymethylthiopyridinium, Thiocarbamoylpyridinium, z.B. 4-Thiocarbamoylpyridinium, Halogenpyridinium, z.B. 3-Brom- oder 4-Brompyridinium, Carboxypyridinium, z.B. 4-Carboxypyridinium, Sulfopyridinium, z.B. 3-Sulfopyridinium, Cyanpyridinium,

z.B. 3-Cyanpyridinium, Carboxyniederalkyl-carbamoylpyridinium, z.B. 3-Carboxymethyl-4-carbamoylpyridinium, Amino-carbamoylpyridinium, z.B. 2-Amino-5-carbamoylpyridinium, Carboxy-carbamoylpyridinium, z.B. 3-Carboxy-4-carbamoylpyridinium, Cyano-halogenmethylpyridinium, z.B. 3-Cyano-4-trifluormethylpyridinium, und Amino-carboxypyridinium, z.B. 2-Amino-3-carboxypyridinium.

Eine quaternäre Ammoniumgruppe $R_2$ ist bevorzugt 2-Niederalkyl-1-pyrazolium, z.B. 2-Methyl-1-pyrazolium, 2-Carboxyniederalkyl-1-pyrazolium, z.B. 2-Carboxymethyl-1-pyrazolium, 3-Niederalkyl-1-triazolium, z.B. 3-Methyl-1-triazolium, oder unsubstituiertes oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinium, z.B. 3- oder 4-Hydroxymethylpyridinium, 4-Carboxypyridinium, 3- oder 4-Carboxymethylpyridinium, 3- oder 4-Chlorpyridinium, 3- oder 4-Brompyridinium oder 3- oder 4-Carbamoyl-pyridinium.

Niederalkoxy $R_3$ ist beispielsweise Methoxy, ferner Aethoxy, n-Propyloxy, Isopropyloxy oder n-Butyloxy.

$R_4$ mit der Bedeutung "geschütztes Carboxyl" ist unter physiologischen Bedingungen spaltbares Carboxyl oder ist durch die weiter unten beschriebenen Schutzgruppen verestertes Carboxyl.

Die in den Verbindungen der Formel I vorhandenen funktionellen Gruppen, insbesondere Carboxyl- und Amino-, ferner Hydroxy- und Sulfogruppen, sind gegebenenfalls durch übliche Schutzgruppen (conventional protecting groups) geschützt, die in der Penicillin-, Cephalosporin- und Peptidchemie verwendet werden.

Solche Schutzgruppen sind leicht, das heisst ohne dass unerwünschte Nebenreaktionen stattfinden, beispielsweise solvolytisch, reduktiv, photolytisch oder auch unter physiologischen Bedingungen, abspaltbar.

Schutzgruppen dieser Art, sowie ihre Abspaltung sind beispielsweise in "Protective Groups in Organic Chemistry", Plenum Press, London, New York, 1973 in "Protective Groups in Organic Chemistry", Willy, New York, 1974, in "The Peptides", Vol. I, Schröder and Lubke, Academic Press, London, New York, 1965, sowie in "Methoden der organischen Chemie", Houben-Weyl, 4. Auflage, Bd. 15/1, Georg Thieme Verlag, Stuttgart, 1974, beschrieben.

So sind Carboxylgruppen, z.B. die Carboxylgruppe $R_4$ oder die Carboxylgruppe in der α-Aminocarbonsäuregruppierung, ferner in $R_1$ und Y vorhandene Carboxylgruppen üblicherweise in veresterter Form geschützt, wobei solche Estergruppierungen unter schonenden Bedingungen leicht spaltbar sind. In dieser Art geschützte Carboxylgruppen sind in erster Linie durch Niederalkylgruppen verestert, welche in 1-Stellung verzweigt oder in 1- oder 2-Stellung durch geeignete Substituenten substituiert sind.

Bevorzugte geschützte Carboxylgruppen, welche durch in 1-Stellung verzweigtes Niederalkyl verestert sind, sind beispielsweise tert.-Niederalkoxycarbonyl, z.B. tert.-Butyloxycarbonyl, Arylmethoxycarbonyl mit einem oder zwei Arylresten, worin die Aryl unsubstituiertes oder z.B. durch Niederalkyl, z.B. tert.-Niederalkyl, z.B. tert.-Butyl, Niederalkoxy, z.B. Methoxy, Hydroxy, Halogen, z.B. Chlor, und/oder Nitro, mono- oder polysubstituiertes Phenyl bedeutet, beispielsweise unsubstituiertes oder z.B. wie oben erwähnt substituiertes Benzyloxycarbonyl, z.B. 4-Nitrobenzyloxycarbonyl oder 4-Methoxybenzyloxycarbonyl, oder unsubstituiertes oder z.B. wie oben erwähnt substituiertes Diphenylmethoxycarbonyl, z.B. Diphenylmethoxycarbonyl oder Di-(4-methoxyphenyl)-methoxycarbonyl.

Bevorzugte geschützte Carboxylgruppen, welche durch in 1- oder 2-Stellung geeignet substituiertes Niederalkyl verestert sind, sind beispielsweise 1-Niederalkoxyniederalkoxycarbonyl, z.B. Methoxymethoxycarbonyl, 1-Methoxyäthoxycarbonyl oder 1-Aethoxymethoxycarbonyl, 1-Niederalkylthioniederalkoxycarbonyl, z.B. 1-Methylthiomethoxycarbonyl oder 1-Aethylthioäthoxycarbonyl, Aroylmethoxycarbonyl, worin die Aroylgruppe unsubstituiert oder z.B. durch Halogen, wie Brom, substituiertes Benzoyl darstellt, z.B. Phenacyloxycarbonyl, sowie 2-Halogenniederalkoxycarbonyl, z.B. 2,2,2-Trichloräthoxycarbonyl, 2-Bromäthoxycarbonyl oder 2-Jodäthoxycarbonyl. Carboxylgruppen sind ebenfalls als organische Silyloxycarbonylgruppen geschützt.

Eine Silyloxycarbonylgruppe ist beispielsweise eine Triniederalkylsilyloxycarbonylgruppe, z.B. Trimethylsilyloxycarbonyl. Das Siliciumatom der Silyloxycarbonylgruppe kann auch nur durch zwei Niederalkylgruppen, z.B. Methylgruppen, und die Carboxylgruppe oder Aminogruppe eines zweiten Moleküls der Formel I substituiert sein. Verbindungen mit solchen Schutzgruppen lassen sich z.B. bei Verwendung von Dimethyldichlorsilan als Silylierungsmittel herstellen.

Bevorzugte geschützte Carboxylgruppen sind tert.-Niederalkoxycarbonyl, wie tert.-Butyloxycarbonyl, und in erster Linie gegebenenfalls, z.B. wie oben erwähnt, substituiertes Benzyloxycarbonyl, z.B. 4-Nitrobenzyloxycarbonyl, oder Diphenylmethoxycarbonyl.

Eine unter physiologischen Bedingungen spaltbare, veresterte Carboxylgruppe $R_4$ ist in erster Linie eine Acyloxymethoxycarbonylgruppe, worin Acyl z.B. die Acylgruppe einer organischen Carbonsäure, in erster Linie einer gegebenenfalls substituierten Niederalkancarbonsäure bedeutet, oder worin Acyloxymethyl den Rest eines Lactons bildet. Solche Gruppen sind Niederalkanoyloxymethoxycarbonyl, z.B. Acetyloxymethoxycarbonyl oder Pivaloyloxymethoxycarbonyl, Niederalkoxycarbonyloxyniederalkoxycarbonyl, z.B. 1-Aethyloxycarbonyloxyäthyloxycarbonyl, Aminoniederalkanoylmethoxycarbonyl, insbesondere α-Amino-niederalkanoyloxymethoxycarbonyl, z.B. Glycyloxymethoxycarbonyl, L-Valyloxymethoxycarbonyl oder L-Leucyloxymethoxycarbonyl,

ferner Phthalidyloxycarbonyl, z.B. 2-Phthalidyloxycarbonyl, oder Indanyloxycarbonyl, z.B. 5-Indanyloxycarbonyl.

Aminogruppen, z.B. die Aminogruppe in der α-Aminocarbonsäuregruppierung, sowie in $R_1$ und Y vorhandene Aminogruppen, können z.B. in Form einer leicht spaltbaren Acylamino-, Arylmethylamino-, verätherten Mercaptoamino-, 2-Acylniederalk-1-enylamino- oder Silylaminogruppe geschützt sein.

In einer leicht spaltbaren Acylaminogruppe ist Acyl beispielsweise die Acylgruppe einer organischen Carbonsäure mit bis zu 18 Kohlenstoffatomen, insbesondere einer unsubstituierten oder einer z.B. durch Halogen oder Aryl substituierten Alkancarbonsäure oder der unsubstituierten oder z.B. durch Halogen, Niederalkoxy oder Nitro substituierten Benzoesäure oder eines Kohlensäurehalbesters. Solche Acylgruppen sind beispielsweise Niederalkanoyl, z.B. Formyl, Acetyl, oder Propionyl, Halogenniederalkanoyl, z.B. 2-Halogenacetyl, insbesondere 2-Chlor-, 2-Brom-, 2-Jod-, 2,2,2-Trifluor- oder 2,2,2-Trichloracetyl, unsubstituiertes oder z.B. durch Halogen, Niederalkoxy oder Nitro substituiertes Benzoyl, z.B. Benzyol, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl.

In 1-Stellung des Niederalkylrests verzweigtes Niederalkoxycarbonyl ist beispielsweise tert.-Niederalkoxycarbonyl, z.B. tert.-Butyloxycarbonyl, Arylmethoxycarbonyl mit einem oder zwei Arylresten, worin Aryl vorzugsweise unsubstituiertes oder z.B. durch Niederalkyl, insbesondere tert.-Niederalkyl, z.B. tert.-Butyl, Niederalkoxy, z.B. Methoxy, Hydroxy, Halogen, z.B. Chlor, und/oder Nitro, mono- oder polysubstituiertes Phenyl bedeutet, beispielsweise unsubstituiertes oder substituiertes Benzyloxycarbonyl, z.B. 4-Nitrobenzyloxycarbonyl oder unsubstituiertes oder substituiertes Diphenylmethoxycarbonyl, z.B. Benzhydryloxycarbonyl oder Di-(4-methoxyphenyl)-methoxycarbonyl.

In 1- oder 2-Stellung geeignet substituiertes Niederalkoxycarbonyl ist beispielsweise Triniederalkylsilyloxycarbonyl, z.B. Trimethylsilyloxycarbonyl, Aroylmethoxycarbonyl, worin Aroyl unsubstituiertes oder z.B. durch Halogen, z.B. Brom, substituiertes Benzoyl darstellt, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen unsubstituierten oder z.B. durch Niederalkyl, Niederalkoxy, Aryl, Halogen oder Nitro, substituierten aliphatischen, araliphatischen, cycloaliphatischen oder aromatischen Kohlenwasserstoffrest mit bis zu 15 C-Atomen bedeuten, z.B. unsubstituiertes oder wie oben erwähnt substituiertes Niederalkyl, Phenylniederalkyl, Cycloalkyl oder Phenyl, beispielsweise 2-Triniederalkylsilyläthoxycarbonyl, z.B. 2-Trimethylsilyläthoxycarbonyl oder 2-(Di-n-butylmethylsilyl)-äthoxycarbonyl, oder 2-Triarylsilyläthoxycarbonyl, z.B. 2-Triphenylsilyläthoxycarbonyl.

Weitere als Aminoschutzgruppen in Frage kommende Acylgruppen sind auch Acylgruppen von organischen Phosphor-, Phosphon- oder Phosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, Diphenylphosphoryl, unsubstituiertes oder z.B. durch Nitro substituiertes Diphenylniederalkylphosphoryl, z.B. Dibenzylphosphoryl oder Di-4-nitrobenzylphosphoryl, Phenyloxyphenylphosphonyl, Diniederalkylphosphinyl, z.B. Diäthylphosphinyl, ferner Diphenylphosphinyl.

In einer Arylmethylaminogruppe, die Mono-, Di- oder insbesondere Triarylmethylamino darstellt, sind die Arylreste insbesondere Phenylreste. Solche Gruppen sind beispielsweise Benzyl-, Diphenylmethyl- und insbesondere Tritylamino.

In einer verätherten Mercaptoaminogruppe ist die verätherte Mercaptogruppe in erster Linie Arylthio der Arylniederalkylthio, worin Aryl insbesondere unsubstituiertes oder z.B. durch Niederalkyl, z.B. Methyl oder tert.-Butyl, Niederalkoxy, z.B. Methoxy, Halogen, z.B. Chlor, und/oder Nitro substituiertes Phenyl ist. Eine solche Aminoschutzgruppe ist z.B. 4-Nitrophenylthio.

In einer als Aminoschutzgruppe verwendbaren 2-Acylniederalk-1-en-yl-gruppe ist Acyl z.B. die Acylgruppe einer Niederalkancarbonsäure, der unsubstituierten oder z.B. durch Niederalkyl, z.B. Methyl oder tert.-Butyl, Niederalkoxy, z.B. Methoxy, Halogen, z.B. Chlor, und/oder Nitro substituierten Benzoesäure, oder insbesondere die Acylgruppe eines Kohlensäurehalbesters, z.B. eines Kohlensäureniederalkylhalbesters. Solche Aminoschutzgruppen sind in erster Linie 1-Niederalkanoyl-prop-1-en-2-yl, z.B. 1-Acetylprop-1-en-2-yl, oder 1-Niederalkoxy-carbonylprop-1-en-2-yl, z.B. 1-Aethoxycarbonylprop-1-en-2-yl.

Eine Silylaminogruppe ist beispielsweise eine Triniederalkylsilylaminogruppe, z.B. Trimethylsilylamino. Das Siliciumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. bei Verwendung von Dimethyldichlorsilan als Silylierungsmittel herstellen.

Eine Aminogruppe kann auch in protonierter Form geschützt werden. Als Anionen kommen in erster Linie die Anionen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, unsubstituiertes oder z.B. wie angegeben substituiertes Benzyloxycarbonyl, z.B. 4-Nitrobenzyloxycarbonyl oder Diphenylmethoxycarbonyl, 2-Halogenniederalkoxycarbonyl, z.B. 2,2,2-Trichloräthoxycarbonyl, ferner Trityl oder Formyl.

Hydroxygruppen, z.B. in $R_1$ und Y vorhandene Hydroxygruppen, können beispielsweise als Acylgruppen, z.B. durch Halogen substituiertes Niederalkanoyl, z.B. 2,2-Dichloracetyl, oder insbesondere durch die im Zusammenhang mit geschützten Aminogruppen genannten Acylreste von Kohlensäurehalbestern geschützt sein. Bevorzugte Hydroxyschutzgruppen sind beispielsweise 2,2,2-Trichloräthoxycarbonyl, organische Silylreste mit den oben genannten Substituenten, ferner leicht abspaltbare veräthernde Gruppen, wie tert.-Niederalkyl, z.B. tert.-Butyl, 2-oxa- oder 2-thia-aliphatische oder -cycloaliphatische Kohlenwasserstoffreste, beispielsweise 1-Niederalkoxyniederalkyl oder 1-Niederalkylthioniederalkyl, z.B. Methoxymethyl, 1-Methoxyäthyl, 1-Aethoxyäthyl, 1-Methylthiomethyl, 1-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl oder entsprechende Thiaanaloge, sowie unsubstituiertes oder substituiertes 1-Phenylniederalkyl, z.B. unsubstituiertes oder substituiertes 1-Phenylniederalkyl, z.B. unsubstituiertes oder substituiertes Benzyl oder Diphenylmethyl, wobei die Phenylreste beispielsweise durch Halogen, wie Chlor, Niederalkoxy, z.B. Methoxy, und/oder Nitro substituiert sind.

Sulfogruppen, z.B. in $R_1$ und Y vorhandene Sulfogruppen, sind vorzugsweise durch einen aliphatischen, cycloaliphatischen, cycloaliphatischaliphatischen, aromatischen oder araliphatischen Alkohol, z.B. durch Niederalkanol, oder durch eine Silylgruppe, z.B. durch Triniederalkylsilyl, verestert. Eine Sulfogruppe ist analog einer Carboxygruppe geschützt.

Salze von erfindungsgemässen Verbindungen sind in erster Linie pharmazeutisch verwendbare, nicht-toxische Salze von Verbindungen der Formel I.

Solche Salze werden beispielsweise von den in Verbindungen der Formel I vorhandenen sauren Gruppen, z.B. Carboxyl- oder Sulfogruppen gebildet und sind in erster Linie Metall- oder Ammoniumsalze, beispielsweise Alkalimetall- und Erdalkalimetall-, z.B. Natrium-, Kalium-, Magnesium- oder Calciumsalze, sowie Ammoniumsalze, welche mit Ammoniak oder geeigneten organischen Aminen gebildet werden, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen. Solche Basen sind beispielsweise Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthyldiaminoäthylester, Niederalkylenamine, z.B. 1-Aethylpiperidin, Cycloalkylamine, z.B. Dicyclohexylamin, oder Benzylamine, z.B. N,N'-Dibenzyläthylendiamin, ferner Basen vom Pyridintyp, z.B. Pyridin, Collidin oder Chinolin.

Die in den Verbindungen der Formel I vorhandenen basischen Gruppen, z.B. Aminogruppen, können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden.

Liegen in Verbindungen der Formel I mehrere saure Gruppen, z.B. zwei Carboxylgruppen, oder basische Gruppen vor, können Mono- oder Polysalze gebildet werden. Da die Verbindungen der Formel I mindestens eine saure Gruppe, z.B. die Carboxylgruppe in der α-Aminocarbonsäuregruppierung, und mindestens eine basische Gruppe, z.B. die Amino-gruppe in der α-Aminocarbonsäuregruppierung, besitzen, können Verbindungen der Formel I in Form von inneren Salzen, d.h. in zwitterionischer Form, vorliegen. In Verbindung der Formel I können eine saure und eine basische Gruppe in Form eines inneren Salzes und zusätzliche saure und/oder basische Gruppen beispielsweise als Säureadditions- und/oder

Baseadditionssalz vorliegen.

Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt werden.

Die Verbindungen der Formel I, worin die funktionellen Gruppen in freier Form und die Carboxylgruppen gegebenenfalls in physiologisch spaltbarer veresterter Form vorliegen, und ihre pharmazeutisch verwendbaren, nichttoxischen Salze sind wertvolle, antibiotisch Wirkstoffe, die insbesondere als antibakterielle Antibiotika verwendet werden können. Beispielsweise sind sie in vitro gegen grampositive und gramnegative Mikroorganismen, inklusive β-Lactamase produzierende Stämme, z.B. gegen Kokken wie Staphylococcus aureus, Strepococcus pneumoniae, Streptococcus pyogenes, Neisseria gonorrhoeae und Neisseria meningitidis in Minimalkonzentrationen von ca. 0,01 bis ca 64 µg/ml, gegen Enterobakterien wie Escherichia coli, Salmonella thyphimurium, Klebsiella pneumoniae, Proteus spp, Enterobacter cloacae Serratia marcescens, Haemophilus influenzae und Pseudomonas aeruginosa in Minimalkonzentrationen von ca. 0,01 µg/ml bis ca. 64 µg/ml wirksam. In vivo, bei subkutaner Applikation an der Maus, sind sie beispielsweise gegen systemische Infektionen mit Staphylokokken in Minimaldosen von ca. 30 mg/kg und gegen systemische Infektionen mit Enterobakterien in einem Dosisbereich von ca. 0,3 mg/kg bis ca. 85 mg/kg wirksam.

In dem folgenden Versuchsbericht wird anhand von ausgewählten Verbindungen die Wirksamkeit von Verbindungen der Formel I gezeigt:

Versuchsbericht

I. Getestete Verbindungen:

Für die folgenden Verbindungen wurde die antibiotische Wirksamkeit getestet:

1. 3-Acetoxymethyl-7β-[2-((2R)-2-amino-2-carboxyäthoxycarbonylamino)-acetamido]-3-cephem-4-carbonsäure-natriumsalz (Beispiel 1c)).
2. 3-Acetoxymethyl-7α-methoxy-7β-[2-((2R)-2-amino-2-carboxyäthoxycarbonylamino)-acetamido]-3-cephem-4-carbonsäure-natriumsalz (Beispiel 1d)).
3. 3-(1-Methyl-1H-tetrazol-5-ylthiomethyl)-7α-methoxy-7β-[2-((2R)-2-amino-2-carboxyäthoxycarbonylamino)-acetamido]-3-cephem-4-carbonsäurenatriumsalz.(Beispiel 1e)).
4. 3-(1-Carboxymethyl-1H-tetrazol-5-ylthiomethyl)-7α-methoxy-7β-[2-((2R)-2-amino-2-carboxyäthoxycarbonylamino)-acetamido]-3-cephem-4-carbonsäure-dinatriumsalz (Beispiel 1f)).
5. 3-[1-(2-Dimethylaminoäthyl)-1H-tetrazol-5-ylthiomethyl]-7α-methoxy-7β-[2-((2R)-2-amino-2-carboxyäthoxycarbonylamino)-acetamido]-3-cephem-4-carbonsäure-natriumsalz (Beispiel 1g)).
6. 3-(1-Sulfomethyl-1H-tetrazol-5-ylthiomethyl)-7α-methoxy-7β-[2-((2R)-2-amino-2-carboxyäthoxycarbonylamino)-acetamido]-3-cephem-4-carbonsäure-dinatriumsalz (Beispiel 1h)).
7. 3-(4-Carbamoylpyridiniomethyl)-7α-methoxy-7β-[2-((2R)-2-amino-2-carboxyäthoxycarbonylamino)-acetamido]-3-cephem-4-carboxylat (Beispiel 1i)).
8. 3-Carbamoyloxymethyl-7α-methoxy-7β-[2-((2R)-2-amino-2-carboxyäthoxycarbonylamino)-acetamido]-3-cephem-4-carbonsäure-natriumsalz (Beispiel 2d)).
9. 7α-Methoxy-7β-[2-((2R)-2-amino-2-carboxyäthoxycarbonylamino)-acetamido]-3-cephem-4-carbonsäure-natriumsalz (Beispiel 3c)).
10. 3-Acetoxymethyl-7α-methoxy-7β-[(2R)-2-((2R)-2-amino-2-carboxyäthoxycarbonylamino)-2-methylacetamido]-3-cephem-4-carbonsäure-natriumsalz (Beispiel 5c)).

11. 3-(1-Methyl-1H-tetrazol-5-ylthiomethyl)-7α-methoxy-7β-[(2R)-2-((2R)-2-amino-2-carboxyäthoxycarbonylamino)-2-methylacetamido]-3-cephem-4-carbonsäure-natriumsalz (Beispiel 5d)).

12. 3-Acetoxymethyl-7β-[(2R,S)-2-((2R)-2-amino-2-carboxyäthoxycarbonylamino)-2-(2-aminothiazol-4-yl)-acetamido]-3-cephem-4-carbonsäure-natriumsalz (Beispiel 8c)).

13. 3-(1-Methyl-1H-tetrazol-5-ylthiomethyl)-7β-[(2R,S)-2-((2R)-2-amino-2-carboxyäthoxycarbonylamino)-2-(2-aminothiazol-4-yl)-acetamido]-3-cephem-4-carbonsäure-natriumsalz (Beispiel 8d)).

14. 3-(1-Methyl-1H-tetrazol-5-ylthiomethyl)-7α-methoxy-7β-[(2R)-2-((2R)-2-amino-2-carboxyäthoxycarbonylamino-2-methoxymethylacetamido]-3-cephem-4-carbonsäure-natriumsalz (Beispiel 12e)).

15. 3-(1-Methyl-1H-tetrazol-5-ylthiomethyl)-7α-methoxy-7β-[(2R)-2-((2R)-2-amino-2-carboxyäthoxycarbonylamino-2-hydroxymethylacetamido]-3-cephem-4-carbonsäure-natriumsalz (Beispiel 12g)).

16. 3-Acetoxymethyl-7β-[(2R)-2-((2R)-2-amino-2-carboxyäthoxycarbonylamino)-2-(2-aminothiazol-4-yl)-acetamido]-3-cephem-4-carbonsäure-natriumsalz (Beispiel 9d)).

17. 3-Acetoxymethyl-7β-[(2S)-2-((2R)-2-amino-2-carboxyäthoxycarbonylamino)-2-(2-aminothiazol-4-yl)-acetamido]-3-cephem-4-carbonaäure-natriumsalz (Beispiel 9e)).

18. 3-[1-(2-Dimethylaminoäthyl)-1H-tetrazol-5-ylthiomethyl]-7β-[(2R,S)-2-((2R)-2-amino-2-carboxyäthoxycarbonylamino)-2-(2-aminothiazol-4-yl)-acetamido]-3-cephem-4-carbonsäure-natriumsalz (Beispiel 9f)).

19. 3-(1-Carboxymethyl-1H-tetrazol-5-ylthiomethyl)-7β-[(2R,S)-2-((2R)-2-amino-2-carboxyäthoxycarbonylamino)-2-(2-aminothiazol-4-yl)-acetamido]-3-cephem-4-carbonsäure-natriumsalz (Beispiel 9g)).

20. 3-(1-Sulfomethyl-1H-tetrazol-5-ylthiomethyl)-7β-[(2R,S)-2-((2R)-2-amino-2-carboxyäthoxycarbonylamino)-2-(2-aminothiazol-4-yl)-acetamido]-3-cephem-4-carbonsäure-natriumsalz (Beispiel 9h)).

21. 3-(1-Methyl-1H-tetrazol-5-ylthiomethyl)-7α-methoxy-7β-[(2R)-2-((4R)-4-amino-4-carboxybutyrylamido)-2-methylacetamido]-3-cephem-4-carbonsäure-natriumsalz (Beispiel 11f)).

22. 3-(1-Methyl-1H-tetrazol-5-ylthiomethyl)-7β-[(2R,S)-2-((3R)-3-amino-3-carboxypropionamido)-2-(2-aminothiazol-4-yl)-acetamido]-3-cephem-4-carbonsäure-natriumsalz (Beispiel 10 d)).
23. 3-(1-Methyl-1H-tetrazol-5-ylthiomethyl)-7β-[(2S)-2-((2R)-2-amino-2-carboxyäthoxycarbonylamino)-2-phenylacetamido]-3-cephem-4-carbonsäure-natriumsalz (Beispiel 14b)).
24. 3-(1-Methyl-1H-tetrazol-5-ylthiomethyl)-7β-[(2R)-2-((2R)-2-amino-2-carboxyäthoxycarbonylamino)-2-(2-aminothiazol-4-yl)-acetamido]-3-cephem-4-carbonsäure-natriumsalz (Beispiel 15b)).
25. 3-(1-Methyl-1H-tetrazol-5-ylthiomethyl)-7β-[(2S)-2-((2R)-2-amino-2-carboxyäthoxycarbonylamino)-2-(2-aminothiazol-4-yl)-acetamido]-3-cephem-4-carbonsäure-natriumsalz (Beispiel 15c)).
26. 3-(4-Carbamoylpyridiniomethyl)-7β-[(2R,S)-2-((2R)-2-amino-2-carboxyäthoxycarbonylamino)-2-(2-aminothiazol-4-yl)-acetamido]-3-cephem-4-carboxylat (Beispiel 16)).
27. 3-Acetoxymethyl—7α-methoxy-7β-[4-((2R)-2-amino-2-carboxyäthoxycarbonylamino)-butyramido]-3-cephem-4-carbonsäure-natriumsalz (Beispiel 18c)).
28. 3-(1-Methyl-1H-tetrazol-5-ylthiomethyl)-7α-methoxy-7β-[4-((2R)-2-amino-2-carboxyäthoxycarbonylamino)-butyramido]-3-cephem-4-carbonsäure-natriumsalz (Beispiel 18e)).
29. 3-Acetoxymethyl-7β-[4-((2R)-2-amino-2-carboxyäthoxycarbonylamino)-butyramido]-3-cephem-4-carbonsäure-natriumsalz (Beispiel 18d)).
30. 3-(1-Methyl-1H-tetrazol-5-ylthiomethyl)-7β-[4-((2R)-2-amino-2-carboxyäthoxycarbonylamino)-butyramido]-3-cephem-4-carbonsäure-natriumsalz (Beispiel 18f)).

II. Experimentelles:

A. Die antibiotische Aktivität der Testverbindungen in vitro wurde durch die Agarverdünnungsmethode nach Ericsson, H.M., und Sherris, S.C., 1971, Acta Path. Microb. Scand. Section B, Suppl. No. 217, Bd. 1-90, in DST Agar ermittelt. Die gefundenen, das Wachstum der Testorganismen noch hemmenden Minimalkonzentrationen (MIC = minimum inhibiting concentrations) werden in Mikrogramm pro Milliliter (μg/ml)

für die geprüften Verbindungen in der Tabelle 1 angegeben.

B. Die chemotherapeutische Wirksamkeit in vivo gegen systemische Infektionen in weiblichen SPF, $MF_2$ Mäusen wurde nach der Methode von Zak., O., et al., 1979, Drugs Exptl. Clin. Res. 5, 45-59, ermittelt. Die gefundenen $ED_{50}$-Werte in Milligramm Substanz pro Kilogramm Maus (mg/kg) für eine Anzahl von Mikroorganismen werden für die oral (p.o.) oder subcutan (s.c.) applizierten Testverbindungen in der Tabelle 2 angegeben.

III. Versuchsergebnisse:

Tabelle 1: Antibiotische Aktivität (in vitro)

| Testverbindung | MIC [µg/ml] | | | |
|---|---|---|---|---|
| | Escherichia coli 205 | Klebsiella pneumoniae 327 | Salmonella typhimurium 277 | Pseudomonas aeruginosa 799/61 |
| 1 | 4 | 4 | 4 | 4 |
| 2 | 1 | 1 | 1 | 0,5 |
| 3 | 1 | 1 | 1 | 0,5 |
| 4 | 1 | 1 | 0,5 | 0,5 |
| 5 | 2 | 2 | 2 | 1 |
| 6 | 2 | 2 | 1 | 1 |
| 7 | 8 | 8 | 4 | 1 |
| 8 | 1 | 1 | 1 | 1 |
| 9 | 4 | 8 | 4 | 16 |
| 10 | 1 | 1 | 1 | 1 |
| 11 | 1 | 1 | 1 | 1 |
| 12 | 0,05 | 0,05 | 0,05 | 0,1 |
| 13 | 0,02 | 0,05 | 0,02 | 0,05 |
| 14 | 8 | 8 | 2 | 1 |
| 15 | 8 | 2 | 2 | 1 |

| Testverbindung | MIC [µg/ml] | | | |
|---|---|---|---|---|
| | Escherichia coli 205 | Klebsiella pneumoniae 327 | Salmonella typhimurium 277 | Pseudomonas aeruginosa 799/61 |
| 16 | 0,1 | 0,1 | 0,1 | 0,5 |
| 17 | 0,002 | 0,002 | 0,002 | 0,1 |
| 18 | 0,1 | 0,1 | 0,1 | 0,2 |
| 19 | 0,2 | 0,2 | 0,2 | 0,1 |
| 20 | 0,2 | 0,2 | 0,2 | 0,1 |
| 21 | 2 | 2 | 1 | 0,5 |
| 22 | 0,02 | 0,1 | 0,1 | 0,1 |
| 23 | 2 | 2 | 1 | 1 |
| 24 | 0,1 | 0,2 | 0,1 | 0,1 |
| 25 | 0,02 | 0,02 | 0,02 | 0,05 |
| 26 | 0,2 | 0,5 | 0,2 | 0,5 |
| 27 | 1 | 1 | 1 | 0,5 |
| 28 | 1 | 0,5 | 0,5 | 0,2 |
| 29 | 32 | 4 | 2 | 1 |
| 30 | 2 | 0,5 | 0,5 | 0,5 |

Tabelle 2: Chemotherapeutische Wirksamkeit (in vivo)

| Testverbindung | $ED_{50}$ [mg/kg, s.c.] | | |
|---|---|---|---|
| | Staphylococcus anvens 10B | Escherichia coli 205 | Proteus morganii 2359 |
| 1 | 30 | 85 | >100 |
| 2 | >30 | 6 | 15 |
| 3 | 40 | 6 | 8 |
| 4 | >30 | 5 | 10 |
| 5 | 30 | 7 | 10 |

| Test-verbin-dung | $ED_{50}$ [mg/kg, s.c.] | | |
|---|---|---|---|
| | Staphylococcus anvens 10B | Escherichia coli 205 | Proteus morganii 2359 |
| 6 | 20 | 5.5 | 10 |
| 7 | 10 | 30 | 85 |
| 8 | >30 | 6 | 10 |
| 9 | >30 | >30 | >30 |
| 10 | >30 | >16 | 12 |
| 11 | 30 | 10 | 5.5 |
| 12 | 30 | 1 | 35 |
| 13 | >30 | 0.2 | 13 |
| 14 | 20 | 15 | 17 |
| 15 | >30 | 12 | 10 |
| 16 | >30 | 1 | 70 |
| 17 | 30 | 0.4 | 15 |
| 18 | >30 | n.g. | 14 |
| 19 | >30 | n.g. | 22 |
| 20 | >30 | n.g. | 25 |
| 21 | >30 | 5.5 | 5 |
| 22 | >30 | <1 | 3-10 |
| 23 | >30 | 13 | 50 |
| 24 | >30 | <1 | 5 |
| 25 | >30 | 0,28 | ca. 3 |
| 26 | 15 | 9 | 10 |
| 27 | >30 | 14 | 8 |
| 28 | >30 | 4 | 5 |
| 29 | >30 | 7 | 75 |
| 30 | >30 | 1,7 | 10 |

Verbindungen der Formel I, worin die funktionellen Gruppen geschützt sind, werden als Ausgangsmaterialien zur Herstellung von Verbindungen der Formel I verwendet, worin funktionelle Gruppen in freier Form oder in physiologisch spaltbarer Form vorliegen.

Die vorliegende Erfindung betrifft bevorzugt solche Verbindungen der Formel I, worin funktionelle Gruppen in freier Form oder in physiologisch spaltbarer geschützter Form vorliegen, und deren pharmazeutisch annehmbare Salze, da hauptsächlich diese Verbindungen die angegebene Wirksamkeit besitzen und für den angegebenen Zweck verwendet werden können.

Hervorzuheben sind solche Verbindungen der Formel I, worin $\ell$ eine ganze Zahl von 0 bis 2, m eine ganze Zahl von 0 (direkte Bindung) bis 4, n eine ganze Zahl von 1 bis 4, die Gruppen $-(C_mH_{2m})-$ und $-(C_nH_{2n})-$ unverzweigt sind, X Sauerstoff oder die Gruppe -NH-, W die Gruppe -CO- oder X-W- zusammen die Gruppe -CO-, Y Wasserstoff, Niederalkyl, z.B. Methyl, unsubstituiertes oder durch Amino substituiertes Furyl, Thienyl, Thiazolyl oder Thiadiazolyl, z.B. Furyl, Thienyl, Aminothiazolyl oder Aminothiadiazolyl, $R_1$ Wasserstoff, Niederalkoxy, z.B. Methoxy oder Aethoxy, Halogen, z.B. Chlor, oder eine Gruppe der Formel $-CH_2-R_2$, worin $R_2$ Niederalkanoyloxy, z.B. Acetyloxy, Carbamoyloxy, N-Niederalkylcarbamoyloxy, unsubstituiertes oder durch Niederalkyl, z.B. Methyl, Diniederalkylaminoniederalkyl, z.B. Dimethylaminomethyl, Carboxyniederalkyl, z.B. Carboxymethyl, Amino, Carboxyniederalkylamino, z.B. 2-Carboxyäthylamino oder Carbamoyl substituiertes Triazolylthio, z.B. 1H-1,2,3-Triazol-5-ylthio, Tetrazolylthio, z.B. 1H-Tetrazol-5-ylthio, Thiazolylthio, Thiadiazolylthio, z.B. 1,3,4-Thiadiazol-5-ylthio, Oxazolylthio, Oxadiazolylthio oder 5,6-Dioxotetrahydro-as-triazinylthio, z.B. 5,6-Dioxo-1,2,5,6-etrahydro-as-triazin-3-ylthio oder 5,6-Dioxo-1,4,5,6-tetrahydro-as-triazin-3-ylthio, 2-Niederalkyl-1-pyrazolium, z.B. 2-Methyl-1-pyrazolium, 2-Carboxyniederalkyl-1-pyrazolium, z.B. 2-Carboxymethyl-1-pyrazolium, 3-Niederalkyl-1-triazolium, z.B. 3-Methyl-1-triazolium, oder unsubstituiertes oder durch Hydroxyniederalkyl, z.B. Hydroxymethyl,

Carboxy, Carboxyniederalkyl, z.B. Carboxymethyl, Halogen, z.B. Chlor oder Brom oder Carbamoyl substituiertes Pyridinium, z.B. 3- oder 4-Hydroxymethylpyridinium, 4-Carboxypyridinium, 3- oder 4-Carboxymethylpyridinium, 3- oder 4-Brompyridinium oder 3- oder 4-Carbamoylpyridinium, $R_3$ Wasserstoff oder Niederalkoxy, z.B. Methoxy, und $R_4$ Carboxyl, Pivaloyloxymethoxycarbonyl oder 1-Aethoxycarbonyloxyäthoxycarbonyl bedeuten, Hydrate und pharmazeutisch verwendbare Salze von solchen Verbindungen.

Besonders hervorzuheben sind solche Verbindungen der Formel I, worin $\ell$ 0, m eine ganze Zahl von 0 (direkte Bindung) bis 4, n eine ganze Zahl von 1 bis 4 , die Gruppen $-(C_mH_{2m})-$ und $-(C_nH_{2n})-$ unverzweigt sind, X Sauerstoff oder die Gruppe -NH-, W die Gruppe -CO- oder X-W zusammen die Gruppe -CO-, Y Wasserstoff, Niederalkyl, z.B. Methyl, Furyl, z.B. 2- oder 3-Furyl, Thienyl, z.B. 2- oder 3-Thienyl, Aminothiazolyl, z.B. 2-Aminothiazol-4-yl, oder Aminothiadiazolyl, z.B. 5-Amino-1,2,4-thiadiazol-3-yl, $R_1$ Wasserstoff, Niederalkoxy, z.B. Methoxy, Halogen, z.B. Chlor, oder eine Gruppe der Formel $-CH_2-R_2$, worin $R_2$ Niederalkanoyloxy, z.B. Acetyloxy, Carbamoyloxy, unsubstituiertes oder durch Niederalkyl, z.B. Methyl, Diniederalkylaminoniederalkyl, z.B. 2-Dimethylaminoäthyl, Sulfoniederalkyl, z.B. Sulfomethyl, Carboxyniederalkyl, z.B. Carboxymethyl, oder durch Carbamoyl substituiertes Triazolylthio, z.B. 1H-1,2,3-Triazol-5-ylthio, Tetrazolylthio, z.B. 1H-Tetrazol-5-ylthio, 1-Methyl-1H-tetrazol-5-ylthio, 1-Sulfomethyl-1H-tetrazol-5-ylthio, 1-Carboxymethyl-1H-tetrazol-5-ylthio oder 1-(2-Dimethylaminoäthyl)-1H-tetrazol-5-ylthio, Thiadiazolylthio, z.B. 1,3,4-Thiadiazol-5-ylthio oder 2-Methyl-1,3,4-thiadiazol-5-ylthio, 5,6-Dioxotetrahydrotriazin-3-ylthio, z.B. 2-Methyl-5,6-dioxo-1,2,5,6-tetrahydro-as-triazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, oder unsubstituiertes oder durch Hydroxyniederalkyl, z.B. Hydroxymethyl, Carboxy, Carboxyniederalkyl, z.B. Carboxymethyl, Halogen, z.B. Chlor oder Brom, oder

Carbamoyl substituiertes Pyridinium, z.B. 3- oder 4-Hydroxymethylpyridinium, 4-Carboxypyridinium, 3- oder 4-Carboxymethylpyridinium, 3- oder 4-Chlorpyridinium, 3- oder 4-Carboxymethylpyridinium, 3- oder 4-Chlorpyridinium, 3- oder 4-Brompyridinium oder 3- oder 4-Carbamoylpyridinium bedeutet, $R_3$ Wasserstoff oder Methoxy und $R_4$ Carboxyl bedeuten, Hydrate und pharmazeutisch verwendbare Salze von solchen Verbindungen.

Bevorzugt sind Verbindungen der Formel I, worin $\ell$ 0, m eine ganze Zahl von 0 (direkte Bindung) bis 3, n 1 oder 2, die Gruppe $-(C_mH_{2m})-$ unverzweigt ist, X Sauerstoff, W die Gruppe -CO- oder X-W zusammen die Gruppe -CO-, Y Wasserstoff, Niederalkyl, z.B. Methyl, Furyl, z.B. 2- oder 3-Furyl, Aminothiazolyl, z.B. 2-Aminothiazol-4-yl, oder Aminothiadiazolyl, z.B. 5-Amino-1,2,4-thiadiazol-3-yl, $R_1$ Wasserstoff, Niederalkoxy, z.B. Methoxy, Halogen, z.B. Chlor, oder eine Gruppe der Formel $CH_2-R_2$, worin $R_2$ Niederalkanoyloxy, z.B. Acetyloxy, Carbamoyloxy, Tetrazolylthio, z.B. 1H-Tetrazol-5-ylthio, durch Niederalkyl, z.B. Methyl, Diniederalkylaminoniederalkyl, z.B. Dimethylaminoäthyl, Sulfoniederalkyl, z.B. Sulfomethyl, oder Carboxyniederalkyl, z.B. Carboxymethyl substituiertes Tetrazolylthio, z.B. 1-Methyl-1-tetrazol-5-ylthio, 1-(2-Dimethylaminoäthyl)-1H-tetrazol-5-ylthio, 1-Sulfomethyl-1H-tetrazol-5-ylthio, oder 1-Carboxymethyl-1H-tetrazol-5-ylthio, Thiadiazolylthio, z.B. 1,3,4-Thiadiazol-5-ylthio, oder durch Niederalkyl, z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxo-tetrahydro-as-triazinylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinium oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinium, z.B. 3- oder 4-Hydroxymethylpyridinium, 4-Carboxypyridinium, 3- oder 4-Carboxymethylpyridinium, 3- oder 4-Chlorpyridinium, 3- oder 4-Brompyridinium oder 3- oder 4-Carbamoylpyridinium bedeutet, $R_3$ Wasserstoff oder Methoxy und $R_4$

Carboxyl bedeuten, Hydrate und pharmazeutisch verwendbare Salze von solchen Verbindungen.

Die Erfindung betrifft insbesondere die in den Beispielen beschriebenen Verbindungen der Formel I, deren pharmazeutisch verwendbare Salze, sowie die dort beschriebenen Ausgangsstoffe und Zwischenprodukte.

Die Erfindung betrifft vor allem die im Versuchsbericht aufgezählten pharmazeutisch verwendbaren Salze von Verbindungen der Formel I bzw. deren Enantiomere.

Herstellungsverfahren:

Verbindungen der Formel I, worin die Carboxylgruppen in freier Form vorliegen oder in physiologisch spaltbarer Form verestert sind, Hydrate und Salze von solchen Verbindungen, die eine salzbildende Gruppe aufweisen, werden beispielsweise hergestellt, indem man

a) in einer Verbindung der Formel

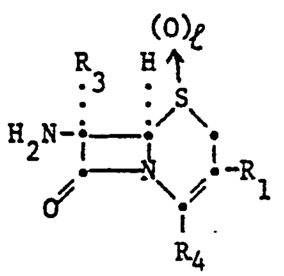

(II),

worin $\ell$, $R_1$, $R_3$ und $R_4$ die unter Formel I genannten Bedeutungen haben und die 7β-Aminogruppe gegebenenfalls durch eine die Acylierung erlaubende Gruppe geschützt ist, durch Umsetzung mit einer Carbonsäure der Formel

$$HOOC-\underset{\displaystyle |}{\overset{NH_2}{CH}}-(C_nH_{2n})-X-W-NH-(C_mH_{2m})-\overset{Y}{\underset{}{CH}}-COOH \qquad \text{(III)},$$

worin m und n und X, W und Y die unter Formel I genannten Bedeutungen haben und die α-Aminocarbonsäuregruppierung $HOOC-CH(NH_2)-$ und in der Gruppe Y vorhandene funktionelle Gruppen in geschützter Form vorliegen, oder mit einem reaktionsfähigen funktionellen Säurederivat oder einem Salz davon acyliert, oder

b) in einer Verbindung der Formel

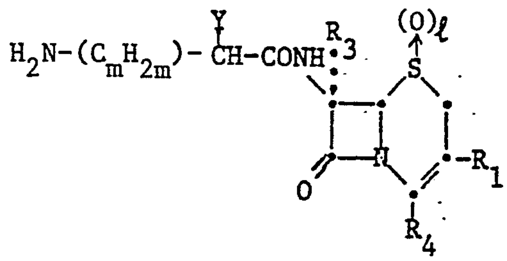 (IV) ,

worin ℓ, m, Y, $R_1$ und $R_4$ die unter Formel I genannten Bedeutungen haben und die α-Aminogruppe gegebenenfalls durch eine die Acylierungsreaktion erlaubende Gruppe geschützt ist und in der Gruppe Y vorhandene funktionelle Gruppen gegebenenfalls in geschützter Form vorliegen, die Aminogruppe durch Umsetzung mit einem reaktionsfähigen funktionellen Derivat einer Säure der Formel

$$HOOC-\underset{NH_2}{CH}-(C_nH_{2n})-X-W-OH \qquad (V),$$

worin n, X und W die unter Formel I genannten Bedeutungen haben und die α-Aminocarbonsäuregruppierung $HOOC-CH(NH_2)-$ in geschützter Form vorliegt, oder, wenn X-W zusammen die Gruppe -CO- bedeutet, auch mit einer entsprechenden freien Säure oder mit einem Salz davon acyliert, oder

c) in einer Verbindung der Formel

$$HOOC-\underset{NH_2}{CH}-(C_nH_{2n})-X-H \qquad (VI),$$

worin n und X die unter Formel I genannten Bedeutungen haben und die α-Aminocarbonsäuregruppierung $HOOC-CH(NH_2)-$ in geschützter Form vorliegt, die Gruppe -X-H durch Umsetzung mit einem reaktionsfähigen funktionellen Derivat einer Säure der Formel

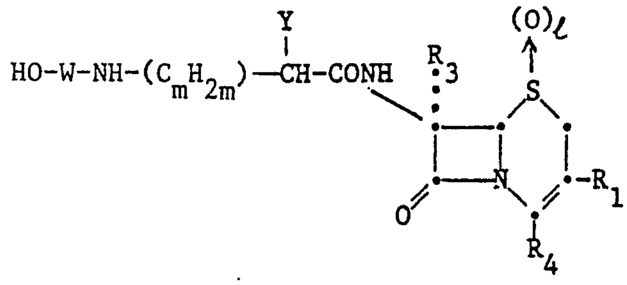

(VII),

worin $\ell$, m, W, Y, $R_1$, $R_3$ und $R_4$ die unter Formel I genannten Bedeutungen haben und in der Gruppe Y vorhandene funktionelle Gruppen gegebenenfalls in geschützter Form vorliegen, acyliert, oder

d) eine 2-Cephemverbindung der Formel

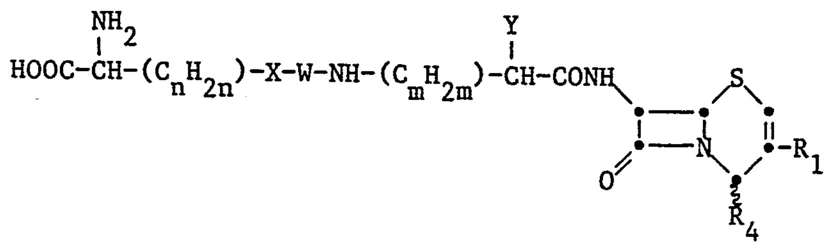

(VIII),

worin m, n, X, W, Y, $R_1$, $R_3$ und $R_4$ die unter Formel I genannten Bedeutungen haben und die Aminocarbonsäuregruppierung $HOOC-CH(NH_2)-$ und in der Gruppe Y vorhandene funktionelle Gruppen in geschützter Form vorliegen, zur entsprechenden 3-Cephemverbindung der Formel I isomerisiert und, wenn erwünscht, eine erfindungsgemäss erhältliche Verbindung der Formel I in eine andere Verbindung der Formel I umwandelt und/oder, wenn erwünscht, eine erfindungsgemäss erhältliche Verbindung der Formel I, worin 0 bedeutet, in eine Verbindung der Formel I überführt, worin $\ell$ 1 oder 2 bedeutet, und/oder eine Verbindung der Formel I, worin $\ell$ 1 oder 2 bedeutet, in eine Verbindung der Formel I überführt, worin $\ell$ 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie

Verbindung mit einer salzbildenden Gruppe in ein Salz überführt und/oder ein erhältliches Gemisch von isomeren Verbindungen der Formel I in die einzelnen Isomeren auftrennt.

Verfahren a), b) und c) (Acylierungen):

In Ausgangsmaterialien der Formeln II und IV sind die Aminogruppen gegebenenfalls durch die Acylierungsreaktion erlaubende Gruppen gegebenenfalls geschützt. Solche Gruppen sind beispielsweise organische Silylgruppen, ferner Ylidengruppen, die zusammen mit der Aminogruppe eine Schiff'sche Base bilden. Die genannten organischen Silylgruppen sind z.B. solche Gruppen, die auch mit einer Carboxylgruppe $R_4$ eine geschützte Carboxylgruppe zu bilden vermögen. Es sind dies in erster Linie Triniederalkylsilyl, insbesondere Trimethylsilyl. Bei der Silylierung zum Schutz einer 4-Carboxylgruppe in einem Ausgangsmaterial der Formel II oder V kann bei Verwendung eines Ueberschusses des Silylierungsmittels die Aminogruppe ebenfalls silyliert werden. Die genannten Ylidengruppen sind in erster Linie 1-Arylniederalkyliden-, insbesondere 1-Arylmethylengruppen, worin Aryl besonders für einen carbocyclischen, in erster Linie monocyclischen Arylrest steht, z.B. für gegebenenfalls durch Niederalkyl, Hydroxy, Niederalkoxy und/oder Nitro substituiertes Phenyl.

Die übrigen in den Ausgangsmaterialien der Formeln II-VII vorhandenen funktionellen Gruppen können z.B. durch die unter den Verbindungen der Formel II genannten Schutzgruppen geschützt sein. Vorzugsweise sind die an der Acylierungsreaktion nicht teilnehmenden funktionellen Gruppen, insbesondere gegebenenfalls vorhandene acylierbare Amino-, Hydroxy- und Mercaptogruppen, entsprechend geschützt.

In Verbindungen der Formeln III, IV, V, VI oder VII kann eine vorhandene Aminogruppe auch in ionischer Form geschützt sein, d.h. das entsprechende Ausgangsmaterial mit einer solchen Aminogruppe kann in Form eines Säureadditionssalzes, vorzugsweise mit einer starken anorganischen Säure, wie einer Halogenwasserstoffsäure, z.B. Salzsäure

oder Schwefelsäure, oder mit einer organischen Säure, z.B. p-Toluolsulfonsäure, verwendet werden.

Falls eine freie Säure der Formel III, worin vorhandene funktionelle Gruppen ausser der an der Reaktion teilnehmenden Carboxylgruppe geschützt sein können, oder eine freie Säure der Formel V, worin X-W die Gruppe -CO- bedeutet, zur Acylierung eingesetzt wird, wird die Reaktion üblicherweise in Gegenwart von geeigneten Kondensationsmitteln, wie Carbodiimiden, beispielsweise N,N'-Diäthyl-, N,N'-Dipropyl-, N,N'-Dicyclohexyl- oder N-Aethyl-N'-3-dimethylaminopropylcarbodiimid, geeigneten Carbonylverbindungen, beispielsweise Carbonyldiimidazol, oder 1,2-Oxazoliumverbindungen, wie 2-Aethyl-5-phenyl-1,2-oxazolium-3'-sulfonat oder 2-tert.-Butyl-5-methyl-1,2-oxazoliumperchlorat, oder einer geeigneten Acylaminoverbindung, z.B. 2-Aethoxy-1-äthoxycarbonyl-1,2-dihydro-chinolin, durchgeführt.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen, z.B. in einem Temperaturbereich von etwa -40°C bis etwa +100°C, bevorzugt von etwa -20°C bis etwa +50°C,und/oder in einer Inertgas-, z.B. Stickstoffatmosphäre, durchgeführt.

Ein reaktionsfähiges, d.h. Amid-bildendes, bzw. Ester-bildendes, funktionelles Derivat von Säuren der Formeln III, V oder VII, worin vorhandene funktionelle Gruppen ausser der an der Reaktion beteiligten Carboxylgruppe geschützt sein können, ist in erster Linie ein Anhydrid einer solchen Säure, inklusive und vorzugsweise ein gemischtes Anhydrid. Gemischte Anhydride sind z.B. diejenigen mit anorganischen Säuren wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure, Diäthylphosphorsäure oder phosphoriger Säure, oder mit einer schwefelhaltigen Säure, z.B. Schwefelsäure oder mit

Cyanwasserstoffsäure. Weitere gemischte Anhydride sind z.B. diejenigen, welche mit organischen Carbonsäuren gebildet werden, z.B. mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Weitere zur Reaktion mit der entsprechenden Amino-, Hydroxy- oder Mercaptogruppe geeignete Derivate von Säuren der Formeln III, V oder VII, worin vorhandene funktionelle Gruppen ausser der an der Reaktion teilnehmenden Carboxylgruppe geschützt sein können, sind aktivierte Ester, beispielsweise Ester mit vinylogen Alkoholen, d.h. mit Enolen wie vinylogen Niederalkenolen oder Iminomethylesterhalogeniden, z.B. Dimethyliminomethylesterchlorid, hergestellt aus der Carbonsäure und z.B. Dimethyl-(1-chlor-äthyliden)-iminiumchlorid der Formel $[(CH_3)_2N^{\oplus}=C(Cl)CH_3]Cl^{\ominus}$, das man seinerseits z.B. aus N,N-Dimethylacetamid und Phosgen oder Oxalylchlorid erhalten kann, Arylester, z.B. durch Halogen, wie Chlor und/oder Nitro, substituierte Phenylester, z.B. 4-Nitro-phenyl-, 2,3-Dinitrophenyl- oder Pentachlorphenylester, N-heteroaromatische Ester, wie N-Benztriazol-, z.B. 1-Benztriazolester, oder N-Diacyliminoester, wie N-Succinylimino- oder N-Phthalyliminoester.

Die Acylierung mit einem reaktionsfähigen funktionellen Derivat von Säuren der Formeln III, V oder VII, z.B. mit einem entsprechenden Anhydrid, insbesondere einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkylanilin, z.B. N,N-Dimethylanilin, oder eines cyclischen tertiären Amins, wie eines N-niederalkylierten

Morpholins, wie N-Methylmorpholin, oder einer Base vom Pyridin-Typ, z.B. Pyridin, einer anorganischen Base, wie beispielsweise eines Alkalimetall- der Erdalkalimetallhydroxids, -carbonats- oder -hydrogencarbonats, z.B. Natrium-, Kalium- oder Calciumhydroxid, -carbonat oder -hydrogencarbonat, oder eines Oxirans, beispielsweise eines 1,2-Niederalkylenoxids, wie Aethylenoxid oder Propylenoxid, durchgeführt.

Die obigen Acylierungen werden vorzugsweise in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, cyclischem Aether, z.B. Tetrahydrofuran, einem Ester, z.B. Essigsäureäthylester, oder einem Nitril, z.B. Acetonitril, oder Mischungen davon, wenn notwendig oder erwünscht, bei erniedrigter oder erhöhter Temperatur, z.B. in einem Temperaturbereich von etwa -40°C bis etwa +100°C, bevorzugt von etwa -10°C bis etwa +50°C, und/oder in einer Inertgas-, z.B. Stickstoffatmosphäre.

Die Acylierung einer Verbindung der Formel II kann auch durch Verwendung eines geeigneten Derivates der Säure der Formel III in Gegenwart einer geeigneten Acylase erfolgen. Solche Acylasen sind bekannt und können durch eine Anzahl von Mikroorganismen gebildet werden, z.B. durch Acetobacter, wie Acetobacter aurantium, Achromobacter, wie Achromobacter aeris, Aeromonas, wie Aeromonas hydrophila, oder Bacillus, wie Bacillus megaterium 400. In einer solchen enzymatischen Acylierung werden insbesondere Amide, Ester oder Thioester, wie Niederalkyl-, z.B. Methyl- oder Aethylester, der Carbonsäure der Formel III als entsprechende Derivate eingesetzt. Ueblicherweise wird eine solche Acylierung in einem den entsprechenden Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden Medium,

z.B. in einem Temperaturbereich von etwa +20°C bis etwa +40°C, bevorzugt bei etwa +37°C, durchgeführt.

Ein in in der Acylierungsreaktion eingesetztes reaktionsfähiges funktionelles Derivat einer Säure der Formel III kann, wenn erwünscht, in situ gebildet werden. So kann man z.B. ein gemischtes Anhydrid herstellen, indem man eine Säure der Formel III oder eine Säure der Formel V, worin X-W die Gruppe -CO- bedeutet, gegebenenfalls mit entsprechend geschützten funktionellen Gruppen oder ein geeignetes Salz davon, z.B. ein Ammoniumsalz, welches z.B. mit einer organischen Base, wie Pyridin oder 4-Methylmorpholin gebildet wird, oder ein Metall-, z.B. Alkalimetallsalz, mit einem geeigneten Säurederivat, beispielsweise einem entsprechenden Säurehalogenid, einer gegebenenfalls substituierten Niederalkancarbonsäure, z.B. Trichloracetylchlorid, einem Halbester eines Kohlensäurehalbhalogenids, z.B. Chlorameisensäureäthylester, oder -isobutylester, oder mit einem Halogenid einer Diniederalkylphosphorsäure, z.B. Diäthylphosphorbromidat, das man durch Umsetzen von Triäthylphosphit mit Brom bilden kann, umsetzt. Das so erhältliche gemischte Anhydrid lässt sich ohne Isolierung in der Acylierungsreaktion verwenden.

Ein Säurechlorid einer Säure der Formel V, worin X-W eine Gruppierung -O-CO-, -S-CO oder -NH-CO- ist und worin die Aminocarbonsäuregruppierung HOOC-CH($NH_2$)- in geschützter Form vorliegt, kann z.B. in situ gebildet werden, indem man eine Aminocarbonsäure der Formel VI, worin X Sauerstoff, Schwefel oder die Gruppe -NH- bedeutet, und die Gruppierung HOOC-CH($NH_2$)- in geschützter Form vorliegt, in Gegenwart eines Salzsäureakzeptors in einem inerten organischen Lösungsmittel oder Lösungsmittelgemisch mit Phosgen behandelt. Die Salzsäureakzeptoren, Lösungsmittel und Reaktionsbedingungen sind die gleichen, die für die Acylierung von Verbindungen der Formel II oder IV genannt sind; beispielsweise kann die Reaktion in Gegenwart von Pyridin in Methylenchlorid und Toluol bei etwa 0° bis etwa +10°C stattfinden.

Auf die gleiche Weise kann, z.B. in situ, ein Säurechlorid einer Säure der Formel VII, worin W die Gruppe $-SO_2NH-CO-$ bedeutet und die 4-Carboxylgruppe und in den Gruppen Y und $R_1$ vorhandene funktionelle Gruppen geschützt sind, aus einer entsprechend geschützten Verbindung der Formel IV durch Behandlung mit Chlorsulfonylisocyanat hergestellt werden.

Verfahren d) (Isomerisierung):

In einem 2-Cephem-Ausgangsmaterial der Formel VIII weist die gegebenenfalls geschützte 4-Carboxylgruppe vorzugsweise die α-Konfiguration auf.

2-Cephem-Verbindungen der Formel VIII können isomerisiert werden, indem man sie mit einem schwach-basischen Mittel behandelt und die entsprechende 3-Cephem-Verbindung isoliert. Geeignete Isomerisierungsmittel sind z.B. organische, stickstoffhaltige Basen, insbesondere tertiäre heterocyclische Basen aromatischen Charakters, in erster Linie Basen des Pyridin-Typs, wie Pyridin selber, sowie Picoline, Collidine oder Lutidine, ferner Chinolin, tertiäre aromatische Basen, z.B. solche des Anilin-Typs, wie N,N-Diniederalkylaniline, z.B. N,N-Dimethylanilin oder N,N-Diäthylanilin, oder tertiäre aliphatische, azacycloaliphatische oder araliphatische Basen, wie N,N,N-Triniederalkylamine, z.B. N,N,N-Trimethylamin oder N,N-Diisopropyl-N-äthylamin, N-Niederalkyl-azacycloalkane, z.B. N-Methyl-piperidin, oder N-Phenylniederalkyl-N,N-diniederalkyl-amine, z.B. N-Benzyl-N,N-dimethylamin, sowie Gemische von solchen basischen Mitteln, wie das Gemisch einer Base vom Pyridintyp, und eines N,N,N-Tri-niederalkylamins, z.B. Pyridin und Triäthylamin. Ferner können auch anorganische oder organische Salze von Basen, insbesondere von mittelstarken bis starken Basen mit schwachen Säuren, wie Alkalimetall- oder Ammoniumsalze von Niederalkancarbonsäuren, z.B. Natriumacetat, Triäthylammoniumacetat oder N-Methyl-piperidinacetat, sowie andere analoge Basen oder Gemische von solchen basischen Mitteln verwendet werden.

Bei der Isomerisierung von 2-Cephem-Verbindungen der Formel VIII mit basischen Mitteln arbeitet man vorzugsweise in wasserfreiem Medium, in An- oder Abwesenheit eines Lösungsmittels, wie eines gegebenenfalls halogenierten, z.B. chlorierten, aliphatischen, cycloaliphatischen oder aromatischen Kohlenwasserstoffs, oder eines Lösungsmittelgemisches, wobei als Reaktionsmittel verwendete, unter den Reaktionsbedingungen flüssige Basen gleichzeitig auch als Lösungsmittel dienen können, gegebenenfalls unter Kühlen oder unter Erwärmen, vorzugsweise in einem Temperaturbereich von etwa -30°C bis etwa +100°C, in einer Inertgas-, z.B. Stickstoffatmosphäre, und/oder in einem geschlossenen Gefäss.

So erhältliche 3-Cephem-Verbindungen der Formel I lassen sich in an sich bekannter Weise, z.B. durch Adsorptionschromatographie und/oder Kristallisation, von gegebenenfalls noch vorhandenen 2-Cephem-Ausgangsstoffen abtrennen.

Die Isomerisierung von 2-Cephem-verbindungen der Formel VIII zur entsprechenden 3-Cephem-verbindung wird vorzugsweise durchgeführt, indem man diese in 1-Stellung oxidiert, wenn erwünscht, gegebenenfalls Isomerengemische der gebildeten 1-Oxide trennt, und die so erhältlichen 1-Oxide der entsprechenden 3-Cephemverbindungen reduziert.

Als geeignete Oxidationsmittel für die Oxidation in 1-Stellung von 2-Cephem-verbindungen der Formel IV kommen anorganische Persäuren, die ein Reduktionspotential von wenigstens +1,5 Volt aufweisen und aus nicht-metallischen Elementen bestehen, organische Persäuren oder Gemische aus Wasserstoffperoxid und Säuren, insbesondere organische Carbonsäuren, mit einer Dissoziationskonstante von wenigstens $10^{-5}$ in Frage. Geeignete anorganische Persäuren sind Perjod- und Perschwefelsäure. Organische Persäuren sind entsprechende Percarbon- und Persulfonsäuren, die als solche zugesetzt oder durch Verwendung von wenigstens einem Aequivalent Wasserstoffperoxyd und

einer Carbonsäure in situ gebildet werden können. Dabei ist es zweckmässig, einen grossen Ueberschuss der Carbonsäure anzuwenden, wenn z.B. Essigsäure als Lösungsmittel verwendet wird. Geeignete Persäuren sind z.B. Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

Die Oxidation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1-2% und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxid mit Essigsäure, Perchlorsäure oder Trifluoressigsäure.

Die obige Oxidation kann in Gegenwart von geeigneten Katalysatoren durchgeführt werden. So kann z.B. die Oxidation mit Percarbonsäuren durch die Anwesenheit einer Säure mit einer Dissoziationskonstante von wenigstens $10^{-5}$ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxidationsmittels, vorzugsweise einen geringen Ueberschuss von etwa 10% bis etwa 20%, wobei man auch grössere Ueberschüsse, d.h. bis zur 10-fachen Menge des Oxidationsmittels oder darüber verwenden kann. Die Oxidation wird unter milden Bedingungen, z.B. bei Temperaturen von etwa -50°C bis etwa +100°C, vorzugsweise von etwa -10°C bis etwa +40°C durchgeführt.

Die Reduktion der 1-Oxide von 3-Cephem-Verbindungen kann in an sich bekannter Weise durch Behandeln mit einem Reduktionsmittel, wenn notwendig, in Anwesenheit eines aktivierenden Mittels, durchgeführt werden. Als Reduktionsmittel kommen beispielsweise in Betracht:

Katalytisch aktivierter Wasserstoff, wobei Edelmetallkatalysatoren verwendet werden, welche Palladium, Platin oder Rhodium enthalten, und die man gegebenenfalls zusammen mit einem geeigneten Trägermaterial, wie Kohle oder Bariumsulfat, einsetzt; reduzierende Zinn-, Eisen-, Kupfer- oder Mangankationen, welche in Form von entsprechenden Verbindungen oder Komplexen anorganischer oder organischer Art, z.B. als Zinn-II-chlorid, -fluorid,-acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanid-anionen, welche in Form von entsprechenden anorganischen oder organischen Salzen, wie Alkalimetall-, z.B. Natrium- oder Kaliumdithionit, Natrium- oder Kaliumjodid oder Kaliumeisen-II-cyanid, oder in Form der entsprechenden Säuren, wie Jodwasserstoffsäure, verwendet werden; reduzierende trivalente anorganische oder organische Phosphorverbindungen, wie Phosphine, ferner Ester, Amide und Halogenide der phosphonigen, phosphinigen oder phosphorigen Säure, sowie diesen Phosphorsauerstoffverbindungen entsprechende Phosphor-Schwefelverbindungen, worin organische Reste in erster Linie aliphatische, aromatische oder araliphatische Reste, z.B. gegebenenfalls substituiertes Niederalkyl, Phenyl oder Phenylniederalkyl darstellen, wie z.B. Triphenylphosphin, Tri-n-butylphosphin, Diphenylphosphonigsäuremethylester, Diphenylchlorphosphin, Phenyldichlorphosphin, Benzolphosphonigsäuredimethylester, Butanphosphonigsäuremethylester, Phosphorsäuretriphenylester, Phosphorigsäuretrimethylester, Phosphortrichlorid, Phosphortribromid, ferner Phosphorigsäuretriphenylester-Halogenaddukte, z.B. Chlor- oder Bromaddukte, worin die Phenylreste gegebenenfalls durch Niederalkyl, z.B. Methyl, Niederalkoxy, z.B. Methoxy, oder durch Halogen, z.B. Chlor, substituiert sind, etc.; reduzierende Halogensilanverbindungen, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenenfalls substituiertes Niederalkyl oder Phenyl,

aufweisen können, wie Chlorsilan, Bromsilan, Di- oder Trichlorsilan, Di- oder Tribromsilan, Diphenylchlorsilan, oder Dimethylchlorsilan, sowie auch Halogensilanverbindungen, worin alle Wasserstoffe durch organische Reste ersetzt sind, wie Triniederalkylhalogensilan, z.B. Trimethylchlorsilan oder Trimethyljodsilan, oder cyclische, Schwefel enthaltende Silane, wie 1,3-Dithia-2,4,-disilacyclobutane oder 1,3,5-Trithia-2,4,6-trisila-cyclohexane, deren Siliciumatome durch Kohlenwasserstoffreste, wie insbesondere Niederalkyl substituiert sind, z.B. 2,2,4,4-Tetramethyl-1,3-dithia-2,4-disilacyclobutan oder 2,2,4,4,6,6-Hexamethyl-1,3,5-trithia-2,4,6-trisilacyclohexan, etc.; reduzierende quaternäre Chlormethylen-iminiumsalze, insbesondere -chloride oder -bromide, worin die Iminiumgruppe durch einen bivalenten oder zwei monovalente organische Reste, wie gegebenenfalls substituiertes Niederalkylen bzw. Niederalkyl substituiert ist, wie N-Chlormethylen-N,N-diäthyliminiumchlorid oder N-Chlormethylen-pyrrolidiniumchlorid; oder komplexe Metallhydride, wie Natriumborhydride, in Gegenwart von geeigneten Aktivierungsmitteln, wie Cobalt-II-chlorid, sowie Borandichlorid.

Als aktivierende Mittel, die zusammen mit denjenigen der obengenannten Reduktionsmittel verwendet werden, welche selber nicht Lewissäure-Eigenschaften aufweisen, d.h. in erster Linie zusammen mit den Dithionit-, Jod- oder Eisen-II-cyanid- und den nicht-halogenhaltigen trivalenten Phosphor-Reduktionsmitteln oder bei der katalytischen Reduktion eingesetzt werden, sind insbesondere organische Carbon- und Sulfonsäurehalogenide, ferner Schwefel-, Phosphor- oder Siliciumhalogenide mit gleicher oder grösserer Hydrolysenkonstante zweiter Ordnung als Benzoylchlorid, z.B. Phosgen, Oxalylchlorid, Essigsäurechlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsäuredichlorid, Dimethylchlorsilan oder Trichlorsilan, ferner geeignete Säureanhydride,

wie Trifluoressigsäureanhydrid, oder cyclische Sultone, wie Aethansulton, Propansulton, 1,3-Butansulton oder 1,3-Hexansulton zu erwähnen.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte, aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20°C bis etwa 100°C, wobei bei Verwendung von sehr reaktionsfähigen Aktivierungsmitteln die Reaktion auch bei tieferen Temperaturen durchgeführt werden kann.

Nachoperationen:

In einer erhaltenen Verbindung der Formel I können auf übliche an sich bekannte Weise noch nicht geschützte funktionelle Gruppen geschützt oder vorhandene Schutzgruppen gegen andere Schutzgruppen ausgetauscht werden, z.B. durch Abspalten der vorhandenen Schutzgruppe und Einführen der gewünschten anderen Schutzgruppe.

$R_1$-Umwandlungen:

In einer erhaltenen Verbindung der Formel I, worin funktionelle Gruppen gegebenenfalls geschützt sind, kann man in an sich bekannter Weise eine Gruppe $R_1$ durch einen anderen Rest $R_1$ ersetzen oder in einen anderen Rest $R_1$ umwandeln. So ist es z.B. möglich, in einer Verbindung der Formel I, worin $R_1$ eine Gruppe der Formel $-CH_2-R_2$ bedeutet und $R_2$ z.B. einen, durch nucleophile Substituenten ersetzbaren Rest darstellt, oder in einem Salz davon durch Behandeln mit einer entsprechenden Mercaptan- oder mit einer Thiocarbonsäureverbindung einen solchen Rest $R_2$ durch eine verätherte bzw. veresterte Mercaptogruppe $R_2$ zu ersetzen.

Ein geeigneter, durch eine verätherte Mercaptogruppe ersetzbarer Rest ist beispielsweise eine durch eine niederaliphatische Carbonsäure veresterte Hydroxygruppe. Solche veresterten Hydroxygruppen sind insbesondere Acetyloxy und Acetoacetoxy.

Die Reaktion einer solchen Verbindung der Formel I mit einer geeigneten Mercaptanverbindung kann unter sauren, neutralen oder schwach basischen Bedingungen durchgeführt werden. Bei sauren Bedingungen arbeitet man in Gegenwart von konzentrierter Schwefelsäure, welche gegebenenfalls durch ein anorganisches Lösungsmittel, z.B. Polyphosphorsäure, verdünnt wird. Bei neutralen oder schwach basischen Bedingungen führt man die Reaktion in Gegenwart von Wasser und gegebenenfalls einem mit Wasser mischbaren organischen Lösungsmittel durch.

Die basischen Bedingungen können beispielsweise durch Zugabe einer anorganischen Base, wie eines Alkalimetall- oder Erdalkalimetallhydroxids, -carbonats oder -hydrogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, oder Nitrile, z.B. Niederalkansäurenitrile, wie Acetonitril, und ähnliche verwendet werden.

Veresterte Hydroxygruppen $R_2$ in einer Verbindung der Formel I, worin $R_1$ die Gruppe $-CH_2-R_2$ bedeutet, wobei $R_2$ für eine, durch den Acylrest eines gegebenenfalls substituierten Halbamids der Kohlensäure veresterte Hydroxygruppe steht, kann man z.B. einführen, indem man eine entsprechende Verbindung der Formel I, worin $R_2$ für freies Hydroxy steht (das man z.B. durch Abspaltung des Acetylrestes aus einer Acetyloxygruppe $R_2$, z.B. durch Hydrolyse in schwach-basischem Medium, wie mit einer wässrigen Natriumhydroxydlösung bei pH 9-10, oder durch Behandeln mit einer geeigneten Esterase, wie einem entsprechenden Enzym aus <u>Rhizobium</u> <u>tritolii</u>, <u>Rhizobium</u> <u>lupinii</u>, <u>Rhizobium</u> <u>japonicum</u> oder <u>Bacillus</u> <u>subtilis</u>, oder einer geeigneten Citrus-Esterase, z.B. aus Orangenschalen, freisetzen kann), mit einem geeigneten Kohlensäurederivat, insbesondere mit einer Isocyanat- oder Carbaminsäureverbindung, wie einem Silylisocyanat, z.B. Silyltetraisocyanat, einem Sulfonylisocyanat, z.B. Chlorsulfonylisocyanat, oder Carbaminsäurehalogenid, z.B. -chlorid (die zu N-unsubstituierten 3-Aminocarbonyloxymethyl-Verbindungen führen), oder dann mit einer N-substituierten Isocyanat- oder mit einer N-mono- oder N,N-disubstituierten Carbaminsäure-Verbindungen, wie einem entsprechenden Carbaminsäurehalogenid, z.B. -chlorid, umsetzt, wobei man üblicherweise in Gegenwart eines Lösungs- oder Verdünnungsmittels und, wenn notwendig, unter Kühlen oder Erwärmen, in einem geschlossenen Gefäss und/oder in einer Inertgas-, z.B. Stickstoffatmosphäre, arbeitet.

Ferner kann man eine Verbindung der Formel I, worin $R_1$ eine Gruppe $-CH_2-R_2$ bedeutet, wobei $R_2$ z.B. den oben definierten, durch nucleophile Substitution ersetzbaren Rest darstellt, mit einer tertiären organischen Base, insbesondere einem gegebenenfalls substituierten Pyridin, unter neutralen oder schwach sauren Bedingungen, bevorzugt bei einem pH-Wert von etwa 6,5, in Gegenwart von Wasser und gegebenenfalls in einem mit Wasser mischbaren organischen Lösungsmittel umsetzen und so zu Verbindungen der Formel I gelangen, worin $R_1$ den Rest der Formel $-CH_2-R_2$ darstellt und $R_2$ für eine quaternäre Ammoniumgruppe steht. Die schwach-sauren Bedingungen können durch Zugabe einer geeigneten organischen oder anorganischen Säure, beispielsweise Essigsäure, Chlorwasserstoffsäure, Phosphorsäure oder auch Schwefelsäure eingestellt werden. Als organische Lösungsmittel können beispielsweise die vorstehend genannten, mit Wasser mischbaren Lösungsmittel verwendet werden. Zur Erhöhung der Ausbeute können der Reaktionsmischung gewisse Salze zugesetzt werden, beispielsweise Alkalimetall-, wie Natrium- und insbesondere Kaliumsalze, von anorganischen Säuren, wie Halogenwasserstoffsäure, z.B. Chlorwasserstoff- und insbesondere Jodwasserstoffsäure, sowie der Thiocyansäure, oder organischen Säuren, wie Niederalkancarbonsäuren, z.B. Essigsäure. Vertreter solche Salze sind beispielsweise Natriumjodid, Kaliumjodid und Kaliumthiocyanat. Auch Salze von geeigneten Anionenaustauschern, z.B. flüssige Ionenaustauscher in Salzform, wie z.B. Amberlite LA-1 (flüssige sekundäre Amine mit einem Molekulargewicht von 351-393; Oel-löslich und wasserunlöslich; mAeq./g = 2,5-2,7, z.B. in Acetatform), mit Säuren, z.B. Essigsäure, können für diesen Zweck verwendet werden.

Quaternäre Ammoniumgruppen $R_2$ können vorteilhafterweise unter Verwendung eines Zwischenprodukts der Formel I, in welchem $R_2$ für eine substituierte, insbesondere für eine aromatische substituierte Carbonylthiogruppe und in erster Linie für die Benzoylthiogruppe steht, hergestellt werden. Ein solches Zwischenprodukt, das man z.B. durch Umsetzen einer Verbindung der Formel I, worin $R_2$ im Rest $R_1$ eine veresterte Hydroxygruppe, insbesondere eine Niederalkanoyloxy- z.B. Acetyloxygruppe bedeutet, mit einem geeigneten Salz, wie einem Alkalimetall-, z.B. Natriumsalz, einer Thiocarbonsäure, wie einer aromatischen Thiocarbonsäure, z.B. Thiobenzoesäure, erhalten kann, wird mit dem tertiären Amin, insbesondere einer tertiären heterocyclischen Base, wie einem gegegebenenfalls substituierten Pyridin, umgesetzt, wobei man die quaternäre Ammoniumverbindung erhält. Die Reaktion wird üblicherweise in Gegenwart eines geeigneten Entschwefelungsmittels, insbesondere eines Quecksilbersalzes, z.B. Quecksilber-IIperchlorat, und eines geeigneten Lösungs- oder Verdünnungsmittels oder eines Gemisches, wenn notwendig, unter Kühlen oder Erwärmen, in einem geschlossenen Gefäss und/oder in einer Inertgas-, z.B. Stickstoffatomosphäre, durchgeführt.

Einführung von 7α-Methoxy: In einem erhaltenen Zwischenprodukt der Formel I oder IV, worin $R_3$ Wasserstoff bedeutet und alle funktionellen Gruppen in geschützter Form vorliegen, kann die 7α-Methoxygruppe $R_3$ auf an sich bekannte Weise eingeführt werden, beispielsweise, indem man das genannte Zwischenprodukt nacheinander in einem Anionen-bildenden Mittel, einem N-Halogenierungsmittel und Methanol behandelt.

Ein geeignetes Anionen-bildendes Mittel ist in erster Linie eine metallorganische Base, insbesondere eine Alkalimetall-, in erster Linie eine Lithium-organische Base. Solche Verbindungen sind insbesondere entsprechende Alkoholate, wie geeignete Lithium-niederalkanolate, in erster Linie Lithiummethylat, oder entsprechende Metall-Kohlenwasserstoffbasen, wie Lithium-niederalkane und Lithiumphenyl. Die Umsetzung mit der Anion-bildenden metallorganischen Base wird üblicherweise unter Kühlen, z.B. von etwa 0°C bis etwa -80°C, und in Gegenwart eines geeigneten Lösungs- oder Verdünnungsmittel, z.B. eines Aethers, wie Tetrahydrofuran, bei Verwendung von Lithiummethylat auch in Gegenwart von Methanol, und, wenn erwünscht, in einem geschlossenen Gefäss und/oder in einer Inertgas-, z.B. Stickstoffatmosphäre, vorgenommen.

Als N-halogenierende Mittel verwendet man üblicherweise ein sterisch gehindertes, organisches Hypohalogenit, insbesondere -chlorit, und in erster Linie ein entsprechendes aliphatisches Hypohalogenit, z.B. -chlorit, wie ein tert.-Niederalkyl-hypohalogenit, z.B. -chlorit. In erster Linie wendet man das tert.-Butylhypochlorit an, das man mit dem nichtisolierten Produkt der Anionisierungsreaktion umsetzt.

Die N-halogenierte Zwischenverbindung wird bei Anwesenheit eines Ueberschusses der Anion-bildenden Base, insbesondere von Lithiummethylat, unter den Reaktionsbedingungen und ohne isoliert zu werden in eine 7-Acyliminocephemverbindung umgewandelt, und diese durch Zugabe von Methanol in eine 7α-Methoxy-cephem-Verbindung übergeführt. Falls notwendig, müssen aus dem N-halogenierten Zwischenprodukt die Elemente der Halogenwasserstoff-, insbesondere der Chlorwasserstoffsäure, abgespalten werden; dies geschieht unter Zugabe einer Halogenwassertoff-abspaltenden Base, wie eines geeigneten Alkalimetall-niederalkanolats, z.B. Lithium-tert.-butylat, wobei diese Reaktion üblicherweise unter den Bedingungen der Anion- und N-Halogenverbindung-bildenden Reaktion stattfindet, wobei man in Gegenwart von Methanol arbeiten und anstelle der Acyliminoverbindung direkt die 7α-Methoxy-cephem-Verbindung erhalten kann. Man geht üblicherweise aus von einer Verbindung der Formel I, worin funktionelle Gruppen in geschützter Form vorliegen, setzt diese mit einem Ueberschuss des Anion-bildenden Mittels, z.B. Lithiummethylat oder Phenyllithium, in Gegenwart von Methanol um, behandelt dann mit dem N-Halogenierungsmittel, z.B. tert.-Butylhypochlorit, und erhält so direkt die gewünschte Verbindung der Formel I, worin funktionelle Gruppen geschützt sind. Man kann auch das Methanol nachträglich zugeben, wobei man die Dehydrohalogenierung und die Zugabe von Methanol bei etwas höheren Temperaturen als die Anion- und N-Halogenverbindung-bildenden Reaktionen, z.B. bei etwa 0°C bis etwa -20°C, wenn notwendig, in einem geschlossenen Gefäss und/oder in einer Inertgas-, z.B. Stickstoffatmosphäre, durchführen kann.

Umwandlung zum 1-Oxid, 1-Dioxid und 1-Sulfid:

Eine Verbindung der Formel I, worin der Index $\ell$ 0 bedeutet, kann mit den unter Verfahren d) beschriebenen Oxydationsmitteln in das entsprechende 1-Oxid, worin der Index $\ell$ den Wert 1 hat, umgewandelt werden.

1-Oxide der Formel I, welche in β-Konfiguration vorliegen, können in an sich bekannter Weise gemäss dem aus der Deutschen Offenlegungsschrift 30 13 996 bekannten Verfahren, und zwar durch Oxydation eines 1-Sulfids der Formeln I oder VIII ($\ell$=0) mit einer Percarbonsäure, z.B. Peressigsäure oder m-Chlorperbenzoesäure hergestellt werden.

1-Oxide der Formel 1, welche in α- oder β-Konfiguration vorliegen, können in an sich bekannter Weise gemäss dem aus der Deutschen Offenlegungsschrift 30 13 996 bekannten Verfahren und zwar durch Oxydation eines 1-Sulfids der Formel II, worin die 7β-Aminogruppe beispielsweise durch Ylidengruppen geschützt ist, die zusammen mit der Aminogruppe eine Schiff'sche Base bilden, mit einer Percarbonsäure, z.B. m-Chlorbenzoesäure, chromatographischer Trennung der erhaltenen α- und β-1-Oxide der Formel II und anschliessender Acylierung mit einer Carbonsäure der Formel III hergestellt werden.

Eine Verbindung der Formel I, worin der Index $\ell$ 0 oder 1 bedeutet, kann durch Umsetzung mit Sulfid- oder Sulfoxidgruppen in Sulfongruppen überführenden Mitteln in das entsprechende 1-Dioxid, worin $\ell$ den Wert 2 hat, überführt werden.

Solche Mittel sind insbesondere Wasserstoffperoxid, organische Persäuren, insbesondere aliphatische Percarbonsäuren, z.B. Peressigsäure, Perbenzoesäure, Chlorperbenzoesäure, z.B. m-Chlorperbenzoesäure, oder Monoperphthalsäure, oxidierende anorganische Säuren oder deren Salze, z.B. Salpetersäure, Chromsäure, Kaliumpermanganat, oder Alkalimetallhypochlorit, z.B. Natriumhypochlorit, sowie anodische Oxidation. Die Oxidation wird bevorzugt in einem geeigneten inerten Lösungsmittel, beispielsweise einem Halogenkohlenwasserstoff, z.B. Methylenchlorid, Chloroform oder Tetrachlorkohlenstoff, einem Alkohol, z.B. Methanol oder Aethanol, einem Keton, z.B. Aceton, einem Aether, z.B. Diäthyläther, Dioxan oder Tetrahydrofuran, einem Amid, z.B.

Dimethylformamid, einem Sulfon, z.B. Dimethylsulfon, einer flüssigen organischen Carbonsäure, z.B. Essigsäure, oder in Wasser oder einem Gemisch dieser Lösungsmittel, insbesondere einem wasserhaltigen Gemisch, z.B. wässriger Essigsäure, bei Raumtemperatur, oder unter Kühlen oder leichtem Erwärmen, d.h. bei etwa -20 bis etwa +90°, bevorzugt bei etwa +18 bis etwa +30°, durchgeführt. Die Oxidation kann auch stufenweise durchgeführt werden, indem zunächst bei niederer Temperatur, d,h. bei etwa -20° bis etwa 0° bis zur Sulfoxidstufe oxidiert wird, das gegebenenfalls isoliert wird, worauf in einem zweiten Schritt, bevorzugt bei höherer Temperatur, etwa bei Raumtemperatur, das Sulfoxid zum Sulfon, d.h. dem 1,1-Dioxid der Formel (I), oxidiert wird.

Zur Aufarbeitung kann gegebenenfalls noch vorhandenes überschüssiges Oxidationsmittel durch Reduktion, insbesondere durch Behandeln mit einem Reduktionsmittel, wie einem Thiosulfat, z.B. Natriumthiosulfat, zerstört werden.

Ein 1-Oxid der Formel I, worin der Index $\ell$ den Wert 1 hat, sowie ein 1-Dioxid, worin der Index $\ell$ den Wert 2 hat, kann mit den unter Verfahren d) beschriebenen Reduktionsmitteln in das entsprechende 1-Sulfid, worin der Index $\ell$ den Wert 0 hat, umgewandelt werden.

<u>Abspaltung von Schutzgruppen:</u> In einer erhaltenen Verbindung der Formel I, worin eine oder mehrere funktionelle Gruppen geschützt sind, können diese, z.B. geschützte Carboxyl-, Amino-, Hydroxy- und/oder Sulfogruppen, in an sich bekannter Weise, mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

Eine geschützte Carboxylgruppe setzt man in an sich bekannter und je nach Art der Schutzgruppen in verschiedenartiger Weise, vorzugsweise mittels Solvolyse oder Reduktion, frei.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nukleophilen Verbindung, wie Phenol, Anisol oder Aethylenthioglykol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silyläthoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, in

Anwesenheit eines macrocyclischen Polyäthers ("Kronenäther"), oder mit einem Fluorid einer organischen quaternären Base, wie Tetra-niederalkylammoniumfluorid oder Triniederalkyl-aryl-ammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silylgruppe, wie Triniederalkylsilyl, z.B. Trimethylsilyl, verestertes Carboxyl kann üblicherweise solvolytisch, z.B. durch Behandeln mit Wasser, einem Alkohol oder einer Säure freigesetzt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino, gegebenenfalls nach Umwandlung einer 2-Bromniederalkoxycarbonylaminogruppe in eine 2-Jodniederalkoxycarbonylgruppe, Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitrobenzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino, z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 2-Acylniederalk-1-en-1-ylamino, z.B. durch Behandeln mit einer Säure, wie Mineralsäure, z.B. Chlorwasserstoffsäure, oder einer

organischen Säure, z.B. Ameisen-, Essig- oder Trifluoressigsäure, gegebenenfalls in Gegenwart von Wasser, und eine mit einer organischen Silylgruppe geschützte Aminogruppe, z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden. Eine Phosphor-, Phosphon- oder Phosphinamidogruppe kann z.B. durch Behandeln mit einer Phosphorhaltigen Säure, wie einer Phosphor-, Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, in die freie Aminogruppe übergeführt werden.

Ein in Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silyl- oder Stannylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracetyl geschützte Hydroxygruppe wird z.B. durch basische Hydrolyse, während eine durch tert.-Niederalkyl oder durch einen 2-oxa- oder 2-thiaaliphatischen oder -cycloaliphatischen Kohlenwasserstoffrest verätherte Hydroxygruppe durch Acidolyse, z.B. durch Behandeln mit einer Mineralsäure oder einer starken Carbonsäure, z.B. Trifluoressigsäure, freigesetzt.

Eine geschützte, insbesondere veresterte Sulfogruppe wird analog einer geschützten Carboxylgruppe freigesetzt.

Die beschriebenen Spaltungsreaktionen werden unter an sich bekannten Bedinungen durchgeführt, wenn notwendig unter Kühlen oder Erwärmen, in einem geschlossenen Gefäss und/oder in einer Inertgas-, z.B. Stickstoffatmosphäre.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium/Kohle/Katalysator.

<u>Veresterung einer freien Carboxylgruppe</u>: Die Umwandlung einer freien Carboxylgruppe in einer Verbindung der Formel (I) in verestertes Carboxyl, insbesondere in eine veresterte Carboxylgruppe, die unter physiologischen Bedingungen spaltbar ist, erfolgt nach an sich bekannten Veresterungsmethoden, beispielsweise indem man eine Verbindung der Formel I, worin andere vorhandene funktionelle Gruppen gegebenenfalls in geschützter Form vorliegen, oder ein reaktionsfähiges funktionelles Carboxyderivat, inkl. ein Salz davon, mit einem entsprechenden Alkohol oder einem reaktionsfähigen funktionellen Derivat davon, umsetzt.

Die Veresterung von freiem Carboxyl mit dem gewünschten Alkohol wird in Gegenwart eines geeigneten Kondensationsmittels durchgeführt. Uebliche Kondensationsmittel sind z.B. Carbodiimide, beispielsweise N,N'-Diäthyl-, N,N'-Dipropyl-, N,N'-Dicyclohexyl- oder N-Aethyl-N'-(3-dimethylaminopropyl)-carbodiimid, geeignete Carbonylverbindungen, beispielsweise Carbonyldiimidazol, oder 1,2-Oxazoliumverbindungen, z.B. 2-Aethyl-5-phenyl-1,2-oxazolium-3'-sulfonat und 2-tert.-Butyl-5-methylisoxazoliumperchlorat, oder eine geeignete Acylaminoverbindung, z.B. 2-Aethoxy-1-äthoxycarbonyl-1,2-dihydrochinolin. Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran und, wenn notwendig, unter Kühlen oder Erwärmen und/oder in einer Inertgasatmosphäre durchgeführt.

Geeignete reaktionsfähige funktionelle Derivate der zu veresternden Carboxylverbindungen der Formel I sind z.B. Anhydride, insbesondere gemischte Anhydride, und aktivierte Ester.

Gemischte Anhydride sind z.B. diejenigen mit anorganischen Säuren, wie Halogenwasserstoffsäuren, d.h. die entsprechenden Säurehalogenide, z.B. -chloride oder -bromide, ferner Stickstoffwasserstoffsäure, d.h. die entsprechenden Säureazide, sowie phosphorhaltigen Säuren, z.B. Phosphorsäure, Diäthylphosphorsäure oder phosphoriger Säure, oder schwefelhaltigen Säuren, z.B. Schwefelsäure, oder Cyanwasserstoffsäure. Gemischte Anhydride sind weiter z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern,

insbesondere Niederalkylhalbestern der Kohlensäure, wie Aethyl- oder Isobutylhalbestern der Kohlensäure, oder mit organischen, insbesondere aliphatischen oder aromatischen, Sulfonsäure, z.B. p-Toluolsulfonsäure.

Zur Reaktion mit dem Alkohol geeignete aktivierte Ester sind z.B. Ester mit vinylogen Alkoholen (d.h. Enolen), wie vinylogen Niederalkenolen, oder Iminomethylesterhalogeniden, wie Dimethyliminomethylesterchlorid hergestellt aus der Carbonsäure und Dimethylchlormethylideniminiumchlorid der Formel $[(CH_3)_2N=CHCl]^{\oplus}Cl^{\ominus}$, oder Arylester, wie Pentachlorphenyl-, 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthalyliminoester.

Die Acylierung mit einem solchen Säurederivat, wie einem Anhydrid, insbesondere mit einem Säurehalogenid wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkylanilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Ein reaktionsfähiges funktionelles Derivat des veresternden Alkohols ist in erster Linie ein entsprechender Ester, vorzugsweise

mit einer starken anorganischen oder organischen Säure und stellt insbesondere ein entsprechendes Halogenid, z.B. Chlorid, Bromid oder Jodid, oder eine entsprechende Niederalkyl- oder Aryl-, wie Methyl- oder 4-Methylphenylsulfonyloxyverbindung dar.

Ein solcher reaktionsfähiger Ester eines Alkohols kann mit der freien Carboxylverbindung der Formel I oder einem Salz, wie einem Alkalimetäll- oder Ammoniumsalz davon umgesetzt werden, wobei man bei Verwendung der freien Säure vorzugsweise in Gegenwart eines säurebindenden Mittels arbeitet.

Die obigen Veresterungsreaktionen werden in einem inerten, üblicherweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem cyclischen Aether, z.B. Tetrahydrofuran, einem Ester, z.B. Essigsäureäthylester, oder einem Nitril, z.B. Acetonitril, oder Mischungen davon, wenn notwendig, unter Kühlen oder Erwärmen, z.B. in einem Temperaturbereich von etwa bei -40°C bis etwa +100+C, vorzugsweise bei etwa -10°C bis etwa +40°C, und/oder in einer Inertgas-, z.B. Stickstoffatmosphäre.

Ferner kann das Säurederivat, wenn erwünscht, in situ gebildet werden. So erhält man z.B. ein gemischtes Anhydrid durch Behandeln der Carbonsäureverbindung mit entsprechend geschützten funktionellen Gruppen oder eines geeigneten Salzses davon, wie eines Ammoniumsalzes, z.B. mit einem organischem Amin, wie Piperidin oder 4-Methylmorpholin, oder eines Metall-, z.B. Alkalimetallsalzes mit einem geeigneten Säurederivat, wie dem entsprechenden Säurehalogenid einer gegebenenfalls substituierten Niederalkancarbonsäure, z.B. Trichloracetylchlorid, mit einem Halbester eines Kohlensäure-halbhalogenids, z.B. Chlorameisensäureäthylester oder -isobutylester, oder mit einem Halogenid einer Diniederalkylphosphorsäure, z.B. Diäthylphosphorbromidat, und verwendet das so erhältliche gemischte Anhydrid ohne Isolierung.

Salzbildung: Salze von Verbindungen der Formel I können in an sich bekannter Weise hergestellt werden. So kann man Salze von Verbindungen der Formel I z.B. durch Reaktion der sauren Gruppen mit Metallverbindungen, wie Alkalimetallsalzen von geeigneten Carbonsäuren, z.B. dem Natriumsalz der α-Aethylcapronsäure oder Natriumcarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Bei sämtlichen weiter vorn genannten Umsetzungen, die unter basischen Bedingungen durchgeführt werden, können 3-Cephemverbindungen, gegebenenfalls teilweise, zu 2-Cephemverbindungen isomerisieren. Eine erhaltene 2-Cephemverbindung oder ein Gemisch aus einer 2- und einer 3-Cephemverbindung kann in an sich bekannter Weise zur gewünschten 3-Cephemverbindung isomerisiert werden.

Gemische von Isomeren können in an sich bekannter Weise, z.B. durch fraktionierte Kristallisation, Chromatographie, etc. in die einzelnen Isomeren aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Pharmazeutische Präparate:

Die pharmakologisch verwendbaren Verbindungen der Formel I, deren Hydrate oder Salze können zur Herstellung von pharmazeutischen Präparaten verwendet werden.

Pharmazeutische Präparate enthalten eine wirksame Menge des reinen Wirkstoffs der Formel I selbst oder eine wirksame Menge des Wirkstoffs der Formel I im Gemisch mit anorganischen oder organischen, festen oder flüssigen, pharmazeutisch verwendbaren Trägerstoffen, die sich vorzugsweise zur parenteralen Verabreichung eignen.

Vorzugsweise verwendet man die Wirkstoffe der Formel I der vorliegenden Erfindung in Form von injizierbaren, z.B. intravenös, verabreichbaren Präparaten oder von Infusionslösungen. Solche Lösungen sind vorzugsweise isotonische wässrige Lösungen oder Suspensionen, welche z.B. aus lyophilisierten Präparaten, welche den reinen Wirkstoff oder den Wirkstoff zusammen mit einem Trägermaterial, z.B. Mannit, enthalten, vor Gebrauch hergestellt werden können. Die pharmazeutischen Präparate sind vorzugsweise sterilisiert und können Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die,

wenn erwünscht, weitere pharmakologisch wertvolle Stoffe enthalten können, enthalten von etwa 0,1% bis 100 %, insbesondere von etwa 1% bis etwa 50%, Lyophilisate bis zu 100% des Aktivstoffes.

Die pharmazeutischen Präparate werden in an sich bekannter Weise, z.B. mittels konventioneller Lösungs- oder Lyophilisierungsverfahren, hergestellt.

Verwendung:
Verbindungen der Formel I, deren Hydrate oder pharmazeutisch verwendbare Salze können als antibiotisch wirksame Mittel in Form von pharmazeutischen Präparaten in einem Verfahren zur therapeutischen Behandlung des menschlichen oder tierischen Körpers angewendet werden, z.B. zur Behandlung von Infektionen, welche durch grampositive oder gramnegative Bakterien und Kokken, z.B. durch Enterobakterien, z.B. Escherichia coli, Klebsiella pneumoniae oder Proteus spp., verursacht werden.

Je nach Art der Infektionen und Zustand des infizierten Organismus verwendet man tägliche Dosen von etwa 0,5 g bis etwa 5 g s.c., i.v. oder i.m. zur Behandlung von Warmblütern von etwa 70 kg Gewicht.

Ausgangsstoffe: Die im Verfahren zur Herstellung der Verbindungen der vorliegenden Erfindung verwendeten Ausgangsstoffe sind bekannt oder, falls neu, können in an sich bekannter Weise hergestellt werden.

Ausgangsmaterialien der Formel II, sowie entsprechende Verbindungen mit geschützten funktionellen Gruppen, sind bekannt oder können auf an sich bekannte Weise hergestellt werden.

Verbindungen der Formel III, worin der Index n und die Symbole X, W und Y die unter Formel I genannten Bedeutungen haben und die α-Aminocarbonsäuregruppierung $HOOC-CH(NH_2)-$ und gegebenenfalls in der Gruppe Y vorhandene funktionelle Gruppen gegebenenfalls in geschützter

Form vorliegen, werden beispielsweise hergestellt, indem man eine Verbindung der Formel V, worin n, X und W die unter Formel I genannten Bedeutungen haben und die α-Aminocarbonsäuregruppierung HOOC-CH($NH_2$)- in geschützter Form vorliegen, oder wenn X-W zusammen die Gruppe -CO- bedeuten, auch eine freie Säure oder ein reaktionsfähiges funktionelles Säurederivat oder ein Salz davon mit einer Säure der Formel

$$H_2N-(C_mH_{2m})-\underset{}{\overset{Y}{\overset{|}{C}}}H-COOH \qquad (IX),$$

worin m und Y die unter Formel I genannten Bedeutungen haben, und worin die Aminogruppe gegebenenfalls durch eine die Acylierungsreaktion erlaubende Gruppe geschützt ist und in der Gruppe Y vorhandene funktionelle Gruppen geschützt sind und die Carboxylgruppe intermediär geschützt ist, acyliert und, wenn erwünscht, eine erhältliche Verbindung der Formel III in eine andere Verbindung der Formel III mit entsprechend geschützten funktionellen Gruppen überführt.

Schutzgruppen, welche die Acylierungsreaktion erlauben, sowie die Schutzgruppen der α-Aminocarbonsäuregruppierung und in der Gruppe Y vorhandene funktionelle Gruppen, sind die gleichen Schutzgruppen wie die unter den Verbindungen der Formeln I, II, IV, V, VI, VII oder VIII erwähnten.

Zum intermediären Schutz der Carboxylgruppe in der Verbindung der Formel IX können an sich ebenfalls die bereits erwähnten Carboxylschutzgruppen verwendet werden, jedoch müssen sich die zum intermediären Schutz in der vorliegenden Acylierung benützten Carboxylschutzgruppen von den übrigen Schutzgruppen, die in den Verbindungen der Formel III notwendigerweise vorhanden bleiben sollen, in der Art ihrer Abspaltung unterscheiden, so dass sie nach der vorliegenden Acylierungsreaktion selektiv abgespalten werden können. Wenn beispielsweise zum intermediären Schutz der Carboxylgruppe ein hydrogenolytisch

abspaltbare Schutzgruppe, wie eine der genannten gegebenenfalls substituierten Benzylgruppen, z.B. die Benzyl- oder p-Nitrobenzylgruppe, verwendet wird, dann dürfen die anderen Schutzgruppen hydrogenolytisch nicht abspaltbar sein; sie können beispielsweise die genannten, nur acidolytisch abspaltbaren tert.-Niederalkylgruppen, wie tert.-Butyl, bzw. tert.Niederalkoxycarbonylgruppen, wie tert.-Butoxycarbonyl, sein.

Die Acylierung kann im übrigen analog ausgeführt werden wie die Acylierung von Verbindungen der Formel IV mit einer Säure der Formel V, bzw. einem entsprechend geschützten und reaktionsfähigen funktionellen Derivat davon.

In einer erhaltenen Verbindung der Formel III, mit entsprechend geschützten funktionellen Gruppen, kann eine Schutzgruppe, gegebenenfalls selektiv, abgespalten oder eine, gegebenenfalls bei der Acylierungsreaktion frei gewordene, funktionelle Gruppe geschützt werden. Die Verbindungen der Formeln IV, V, VI und VII sind bekannt, sowie entsprechende Verbindungen mit geschützten funktionellen Gruppen sind bekannt oder können auf an sich bekannte Weise hergestellt werden.

2-Cephemausgangsverbindungen der Formel VIII sind neu. Sie können, analog den Acylierungs- und Kondensationsverfahren a) bis d), ausgehend von den bekannten oder auf an sich bekannte Weise herstellbaren 2-Cephemverbindungen der Formel

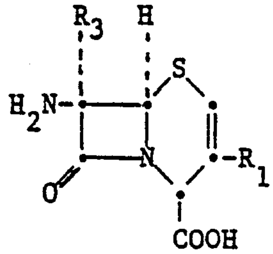

(X),

hergestellt werden. Ausserdem können sie als Nebenprodukte bei den Verfahren a) bis d) entstehen, insbesondere wenn unter basischen Bedingungen gearbeitet wird.

Die folgenden Beispiele dienen zur Illustration der Erfindung; Temperaturen werden in Celsiusgraden angegeben. Die Wellenlängen der UV-Spektren werden in Nanometern (nm) angegeben.

In den Beispielen werden folgende Abkürzungen verwendet:

BOC: tert.Butyloxycarbonyl

F: Schmelzpunkt

DC: Dünnschichtchromatogramm: auf Silicagel-Fertigplatten SL 254 der Fa. Antec, Birsfelden, Schweiz

$Rf_{96}$: Rf-Wert im Lösungsmittelsystem sec. Butanol-Eisessig-Wasser 67:10:23.

Beispiel 1:

1a): 58,26 g 3-Acetoxymethyl-7β-(2-aminoacetamido)-3-cephem-4-carbonsäurediphenylmethylester-p-toluolsulfonsäuresalz werden in 750 ml Tetrahydrofuran zusammen mit 16,11 ml Pyridin und 67 g (2R)-2-BOC-Amino-2-diphenylmethoxycarbonyläthoxycarbonylchlorid 1 Std. bei 0° gerührt. Man nimmt in Essigester auf, wäscht nacheinander mit 1N wässriger Salzsäure, gesättigter wässriger NaCl-Lösung, gesättigter wässriger $NaHCO_3$-Lösung und nochmals NaCl-Lösung bis zum Neutralpunkt. Nach Trocknen über Natriumsulfat und Eindampfen chromatographiert man das erhaltene Rohprodukt an 2000 g Kieselgel (Eluiermittel:Toluol-Essigester-(1:1); Fraktionen à 1000 ml). Nach Vereinigung der produkthaltigen Fraktionen erhält man den 3-Acetoxymethyl-7β-[2-((2R)-2-BOC-amino-2-diphenylmethoxycarbonyläthoxycarbonylamino)-acetamido]-3-cephem-4-carbonsäurediphenylmethylester; $[\alpha]_D^{20°} = +21° \pm 1°$ (0,90% in $CHCl_3$); IR: 3390, 1785, 1740-1690 (breit), 1510 (Schulter), 1495 $cm^{-1}$ ($CH_2Cl_2$); UV: 258 (7800), 263 (7800; EtOH).

1b): Zu einer unter Stickstoffatmosphäre auf -75° abgekühlten Lösung von 45 g 3-Acetoxymethyl-7β- [2-((2R)-2-BOC-amino-2-diphenylmethoxycarbonyläthoxycarbonylamino)-acetamido]-3-cephem-4-carbonsäurediphenylmethylester in 4500 ml Tetrahydrofuran tropft man unter heftigem Rühren im Verlauf von 1 Minute eine vorgekühlte Lösung von 1,5 g Lithium in 270 ml Methanol zu. Dann lässt man 2 Minuten nachrühren, gibt bei -75° 3,33 ml t-Butylhypochlorit zu, rührt 15 Minuten, gibt nochmals 3,33 ml t-Butylhypochlorit zu, rührt 15 min und gibt wiederum 1,86 ml t-Butylhypochlorit zu. Darauf rührt man nochmals 15 min bei -75° und tropft anschliessend unter Rühren nacheinander 68,4 ml Essigsäure und eine Lösung von 19,8 g Natriumthiosulfat in 30 ml Wasser zu. Dann lässt man auf Zimmertemperatur erwärmen, engt im Vakuum auf ca 1 l ein, nimmt in Essigester auf und wäscht nacheinander mit Wasser, gesättigter wässriger $NaHCO_3$-Lösung und mit gesättigter wässriger NaCl-Lösung bis zum Neutralpunkt. Das nach dem Trocknen über Natriumsulfat und dem Eindampfen erhaltene Rohprodukt chromatographiert man an 2000 g Kieselgel (Eluiermittel:Toluol-Essig-

ester-(2:1); Fraktionen à 1000 ml). Durch Vereinigung der produkthaltigen Fraktionen erhält man nach Umfällen aus $CH_2Cl_2$-Diäthyläther-Hexan den 3-Acetoxymethyl-7α-methoxy-7β-[2-((2R)-2-BOC-amino-2-diphenylmethoxycarbonyläthoxycarbonylamino)-acetamido]-3-cephem-4-carbonsäurediphenylmethylester ; $[\alpha]_D^{20°} = +49° \pm 1°$ (0,87% in $ChCl_3$); IR: 3380, 1782, 1740-1690 (breit), 1495 $cm^{-1}$ ($CH_2Cl_2$); UV: 251 (4700), 258 (4800), 264 (4950), 268 (4800; EtOH).

1c) 0,9 g 3-Acetoxymethyl-7β-[2-((2R)-2-BOC-amino-2-diphenylmethoxycarbonyläthoxycarbonylamino)-acetamido]-3-cephem-4-carbonsäurediphenylmethylester werden in einer Mischung von 1,95 ml $CH_2Cl_2$ und 0,713 ml Anisol bei Zimmertemperatur gelöst. Man gibt zu der Lösung 9,3 ml auf 0° gekühlte Trifluoressigsäure und rührt ohne Kühlung während 15 min. Nach Zugabe von 120 ml Hexan-Aether-(2:1) wird 5Minuten gerührt, der Niederschlag abgenutscht und mit 100 ml Hexan-Aether-(2:1)-Mischung nachgewaschen. Man löst den Filterrückstand in 70 ml Methanol, fügt 70 ml Wasser hinzu, stellt durch Zugabe von 1N-wässriger Natronlauge auf pH 7 und extrahiert mit Essigester. Die organische Phase wird dreimal mit Wasser gewaschen, alle wässrigen Phasen werden vereinigt und im Vakuum auf ca. 10 ml eingeengt. Durch Zugabe von ca. 300 ml Aethanol entsteht eine weisse Fällung, die abgenutscht, zweimal je mit Aethanol und Aether gewaschen und im Vakuum getrocknet wird. Man erhält das Hydrat des 3-Acetoxymethyl-7β-[2-((2R)-2-amino-2-carboxyäthoxycarbonylamino)-acetamido]-3-cephem-4-carbonsäurenatriumsalzes; Zersetzung ab 150°; $[\alpha]_D^{20°} = +100° \pm 1°$ (0,76% in $H_2O$); IR: 3600-2400 (breit), 1776, 1732, 1650 (Schulter), 1610, 1557, 1540 $cm^{-1}$ (Nujol); UV: 259 (6800; EtOH).

1d) 8,83 g 3-Acetoxymethyl-7α-methoxy-7β-[2-((2R)-2-BOC-amino-2-diphenylmethoxycarbonyläthoxy carbonylamino)-acetamido]-3-cephem-4-carbonsäurediphenymethylester werden in 18,5 ml $CH_2Cl_2$ und 6,77 ml Anisol mit 88,3 ml Trifluoressigsäure analog Beispiel 1c)

umgesetzt und aufgearbeitet. Man erhält das Hydrat des 3-Acetoxymethyl-7α-methoxy-7β-[2-((2R)-2-amino-2-carboxyäthoxycarbonylamino)-acetamido]-3-cephem-4-carbonsäure-natriumsalzes; Zersetzung ab 155°; $[\alpha]_D^{20°} = +153° \pm 1°$ (0,74% in $H_2O$); IR: 3600-2400 (breit), 1777, 1735, 1710 (Schulter), 1612 (breit), 1538 (breit) $cm^{-1}$ (Nujol); UV: 240 (6400), 264 (7800; EtOH).

1e) 1,6 g 3-Acetoxymethyl-7α-methoxy-7β-[2-((2R)-2-amino-2-carboxyäthoxycarbonylamino)-acetamido]-3-cephem-4-carbonsäure-natriumsalz und 0,93 g 1-Methyl-5-mercapto-1H-tetrazol-natriumsalz werden in 8 ml Wasser aufgeschlämmt und durch Zugabe von 1N wässriger Natronlauge bei pH 6,8 in Lösung gebracht. Dann rührt man unter Stickstoff 4 Std. bei 65°, kühlt ab und trägt in 800 ml Aethanol ein. Der gebildete Niederschlag wird abgenutscht, in wenig Wasser gelöst und an 150 g silyliertem Kieselgel (Antec Opti-Up $C_{12}$) chromatographiert (Eluiermittel: Wasser; Fraktionengrösse 30 ml). Die produkthaltigen Fraktionen werden vereinigt, auf ca. 10 ml Volumen eingeengt und in 400 ml Aethanol eingetragen. Das ausgefallene Produkt wird abfiltriert, je zweimal mit Aethanol und Diäthyläther gewaschen und getrocknet. Man erhält das Hydrat des 3-(1-Methyl-1H-tetrazol-5-ylthiomethyl)-7α-methoxy-7β-[2-((2R)-2-amino-2-carboxyäthoxycarbonylamino)-acetamido]-3-cephem-4-carbonsäure-natriumsalzes; Zersetzung ab 150°; $[\alpha]_D^{20°} = +81° \pm 1°$ (0,81% in $H_2O$); IR: 3600-2400 (breit), 1770, 1730, 1692, 1616 (breit), 1532 $cm^{-1}$ (Nujol); UV: 238 (7400), 270 (9200; EtOH).

1f) 1,5 g 3-Acetoxymethyl-7α-methoxy-7β-[2-((2R)-2-amino-2-carboxyäthoxycarbonylamino)-acetamido]-3-cephem-4-carbonsäure-natriumsalz und 1,0 g 1-Carboxymethyl-5-mercapto-1H-tetrazol werden in 8 ml Wasser wie im Beispiel 1e) beschrieben umgesetzt, aufgearbeitet und chromatographiert. Man erhält das Hydrat des 3-(1-Carboxymethyl-1H-tetrazol-5-ylthiomethyl]-7α-methoxy-7β-[2-((2R)-2-amino-2-carboxyäthoxycarbonylamino)-acetamido]-3-cephem-4-carbonsäure-dinatrium-

salzes. Zersetzung ab 160°; $[\alpha]_D^{20°}$ = +71°+1° (0,58% in $H_2O$); IR: 3600-2400 (breit), 1770, 1720, 1690, 1627, 1535 $cm^{-1}$ (Nujol); UV: 233 (8860), 268 (8800):

1g) 1,5 g 3-Acetoxymethyl-7α-methoxy-7β-[2-((2R)-2-amino-2-carboxyäthoxycarbonylamino)-acetamido]-3-cephem-4-carbonsäure-natriumsalz und 1,09 g 1-Dimethylaminoäthyl-5-mercapto-1H-tetrazol werden in 8 ml Wasser wie im Beispiel 1e) beschrieben umgesetzt, aufgearbeitet und chromatographiert. Man erhält das 3-[1-(2-Dimethylaminoäthyl)-1H-tetrazol-5-ylthiomethyl]-7α-methoxy-7β-[2-((2R)-2-amino-2-carboxyäthoxycarbonylamino)-acetamido]-3-cephem-4-carbonsäure-natriumsalz als Hydrat; Zersetzung ab 145°; $[\alpha]_D^{20°}$ = +86°±1° (0,71% in $H_2O$); IR: 3600-2400 (breit), 1770, 1725, 1695, 1622 (breit), 1532 $cm^{-1}$ (Nujol); UV: 240 (7480), 270 (8200; $H_2O$).

1h) 2,0 g 3-Acetoxymethyl-7α-methoxy-7β-[2-((2R)-2-amino-2-carboxyäthoxycarbonylamino)-acetamido]-3-cephem-4-carbonsäure-natriumsalz und 2,0 g 1-Sulfomethyl-5-mercapto-1H-tetrazol werden in 12 ml Wasser wie im Beispiel 1e) beschrieben umgesetzt, aufgearbeitet und chromatographiert. Man erhält das 3-(1-Sulfomethyl-1H-tetrazol-5-ylthiomethyl)-7α-methoxy-7β-[2-((2R)-2-amino-2-carboxyäthoxycarbonylamino)-acetamido]-3-cephem-4-carbonsäure-dinatriumsalz als Hydrat; Zersetzung ab 165°; $[a]_D^{20°}$ = +64°±1° (0,73% in $H_2O$); IR: 3600-2400 (breit), 1770, 1722-1690 (breit), 1632, 1533 $cm^{-1}$ (Nujol); UV: 240 (7640), 268 (8200; $H_2O$).

1i) 1,5 g 3-Acetoxymethyl-7α-methoxy-7β-[2-((2R)-2-amino-2-carboxyäthoxycarbonylamino)-acetamido]-3-cephem-4-carbonsäure-natriumsalz, 0,523 g Isonicotinamid, 4,74 g Natriumjodid und 0,516 g Trichloressigsäure werden in 3,15 ml Wasser 1 1/2 Std. auf 70° erwärmt. Dann wird abgekühlt und in 800 ml Aethanol eingetragen. Der dabei entstehende Niederschlag wird abfiltriert, mit Aether gewaschen und getrocknet. Anschliessend löst man in wenig Wasser und extrahiert die

Wasserphase nacheinander mit je 175 ml flüssigem Ionenaustauscher LA1 (HOAc-Form), Hexan und Essigester (2 x). Darauf wird die Wasserphase im Vakuum zur Trockene eingedampft und das dabei anfallende Rohprodukt in Wasser mit 1N Salzsäure auf pH 2,2 gestellt und anschliessend an 100 g silyliertem Kieselgel (Antec Opti-Up $C_{12}$) chromatographiert. Die produkthaltigen Fraktionen werden wie im Beispiel 1e) beschrieben weiterbehandelt. Man erhält die 3-(4-Carbamoylpyridiniomethyl)-7α-methoxy-7β-[2-((2R)-2-amino-2-carboxyäthoxycarbonylamino)-acetamido]-3-cephem-4-carbonsäure als Hydrat; Zersetzung ab 140°; $[\alpha]_D^{20°} = +39° \pm 1°$ (0,89% in $H_2O$); IR: 3600-2400 (breit), 1778, 1818 (Schulter), 1690, 1637, 1565, 1530 $cm^{-1}$ (Nujol); UV: 264 (9300; $H_2O$).

1k) 1,5 g 3-Acetoxymethyl-7α-methoxy-7β-[2-((2R)-2-amino-2-carboxyäthoxycarbonylamino)-acetamido]-3-cephem-4-carbonsäure-natriumsalz und 1 g 2-Methyl-3-mercapto-5,6-dioxo-1,4,5,6-tetrahydro-as-triazin werden in 8 ml Wasser wie im Beispiel 1e) beschrieben umgesetzt, aufgearbeitet und chromatographiert. Man erhält das 3-(2-Methyl-5,6-dioxo-1,4,5,6-tetrahydro-as-triazin-3-ylthiomethyl]-7α-methoxy-7β-[2-((2R)-2-amino-2-carboxyäthoxycarbonylamino)-acetamido]-3-cephem-4-carbonsäure-dinatriumsalz als Hydrat; Zersetzung ab 170°; $[\alpha]_D^{20°}$= +62°± ° (0,69% in $H_2O$); IR: 3600-2400 (breit), 1770, 1720, 1650 (breit), 1570, 1510 $cm^{-1}$ (Nujol); UV 234 (17200), 277 (23900; $H_2O$).

Das Ausgangsmaterial für Beispiel 1a)-1k) kann wie folgt hergestellt werden:

1ℓ) 20 g BOC-Glycin werden in 900 ml Tetrahydrofuran gelöst, auf -20° abgekühlt und nacheinander mit 14,5 ml N-Methylmorpholin und 14 ml Chlorameisensäure-isobutylester versetzt. Man rührt 3 Std. bei -20°, senkt darauf die Temperatur auf -40° ab, fügt 45 g 3-Acetoxymethyl-7β-amino-3-cephem-4-carbonsäurediphenylmethylester in fester

Form zu, rührt 10 Minuten bei -40°, sowie 2 1/2 Std. bei 0° und arbeitet darauf wie folgt auf: Die Reaktionsmischung wird in Essigester aufgenommen und nacheinander mit 1N wässriger Salzsäure, gesättigter wässriger $NaHCO_3$-Lösung und nochmals NaCl-Lösung bis zum Neutralpunkt gewaschen. Man trocknet die organische Phase über Natriumsulfat, dampft sie im Vakuum ein und chromatographiert den Rückstand an 2000g Kieselgel (Eluiermittel: Toluol-Essigester (1:1); Fraktionen grösse 1000 ml). Durch Vereinigung der produkthaltigen Fraktionen erhält man nach Umfällen aus $CH_2Cl_2$-Diäthyläther-Hexan den 3-Acetoxymethyl-7β-(2-BOC-aminoacetamido)-3-cephem-4-carbonsäurediphenylmethylester; $[\alpha]_D^{20°}$ = +19±°1° (0,90% in $CHCl_3$); IR: 3390, 1782, 1740 (Schulter, 1720, 1690 (Schulter), 1512 (Schulter), 1495 $cm^{-1}$ ($CH_2CH_2$); UV: 262 (7500; EtOH).

1m) 61,73 g 3-Acetoxymethyl-7β-(2-BOC-aminoacetamido)-3-cephem-4-carbonsäurediphenylmethylester werden mit 46 g p-Toluolsulfonsäuremonohydrat in 250 ml Acetonitril 2 1/2 Std. bei Zimmertemperatur gerührt. Nach Zugabe von 2500 ml Aether, Absaugen des Niederschlages, Waschen mit 1000 ml Aether und Trocknen im Vakuum erhält man das 3-Acetoxymethyl-7β-(2-aminoacetamido)-3-cephem-4-carbonsäurediphenylmethylester-p-toluolsulfonat; $[\alpha]_D^{20°}$ = +25°±1° (0,93% in $CH_3OH$); IR: 3200-2800 (breit), 1780, 1740-1720 (breit), 1700 $cm^{-1}$ ($CH_2CH_2$); UV: 261 (7200, EtOH).

Beispiel 2:

2a) 10,5 g 3-Carbamoyloxymethyl-7β-(2-aminoacetamido)-3-cephem-4-carbonsäurediphenylmethylester-p-toluolsulfonsäuresalz werden mit 12,0 g (2R)-2-BOC-Amino-2-diphenylmethoxycarbonyläthoxycarbonylchlorid wie im Beispiel 1a) beschrieben umgesetzt (2,9 ml

Pyridin, 150 ml Tetrahydrofuran), aufgearbeitet und chromatographiert. Dabei erhält man den 3-Carbamoyloxymethyl-7β-[2-((2R)-2-BOC-amino-2-diphenylmethoxycarbonyläthoxycarbonylamino)-acetamido]-3-cephem-4-carbonsäurediphenylmethylester; $[\alpha]_D^{20°} = +20°\pm1°$ (0,75% in $CHCl_3$); IR: 3460, 3370, 1782, 1740-1690 (breit), 1582, 1495 $cm^{-1}$ ($CH_2Cl_2$); UV: 257 (8200), 263 (8200; EtOH).

2b) 7,6 g 3-Carbamoyloxymethyl-7β-[2-((2R)-2-BOC-amino-2-diphenylmethoxycarbonyläthoxycarbonylamino)-acetamido]-3-cephem-4-carbonsäurediphenylmethylester werden in 760 ml Tetrahydrofuran wie im Beispiel 1b) beschrieben methoxyliert (0,253 Lithium in 45 ml Methanol; 1,478 ml t-Butylhypochlorit, aufgeteilt in zwei Portionen zu 0,562 und einer Portion zu 0,314 ml), aufgearbeitet und chromatographiert (Eluiermittel: Toluol-Essigester (1:1); Fraktionengrösse 500 ml). Man erhält den 3-Carbamoyloxymethyl-7α-methoxy-7β-[2-((2R)-2-BOC-amino-2-diphenylmethoxycarbonyläthoxycarbonylamino)-acetamido]-3-cephem-4-carbonsäure-diphenylmethylester; $[\alpha]_D^{20°} = +49°\pm1°$ (0,87% in $CHCl_3$); IR: 3480, 3390, 1780, 1740-1700 (breit), 1585, 1493 $cm^{-1}$ ($CH_2Cl_2$); UV: 246 (5000), 252 (5000), 258 (5000), 264 (5200; EtOH).

2c) 0,9 g 3-Carbamoyloxymethyl-7β-[2-((2R)-2-BOC-amino-2-diphenylmethoxycarbonyläthoxycarbonylamino)-acetamido]-3-cephem-4-carbonsäure-diphenylmethylester werden in 2 ml $CH_2Cl_2$ und 0,72 ml Anisol mit 10 ml Trifluoressigsäure analog Beispiel 1c) umgesetzt und aufgearbeitet. Man erhält das 3-Carbamoyloxymethyl-7β-[2-((2R)-2-amino-2-carboxyäthoxycarbonylamino)-acetamido]-3-cephem-4-carbonsäure-natriumsalz als Hydrat; IR: 3600-2400 (breit), 1780, 1735, 1710 (Schulter), 1612 (breit), 1538 (breit) $cm^{-1}$ (Nujol); UV: 240 (6600), 264 (7900; EtOH).

2d) 5 g 3-Carbamoyloxymethyl-7α-methoxy-7β-[2-((2R)-2-BOC-amino-2-diphenylmethoxycarbonyläthoxycarbonylamino)-acetamido]-3-cephem-4-carbonsäure-diphenylmethylester werden in 10 ml $CH_2Cl_2$ und 3,83 ml Anisol mit 50 ml Trifluoressigsäure analog Beispiel 1c) umgesetzt und aufgearbeitet. Man erhält das 3-Carbamoyloxymethyl-7α-methoxy-7β-[2-((2R)-2-amino-2-carboxyäthoxycarbonylamino)-acetamido]-3-cephem-4-carbonsäure-natriumsalz als Hydrat; Zersetzung ab 175°; $[\alpha]_D^{20°} = +143° \pm 1°$ (0,84% in $H_2O$); IR: 3600-2400 (breit), 1770, 1715 (breit), 1610 (breit), 1535 (breit) $cm^{-1}$ (Nujol); CN: 239 (6100), 263 (7400; $H_2O$).

Das Ausgangsmaterial für die Beispiele 2a)-2d) kann wie folgt hergestellt werden:

2e) 4,4 g BOC-Glycin und 10 g 3-Carbamoyloxymethyl-7β-amino-3-cephem-4-carbonsäurediphenylmethylester werden wie im Beispiel 1ℓ) beschrieben umgesetzt (3,2 ml N-Methylmorpholin; 3 ml Chlorameisensäure-isobutylester; 200 Tetrahydrofuran), aufgearbeitet und chromatographiert. Man erhält dabei den 3-Carbamoyloxymethyl-7β-(2-BOC-aminoacetamido)-3-cephem-4-carbonsöurediphenylmethylester; $[\alpha]_D^{20°} = +16° \pm 1°$ (0,70% in $CHCl_3$); IR: 3470, 3380, 1781, 1740 (Schulter), 1720-1680 (breit), 1582, 1493 $cm^{-1}$ ($CH_2Cl_2$); UV: 260 (7700; EtOH).

2f) 11,5 g 3-Carbamoyloxymethyl-7β-(2-BOC-aminoacetamido)-3-cephem-4-carbonsäurediphenylmethylester werden mit 7,4 g p-Toluolsulfonsäuremonohydrat in 100 ml Acetonitril analog Beispiel 1m) schrieben umgesetzt und aufgearbeitet. Man erhält das 3-Carbamoyloxymethyl-7β-(2-aminoacetamido)-3-cephem-4-carbonsäure-diphenylmethylester-p-toluolsulfonsäuresalz; $[\alpha]_D^{20°} = +23° \pm 1°$ (0,80% in $H_3CH$); IR: 3500-2500 (breit), 1780, 1722, 1710, 1690 (Schulter), 1603, 1537 $cm^{-1}$ (Nujol); UV: 218 (27000), 258 (6600), 261 (6600; EtOH).

Beispiel 3:

3a) 6,2 g 7β-(2-Aminoacetamido)-3-cephem-4-carbonsäure-diphenylmethylester-p-toluolsulfonsäuresalz werden mit 9 g (2R)-2-BOC-Amino-2-diphenylmethoxycarbonyläthoxycarbonylchlorid wie im Beispiel 1a) beschrieben umgesetzt (2,5 ml Pyridin;150 ml Tetrahydrofuran), aufgearbeitet und chromatographiert (Eluiermittel: Toluol-Essigester (9:1) und (1:1); Fraktionengrösse 500 ml). Man erhält den 7β-[2-((2R)-2-BOC-Amino-2-diphenylmethoxycarbonyläthoxycarbonylamino)-acetamido]-3-cephem-4-carbonsäurediphenylmethylester; IR: 3465, 3371, 1781, 1740-1690 (breit), 1581, 1494 $cm^{-1}$ ($CH_2Cl_2$).

3b) 8 g 7β-[2-((2R)-2-BOC-Amino-2-diphenylmethoxycarbonyläthoxycarbonylamino)-acetamido]-3-cephem-4-carbonsäurediphenylmethylester werden in 800 ml Tetrahydrofuran wie im Beispiel 1b) beschrieben methoxyliert (0,29 g Lithium in 30 ml Methanol; 2,2 ml t-Butylhypochlorit, aufgeteilt in zwei Portionen zu 0,8 und eine Portion zu 0,6 ml), aufgearbeitet und chromatographiert (Eluiermittel: Toluol-Essigester (4:1); Fraktionengrösse 500 ml). Man erhält den 7α-Methoxy-7β-[2-((2R)-2-BOC-amino-2-diphenylmethoxycarbonyläthoxycarbonylamino)-acetamido]-3-cephem-4-carbonsäurediphenylmethylester; $[\alpha]_D^{20°} = +78°\pm1°$ (0,87% in EtOH); IR: 3464, 3368, 1779, 1740-1690 (breit), 1581, 1494 $cm^{-1}$ ($CH_2Cl_2$); UV: 251 (5000), 257 (4900), 264 (4700), 268 (4500; EtOH).

3c) 1,55 g 7α-Methoxy-7β-[2-((2R)-2-BOC-amino-2-diphenylmethoxycarbonyläthoxycarbonylamino)-acetamido]-3-cephem-4-carbonsäurediphenylmethylester werden in 3 ml $CH_2Cl_2$ und 1,3 ml Anisol mit 10 ml Trifluoressigsäure analog Beispiel 1c) umgesetzt und aufgearbeitet. Man erhält das 7α-Methoxy-7β-[2-((2R)-2-amino-2-carboxyäthoxycarbonylamino)-acetamido]-3-cephem-4-carbonsäure-natriumsalz als Hydrat; Zersetzung ab 160°; $[\alpha]_D^{20°} = +173°\pm1°$ (0,62% in $H_2O$); IR: 3600-2400 (breit), 1779, 1710 (breit), 1620 (breit), 1530 (breit) $cm^{-1}$ (Nujol); UV: 261 (3440; $H_2O$).

Das Ausgangsmaterial für die Beispiele 3a)-3c) kann wie folgt hergestellt werden:

3d) 3,6 g BOC-Glycin und 7 g 7β-Amino-3-cephem-4-carbonsäure-diphenylmethylester werden wie im Beispiel 1l) beschrieben umgesetzt (2,7 ml N-Methylmorpholin; 2,6 ml Chlorameisensäure-isobutylester; 160 ml Tetrahydrofuran), aufgearbeitet und chromatographiert (Eluiermittel: Toluol-Essigester (4:1); Fraktionengrösse 500 ml). Man erhält dabei den 7β-(2-BOC-Aminoacetamido)-3-cephem-4-carbonsäure-diphenylmethylester; $[\alpha]_D^{20°} = 1° \pm 1°$ (1,30% in $CHCl_3$); IR: 3475, 3380, 1782, 1740 (Schulter), 1720-1690 (breit), 1580, 1495 $cm^{-1}$ ($CH_2Cl_2$).

3e) 6,8 g 7β-(2-BOC-Aminoacetamido)-3-cephem-4-carbonsäure-diphenylmethylester werden mit 4,9 g p-Toluolsulfonsäuremonohydrat in 70 ml Acetonitril wie im Beispiel 1m) beschrieben umgesetzt und aufgearbeitet. Man erhält das 7β-(2-Aminoacetamido)-3-cephem-4-carbonsäure-diphenylmethylester-p-toluolsulfonsäuresalz, das ohne Charakterisierung weiterverarbeitet wird (Beispiel 3a).

Beispiel 4:

4a) 45,5 g (2R)-2-((2R)-2-BOC-Amino-2-diphenylmethoxycarbonyläthoxycarbonylamino)-2-phenylessigsäure werden in 1200 ml Tetrahydrofuran gelöst, auf 20° abgekühlt und nacheinander mit 9,6 ml N-Methylmorpholin und 9,1 ml Chlorameisensäure-isobutylester versetzt. Man rührt 3 Std. bei -20°, senkt darauf die Temperatur auf -40° ab, fügt 29 g 3-Acetoxymethyl-7β-amino-3-cephem-4-carbonsäurediphenylmethylester in fester Form zu, rührt 10 min bei -40°, sowie 2 1/2 Std. bei 0° und arbeitet wie im Beispiel 1a) beschrieben auf. Das erhaltene Rohprodukt chromatographiert man an 2000 g Kieselgel (Eluiermittel: Methylenchlorid-Essigester (98:2); Fraktionengrösse 1000 ml). Durch Vereinigen der produkthaltigen Fraktionen und Umfällen aus Methylenchlorid-Hexan erhält man den 3-Acetoxymethyl-7β-[(2R)-2-((2R)-2-BOC-amino-2-diphenylmethoxycarbonyläthoxycarbonylamino)-2-phenylacetamido]-3-cephem-4-carbonsäure-diphenylmethylester; $[\alpha]_D^{20°} = -1° \pm 1°$ (1,30% in $CHCl_3$); IR: 3490, 1785, 1745, 1690 (breit), 1595 $cm^{-1}$ ($CH_2Cl_2$); UV: 257 (7200), 263 (7100; EtOH).

4b) 7,3 g 3-Acetoxymethyl-7β-[(2R)-2-((2R)-2-BOC-amino-2-diphenylmethoxycarbonyläthoxycarbonylamino)-2-phenylacetamido]-3-cephem-4-carbonsäure-diphenylmethylester werden in 730 ml Tetrahydrofuran wie im Beispiel 1b) beschrieben methoxyliert (0,21 g Lithium in 30 ml Methanol; 1,7 ml t-Butylhypochlorit, aufgeteilt in zwei Portionen zu 0,6 und 1 Portion zu 0,5 ml), aufgearbeitet und chromatographiert (Eluiermittel: Toluol-Essigester (4:1). Man erhält den 3-Acetoxymethyl-7α-methoxy-7β-[(2R)-2-((2R)-2-BOC-amino-2-diphenylmethoxycarbonyläthoxycarbonylamino)-2-phenylacetamido]-3-cephem-4-carbonsäure-diphenylmethylester; IR: 3360, 1780,1722 (breit), 1495 $cm^{-1}$ (Nujol); UV: 246 (6400), 252 (6500), 258 (6600), 264 (6600; EtOH).

4c) - 6,6 g 3-Acetoxymethyl-7α-methoxy-7β-[(2R)-2-((2R)-2-BOC-amino-2-diphenylmethoxycarbonyl-äthoxycarbonylamino)-2-phenylacetamido]-3-cephem-4-carbonsäure-diphenylmethylester werden in 10 ml $CH_2Cl_2$ und 4,3 ml Anisol mit 20 ml Trifluoressigsäure analog Beispiel 1c) umgesetzt und aufgearbeitet. Man erhält das 3-Acetoxymethyl-7α-methoxy-7β-[(2R)-((2R)-2-amino-2-carboxyäthoxycarbonylamino)-2-phenylacetamido)-3-cephem-4-carbonsäure-natriumsalz als Hydrat; Zersetzung ab 160°; $[\alpha]_D^{20°} = +73° \pm 1°$ (0,63% in $H_2O$); IR: 3500-2500 (breit), 1775, 1732, 1712 (Schulter), 1700 (Schulter), 1625 (breit), 1525 (breit) $cm^{-1}$ (Nujol); UV: 263 (6360; $H_2O$).

Das Ausgangsmaterial für die Beispiele 4a)-4c) kann wie folgt hergestellt werden:

4d) 12,5 g D-Phenylglycin werden in 300 ml $CH_3CN$-$H_2O$-(1:1)-Gemisch suspendiert. Dann wird mit 1N Natronlauge auf pH 10,5 gestellt, wobei sich eine klare Lösung bildet. Zu dieser tropft man anschliessend im Verlaufe von 20 min bei 0° eine Lösung von 39,4 g (2R)-2-BOC-Amino-2-diphenylmethoxycarbonyläthoxy-carbonylchlorid, wobei das pH durch gleichzeitige Zugabe von 1N NaOH (Titrator) bei pH 7,5 konstant gehalten wird. Anschliessend rührt man bei gleichem pH eine weitere Stunde bei 0°. Dann stellt man durch Zugabe von konz. Salzsäure auf pH 3,

extrahiert sofort mit Essigester, wäscht viermal mit gesättigter wässriger NaCl-Lösung, trocknet über Natriumsulfat und dampft im Vakuum ein. Man erhält die (2R)-2-((2R)-2-BOC-Amino-2-diphenylmethoxycarbonyläthoxycarbonylamino)-2-phenylessigsäure, die ohne Charakterisierung weiterverarbeitet wird (Beispiel 4a).

Beispiel 5:

5a) 21 g 3-Acetoxymethyl-7β-((2R)-2-amino-2-methylacetamido)-3-cephem-4-carbonsäurediphenylmethylester-p-toluolsulfonat werden mit 23,4 g (2R)-2-BOC-Amino-2-diphenylmethyloxycarbonyläthoxycarbonylchlorid wie im Beispiel 1a) beschrieben umgesetzt (5,7 ml Pyridin; 300 ml Tetrahydrofuran), aufgearbeitet und chromatographiert (Eluiermittel: Toluol-Essigester (3:2), Fraktionengrösse 1000 ml). Dabei erhält man den 3-Acetoxymethyl-7β-[(2R)-2-((2R)-2-BOC-amino-2-diphenylmethoxycarbonyläthoxycarbonylamino)-2-methylacetamido]-3-cephem-4-carbonsäurediphenylmethylester; $[\alpha]_D^{20°}$ = +20°±1° (0,79% in $CHCl_3$); IR: 3370, 1790, 1722 (breit), 1495 $cm^{-1}$ ($CH_2Cl_2$); UV 258 (7600; EtOH).

5b) 16,5 g 3-Acetoxymethyl-7β-[(2R)-2-((2R)-2-BOC-amino-2-diphenylmethoxycarbonyläthoxycarbonylamino)-2-methylacetamido]-3-cephem-4-carbonsäure-diphenylmethylester werden in 1650 ml Tetrahydrofuran wie im Beispiel 1b) beschrieben methoxyliert (0,542 g Lithium, in 96,5 ml Methanol; 3,1 ml t-Butylhypochlorit, aufgeteilt in 2 Portionen zu 1,20 und 1 Portion zu 0,70 ml), aufgearbeitet und chromatographiert (Eluiermittel: Toluol-Essigester (4:1)). Man erhält den 3-Acetoxymethyl-7α-methoxy-7β-[(2R)-2-((2R)-2-BOC-amino-2-diphenylmethoxycarbonyläthoxycarbonylamino)-2-methylacetamido]-3-cephem-4-carbonsäure-diphenylmethylester; $[\alpha]_D^{20°}$ = +63°±1° (0,88% in $CHCl_3$); IR: 3380, 1782, 1740-1700 (breit), 1495 $cm^{-1}$ ($CH_2Cl_2$); UV: 246 (5400), 251 (5400), 257 (5500), 263 (5600), 267 (5500); EtOH).

5c) 14 g 3-Acetoxymethyl-7α-methoxy-7β-[(2R)-2-((2R)-2-BOC-amino-2-diphenylmethoxycarbonyläthoxycarbonylamino)-2-methylacetamido]-3-cephem-4-carbonsäure-diphenylmethylester werden in 27,5 ml $CH_2Cl_2$ und 10,5 ml Anisol mit 138 ml Trifluoressigsäure analog Beispiel 1c) umgesetzt und aufgearbeitet. Man erhält das 3-Acetoxymethyl-7α-methoxy-7β-[(2R)-2-((2R)-2-amino-2-carboxyäthoxycarbonylamino)-2-methylacetamido]-3-cephem-4-carbonsäure-natriumsalz als Hydrat; Zersetzung ab 160°; $[\alpha]_D^{20°}$ = +160°±1° (0,74% in $H_2O$); IR: 3600-2400 (breit), 1772, 1720, 1690 (Schulter), 1610, 1530 $cm^{-1}$ (Nujol); UV: 239 (6300), 254 (7400; $H_2O$).

5d) 1,77 g 3-Acetoxymethyl-7α-methoxy-7β-[(2R)-2-((2R)-2-amino-2-carboxyäthoxycarbonylamino)-2-methylacetamido]-3-cephem-4-carbonsäure-natriumsalz werden mit 1 g 1-Methyl-5-mercapto-1H-tetrazol-natriumsalz wie im Beispiel 1e) beschrieben umgesetzt. Man erhält das 3-(1-Methyl-1H-tetrazol-5-ylthiomethyl)-7α-methoxy-7β-[(2R)-2-((2R)-2-amino-2-carboxyäthoxycarbonylamino)-2-methylacetamido]-3-cephem-4-carbonsäure-natriumsalz als Hydrat; Zersetzung ab 165°; $[\alpha]_D^{20}$ = +91°±1° (0,76% in $H_2O$); IR: 3610-2400 (breit), 1771, 1722, 1690 (Schulter), 1610, 1530 $cm^{-1}$ (Nujol); UV: 270 (7400; $H_2O$).

Das Ausgangsmaterial für die Beispiele 5a)-5d) kann wie folgt hergestellt werden:

5e) 7,54 g BOC-(D)-Alanin und 15,8 g 3-Acetoxymethyl-7β-amino-3-cephem-4-carbonsäure-diphenylmethylester werden wie im Beispiel 1ℓ) beschrieben umgesetzt (5,11 ml N-Methylmorpholin; 4,94 ml Chlorameisensäure-isobutylester; 300 ml Tetrahydrofuran), aufgearbeitet und chromatographiert (Eluiermittel: Toluol-Essigester (4:1); Fraktionengrösse 1000 ml). Man erhält dabei den 3-Acetoxymethyl-7β-((2R)-2-BOC-amino-2-methylacetamido)-3-cephem-4-carbonsäurediphenylmethylester $[\alpha]_D^{20°}$ = +17°± 1° (0,93% in $CHCl_3$); IR: 3390, 1788, 1722 (breit), 1500 $cm^{-1}$ ($CH_2Cl_2$); UV: 264 (7000; EtOH).

5f) 21 g 3-Acetoxymethyl-7β-((2R)-2-BOC-amino-2-methylacetamido)-3-cephem-4-carbonsäure-diphenylmethylester werden mit 13,1 g p-Toluolsulfonsäuremonohydrat in 50 ml Acetonitril wie im Beispiel 1m) beschrieben umgesetzt und aufgearbeitet. Man erhält das 3-Acetoxymethyl-7β-((2R)-2-amino-2-methylacetamido)-3-cephem-4-carbonsäure-diphenylmethylester-p-toluolsulfonat; $[\alpha]_D^{20°} = +24° \pm 1°$ (0,73% in $CH_3OH$); IR: 3600-2400 (breit), 1785, 1725, 1690, 1550 (breit), 1485 $cm^{-1}$ (Nujol); UV 218 (25800), 260 (6800; EtOH).

Beispiel 6:

6a) Eine auf -20° gekühlte Lösung von 5,79 g (3R)-3-BOC-amino-3-t.Butyloxycarbonylpropionsäure und 2,2 ml N-Methylmorpholin in 150 ml absolutem Methylenchlorid wird unter Stickstoffatmosphäre mit 2,6 ml Chlorameisensäureisobutylester und nach einer Stunde mit 13,36 g 3-Acetoxymethyl-7β-(2-aminoacetamido)-3-cephem-4-carbonsäure-diphenylmethylester-p-toluolsulfonsäuresalz und 2,2 ml N-Methylmorpholin versetzt. Nach 3 Stunden Rühren bei Raumtemperatur wird das Reaktionsgemisch mit 800 ml Essigester verdünnt, je zweimal mit Eiswasser und gesättigter Natriumchloridlösung gewaschen, über Natriumsulfat getrocknet und am Rotationsverdampfer von Lösungsmitteln befreit. Der Rückstand wird an Silicagel mit Diäthyläther-Essigester (1:1) als Eluiermittel gereinigt, woraus 3-Acetoxymethyl-7β-[2-((3R)-3-BOC-amino-3-t.butyloxycarbonylpropionylamino)-acetamido]-3-cephem-4-carbonsäurediphenylmethylester als farbloses amorphes Pulver erhalten wird. DC (Silicagel, Identifikation mit Jod): $R_f \sim 0,44$ (Essigester).

6b) Zu einer auf -75° gekühlten Lösung von 9,97 g der gemäss Beispiel 6a) erhaltenen Verbindung in 1000 ml absolutem Tetrahydrofuran wird unter Rühren und Stickstoffatmosphäre eine Lösung von 361 mg Lithium in 72 ml absolutem Methanol innerhalb 2 Min. gegeben. Dann werden zum Reaktionsgemisch nach 2,5 und 15 Minuten 2,1 ml, 0,8 ml und 0,5 ml t.Butylhypochlorit gegeben. Nach weiteren 15 Minuten bei -75° wird das Reaktionsgemisch mit 17,9 ml Essigsäure und anschliessend

mit 4,55 g Natriumthiosulfat gelöst in 6,5 ml Wasser versetzt und auf 0° aufgewärmt. Die mit Essigester verdünnte Lösung wird je zweimal mit Eiswasser, 5%-iger Natriumbicarbonat- und gesättigter Natriumchloridlösung gewaschen, über Natriumsulfat getrocknet und am Rotationsverdampfer von den Lösungsmitteln befreit. Der Rückstand wird an Silicagel mit Diäthyläther-Essigester 1:1 als Eluiermittel gereinigt, woraus 3-Acetoxymethyl-7α-methoxy-7β-[2-((3R)-3-BOC-amino-3-t.butyloxycarbonylpropionylamino)-acetamido]-3-cephem-4-carbonsäure-diphenylmethylester als dünnschichtchromatographisch einheitliches amorphes Pulver erhalten wird. DC (Silicagel, Identifikation mit Jod): $R_f \sim 0,47$ (Essigester).

6c) Eine Lösung von 4,3 g der gemäss Beispiel 6b) erhaltenen Verbindung in 43 ml Trifluoressigsäure wird in Gegenwart von 4,3 ml Anisol während 90 Minuten bei Raumtemperatur unter einer Stickstoffatmosphäre gerührt. Nach Zugabe von 400 ml Diäthyläther wird der entsprechende Niederschlag abfiltriert, mit wenig Diäthyläther gewaschen und unter vermindertem Druck getrocknet. Das so erhaltene Trifluoressigsäuresalz wird in 20 ml Eiswasser gelöst und mit Essigester (3x10 ml) extrahiert. Die saure wässrige Phase wird durch tropfenweise Zugabe von 2N wässriger Natronlauge auf pH 5,5 gestellt und bei 0° mit Isopropanol (100 ml) versetzt. Der gebildete Niederschlag wird abfiltriert, mit Aethanol gewaschen, zur Entfernung organischer Lösungsmittel nochmals in Wasser gelöst und am Rotationsverdampfer eingeengt. Nach dem Trocknen (16 Stunden, Raumtemperatur 0,05 Torr) wird 3-Acetoxymethyl-7α-methoxy-7β-[2-((3R)-3-amino-3-carboxylpropionylamino)-acetamido]-3-cephem-4-carbonsäure-natriumsalz in Form eines 1,5-Hydrats erhalten; F: ab 168° (unter Zersetzung); DC (Silicagel, Entwickeln mit Ninhydrin): $R_f \sim 0,16$; UV-Spektrum (0,1 N wässrige Salzsäure): 264 nm ($\varepsilon$ = 7000 ); $[\alpha]_D$ =+116 $\pm$ 1° (0,1 N wässrige Salzsäure, c = 1,22 %).

6d) In analoger Weise zu Beispiel 6c) erhält man durch Behandeln von 1,9 g 3-Acetoxymethyl-7β-[2-((3R)-3-BOC-amino-3-t.butyloxy-carbonylpropionylamino)-acetamido]-3-cephem-4-carbonsäurediphenylmethylester mit 20 ml Trifluoressigsäure und 2 ml Anisol 3-Acetoxy-methyl-7β-[2-[(3R)-3-amino-3-carboxylpropionylamino)-acetamido]-3-cephem-4-carbonsäure in Form eines 1.5 Hydrats; F. ab 132° (unter Zersetzung); DC (Silicagel, Entwickeln mit Ninhydrin); $R_f$ ~0,16; UV-Spektrum (0,1 N wässriger Salzsäure): 258 nm (ε= 7500); $[\alpha]_D$ = +72 ± 1° (0,1 N wässriger Salzsäure, c = 1,22%).

Beispiel 7:

7a) Zu einer auf -75° gekühlten Lösung von 4,76 g 3-Acetoxy-methyl-7β-[2-BOC-amino-acetamido]-3-cephem-4-carbonsäure-diphenylmethylester in 476 ml absolutem Tetrahydrofuran wird unter Stickstoffatmosphäre eine Lösung von 222 mg Lithium in 44 ml absolutem Methanol innert ca. 2 Minuten unter starkem Rühren gegeben. Darauf werden ebenfalls bei -75° nach 2 und 5 Minuten je 0,5 ml und nach 15 Minuten 0,3 ml tert.-Butylhypochlorit zugegeben. Nach weiteren 15 Minuten wird das Reaktionsgemisch mit 11 ml Essigsäure und anschliessend mit 2,8 g Natriumthiosulfat gelöst in 4 ml Wasser versetzt und auf Raumtemperatur aufgewärmt. Die mit Essigester verdünnte Lösung wird je zweimal mit Eiswasser, 5%-iger Natriumbicarbonatlösung und gesättigter Natriumchloridlösung gewaschen, über Natriumsulfat getrocknet und am Rotationsverdampfer von Lösungsmittel befreit. Das Rohprodukt wird an Silicagel mit Methylenchlorid-Essigester 3:1 als Eluiermittel gereinigt, woraus 3-Acetoxymethyl-7α-methoxy-7β-(2-BOC-aminoacetamido)-3-cephem-4-carbonsäurediphenylmethylester erhalten wird; F: 124-126° (aus Diäthyläther); DC (Silicagel, Identifikation mit Jod): $R_f$ 0,71; (Diäthyläther-Essigester 201).

7b) Eine Lösung von 1,93 g 3-Acetoxymethyl-7α-methoxy-7β-(2-BOC-amino-acetamido)-3-cephem-4-carbonsäure-diphenylmethylester und 1,1 g p-Toluolsulfonsäure-monohydrat in 15 ml absolutem Acetonitril wird während 3 1/2 Stunden bei Raumtemperatur gerührt. Nach der Zugabe von Diäthyläther (90 ml) wird der gebildete Niederschlag

abfiltriert, nochmals mit Diäthyläther (30 ml) verrührt und nach Filtration am Hochvakuum getrocknet. Das so erhaltene 3-Acetoxymethyl-7α-methoxy-7β-(2-aminoacetamido)-3-cephem-4-carbonsäurediphenylmethylester-p-toluolsulfonsäuresalz kann ohne weitere Reinigung im nächsten Reaktionsschritt eingesetzt werden; F: ab 110° (Zersetzung); DC (Silicagel, Identifikation mit Jod): $R_f$~ 0,10 ; (Essigester-Aethanol 9:1).

7c) Analog Beispiel 6a) erhält man durch Behandeln des gemischten Anhydrids,hergestellt aus 637 mg (3R)-3-BOC-amino-3-t.-butyloxycarbonylpropionsäure und 0,29 ml Chlorameisensäureisobutylester in Gegenwart von 0,24 ml N-Methylmorpholin in 20 ml absolutem Methylenchlorid bei -15° mit 1,4 g 3-Acetoxymethyl-7α-methoxy-7β-(2-aminoacetamido)-3-cephem-4-carbonsäurediphenylmethylester-p-toluolsulfonsäuresalz, gefolgt von 0,24 ml N-Methylmorpholin, 3-Acetoxymethyl-7α-methoxy-7β-[2-((3R)-3-BOC-amino-3-t. butyloxycaebonylpropionylamino)-acetamido]-3-cephem-4-carbonsäurediphenylmethylester.

Beispiel 8:

8a) 17,8 g (2R,S)-2-((2R)-2-BOC-Amino-2-diphenylmethoxycarbonyläthoxycarbonylamino)-2-(2-trichloräthoxycarbonylaminothiazol-4-yl)-essigsäure und 9,4 g 3-Acetoxymethyl-7β-amino-3-cephem-4-carbonsäurediphenylmethylester werden in 180 ml absolutem Tetrahydrofuran zusammen mit 1,8 g Hydroxybenztriazol gelöst. Dann gibt man sofort, sowie nach 1 1/2 und nach 3 Stunden, je 1,63 g Dicyclohexylcarbodiimid in je 50 ml Tetrahydrofuran zu und rührt insgesamt 6 Stunden bei Zimmertemperatur. Das Reaktionsgemisch wird auf 4 l Hexan gegossen, abgenutscht und mit Hexan gewaschen. Der Nutschrückstand wird in 4 l Essigester gegeben und gerührt. Der im Essigester unlösliche Dicyclohexylharnstoff wird abfiltriert und die Essigesterlösung nacheinander mit gesättigten Natriumhydrogencarbonat- und Kochsalzlösungen gewaschen. Nach Trocknen über Natriumsulfat und Eindampfen fällt man das erhaltene Rohprodukt einmal aus Methylenchlorid-Aether-Hexan um, wobei der 3-Acetoxymethyl-7β-[(2R,S)-2-((2R)-2-BOC-amino-2-diphenylmethoxycarbonyläthoxycarbonylamino)-2-(2-trichlor-

äthoxycarbonylaminothiazol-4-yl)-acetamido]-3-cephem-4-carbonsäurediphenylmethylester erhalten wird; $[\alpha]_D^{20°}$ =+5° ± 1° (1,01 % in $C_2H_5OH$); IR: 3300-3400, 1787, 1740-1690 (breit, 1495 $cm^{-1}$ ($CH_2Cl_2$); UV: 258 ( 15300, $C_2H_5OH$).

Falls gewünscht, kann das resultierende binäre (2R,S)-Produktgemisch mittels Chromatographie an Kieselgel in seine (2R)- und (2S)-Enantiomeren aufgetrennt werden.

8b): Zu einer Lösung von 19 g 3-Acetoxymethyl-7β-[(2R,S)-2-((2R)-BOC-amino-2-diphenylmethoxycarbonyläthoxycarbonylamino)-2-(2-trichloräthoxycarbonylaminothiazol-4-yl)-acetamido]-3-cephem-4-carbonsäurediphenylmethylester in 190 ml eines Essigsäure-Acetonitril-Gemisch (1:1) gibt man unter Rühren bei 0° im Verlauf von 10 Minuten portionenweise 19 g Zink-Staub zu und rührt anschliessend weitere 3 Stunden bei 0°. Dann wird die Reaktionslösung vom Zinkrückstand abgenutscht, mit Acetonitril nachgewaschen und im Rotationsverdampfer eingeengt. Der Rückstand wird mit Wasser versetzt, mit 2N NaOH auf pH 8 gestellt, mit Essigester extrahiert und mit NaCl-Lösung neutral gewaschen. Das nach dem Trocknen über Natriumsulfat und Eindampfen erhaltene Rohprodukt chromatographiert man an 900 g Kieselgel, wobei Fraktionen à 500 ml genommen werden. Eluiermittel: Aether, Aether-Essigester-(9:1), Aether-Essigester-(1:1) und Essigester. Durch Vereinigen und Eindampfen der Produkt-haltigen Fraktionen, sowie Umfällung des Eindampfrückstandes aus $CH_2Cl_2$-Hexan, erhält man den 3-Acetoxymethyl-7β-[(2R,S)-2-((2R)-2-BOC-amino-2-diphenylmethoxycarbonyläthoxycarbonylamino)-2-(2-aminothiazol-4-yl)-acetamido]-3-cephem-4-carbonsäurediphenylmethylester; $[\alpha]_D^{20°}$ = +31° ± 1° (1,07% in $CHCl_3$); IR: 3370, 1785, 1740-1696 (breit), 1600, 1595 $cm^{-1}$ ($CH_2Cl_2$); UV: 256 (11000; EtOH).

8c): 5 g 3-Acetoxymethyl-7β-[(2R,S)-2-((2R)-2-BOC-amino-2-diphenylmethoxycarbonyläthoxycarbonylamino-2-(2-aminothiazol-4-yl)-acetamido]-3-cephem-4-carbonsäurediphenylmethylester werden in 10 ml

$CH_2Cl_2$ und 6,5 ml Anisol mit 30 ml Trifluoressigsäure analog Beispiel 1c) umgesetzt und aufgearbeitet. Man erhält das Hydrat des 3-Acetoxymethyl-7β-[(2R,S)-2-((2R)-2-amino-2-carboxyäthoxycarbonylamino)-2-(2-aminothiazol-4-yl)-acetamido]-3-cephem-4-carbonsäurenatriumsalzes; Zersetzung ab 150°; $[\alpha]_D^{20°}$ = +103° ± 1° (0,90% in $H_2O$); IR: 3600-3100 (breit), 1760, 1720, 1680, 1615, 1527, 1460 $cm^{-1}$ (Nujol); UV: 252 (11200; $H_2O$).

8d) 1,8 g 3-Acetoxymethyl-7β-[(2R,S)-2-((2R)-2-amino-2-carboxyäthoxycarbonylamino)-2-(2-aminothiazol-4-yl)-acetamido]-3-cephem-4-carbonsäurenatriumsalz und 0,86 g 1-Methyl-5-mercapto-1H-tetrazol-natriumsalz werden in 10 ml Wasser wie im Beispiel 1e) beschrieben umgesetzt, aufgearbeitet und chromatographiert. Man erhält das Hydrat des 3-(1-Methyl-1H-tetrazol-5-ylthiomethyl)-7β-[(2R,S)-2-((2R)-2-amino-2-carboxyäthoxycarbonylamino)-2-(2-aminothiazol-4-yl)-acetamido]-3-cephem-4-carbonsäure-natriumsalz; Zersetzung ab 220° $[\alpha]_D^{20°}$ = +18° ± 1° (0,85% in $H_2O$); IR: 3600-3080 (breit), 1760, 1713, 1680, 1609, 1520, 1460 $cm^{-1}$ (Nujol); UV: 256 (10500; $H_2O$).

Das Ausgangsmaterial für die Beispiele 8a)-8d) kann wie folgt hergestellt werden:

8e) 10 g (2R,S)-2-(2-Trichloräthoxycarbonylaminothiazol-4-yl)-glycin, das man gemäss der Japanischen Patentanmeldung 52,083,836 (DerwentNr. 61588Y/35) erhält, werden in 250 ml Acetonitril-Wasser (1:1) suspendiert und durch Zugabe von 1N Natronlauge bei pH 9,5 in Lösung gebracht. Dann tropft man bei 0° unter Rühren 13,6 g (2R)-2-BOC-Amino-2-diphenylmethoxycarbonyläthoxycarbonylchlorid in 100 ml Acetonitril im Verlauf von 15 Minuten zu, wobei das pH durch Zugabe von 1N Natronlauge auf 9,5 konstant gehalten wird. Unter den gleichen Bedingungen rührt man 3 Stunden nach, stellt mit 2N Salzsäure auf pH 3, extrahiert mit Essigester, wäscht viermal mit gesättigter wässriger NaCl-Lösung, trocknet über $Na_2SO_4$ und dampft im Vakuum ein. Dabei erhält man die (2R,S)-2-[(2R)-2-BOC-Amino-2-diphenyl-

methoxycarbonyläthoxycarbonylamino]-2-[2-trichloräthoxycarbonylamidothiazol-4-yl]-essigsäure, welche ohne Charakterisierung roh weiterverarbeitet wird.

Sollen anstelle der im Beispiel 8a-d) beschriebenen 7β-[(2R,S)-2-((2R)-2-Amino-2-carboxyäthoxycarbonylamino)-2-(2-aminothiazol-4-yl)-acetamido-Derivate die entsprechenden (R)- bzw. (S)-Verbindungen synthetisiert werden, so ist das racemische Ausgangsmaterial in an sich bekannter Weise in seine Antipoden zu trennen.

Beispiel 9:

9a) 30 g 3-Acetoxymethyl-7β-[(2R,S)-2-((2R)-2-BOC-amino-2-diphenylmethoxycarbonyläthoxycarbonylamino)-2-(2-trichloräthoxycarbonylaminothiazol-4-yl)-acetamido]-3-cephem-4-carbonsäurediphenylmethylester (Herstellung siehe Beispiel 8a)) werden an 2 kg Kieselgel chromatographiert (Fraktionen à 1 Liter). Dabei eluiert man in den Fraktionen 33-36 (Eluiermittel: Toluol-Essigester-85:15-Gemisch) 3-Acetoxymethyl-7β-[(2R)-2-((2R)-2-BOC-amino-2-diphenylmethoxycarbonyläthoxycarbonylamino)-2-(2-trichloräthoxycarbonylaminothiazol-4-yl)-acetamido]-3-cephem-4-carbonsäurediphenylmethylester.

Die Konfigurationszuordnung am α-Kohlenstoffatom des Acetylrestes in der 7β-Acylseitenkette erfolgt aufgrund von Drehungsverschiebungen und NMR-Vergleichen (CH-7) mit Ureidocephalosporinen, vgl. z.B. H. Breuer et al., J. Antibiot. 31, 546-560, sowie DOS 2,924,296; $[\alpha]_D^{20}$: -7° ± 1° (0,87% in $CHCl_3$); IR: 3300-3400, 1787, 1740-1690, 1495 $cm^{-1}$ ($CH_2CH_2$); UV: 258 (15300; $C_2H_5OH$).

Die anschliessend eluierten Fraktionen 37-45 bestehen aus einem binären Gemisch der obigen (2R)-Verbindung mit dem entsprechenden (2S)-Isomeren (vgl. Fraktionen 46-60).

Aus den Fraktionen 46-60 erhält man den 3-Acetoxymethyl-7β-[(2S)-2-((2R)-2-BOC-amino-2-diphenylmethoxycarbonyläthoxycarbonylamino)-2-(2-trichloräthoxycarbonylaminothiazol-4-yl)-acetamido]-3-cephem-4-carbonsäurediphenylmethylester. Die Konfiguration am α-Kohlenstoffatom in der 7β-Acylseitenkette erfolgt nach der oben beschriebenen Methode; $[\alpha]_D^{20°}$ = +25° ± 1° (0,76% in $CHCl_3$); IR: 3300-3400, 1787, 1740-1690, 1495 $cm^{-1}$ ($CH_2Cl_2$); UV: 258 (15000; $C_2H_5OH$).

9b) 4,68 g 3-Acetoxymethyl-7β-[(2R)-2-((2R)-2-BOC-amino-2-diphenylmethoxycarbonyläthoxycarbonylamino)-2-(2-trichloräthoxycarbonylaminothiazol-4-yl)-acetamido]-3-cephem-4-carbonsäurediphenylmethylester werden mit 4,68 g Zinkstaub in 46,8 ml Essigsäure-Acetonitril-(1:1)-Gemisch analog Beispiel 8b) umgesetzt, aufgearbeitet und chromatographiert. Dabei erhält man den 3-Acetoxymethyl-7β-[(2R)-2-((2R)-2-BOC-amino-2-diphenylmethoxycarbonyläthoxycarbonylamino)-2-(2-aminothiazol-4-yl)-acetamido]-3-cephem-4-carbonsäurediphenylmethylester; $[\alpha]_D^{20°}$ = -15° ± 1° (0,92% in $CHCl_3$); IR: 3370, 1785, 1740-1696, 1600, 1595 $cm^{-1}$ ($CH_2Cl_2$); UV: 256 (11500; $C_2H_5OH$).

9c) 6,54 g 3-Acetoxymethyl-7β-[(2S)-2-((2R)-2-BOC-amino-2-diphenylmethoxycarbonyläthoxycarbonylamino)-2-(2-trichloräthoxycarbonylaminothiazol-4-yl)-acetamido]-3-cephem-4-carbonsäure-diphenylmethylester werden mit 6,54 g Zinkstaub in 65,4 ml Essigsäure-Acetonitril-(1:1)-Gemisch analog zu Beispiel 8b) umgesetzt, aufgearbeitet und chromatographiert. Dabei erhält man den 3-Acetoxymethyl-7β-[(2S)-2-((2R)-2-BOC-amino-2-diphenylmethoxycarbonyläthoxycarbonylamino)-2-(2-aminothiazol-4-yl)-acetamido]-3-cephem-4-carbonsäurediphenylmethylester; $[\alpha]_D^{20°}$ = 20° ± 1° (0,69% in $CHCl_3$); IR: 3370, 1785, 1740-1696, 1600, 1595 $cm^{-1}$ ($CH_2Cl_2$); UV: 256 (10900; $C_2H_5OH$).

9d) 2,9 g 3-Acetoxymethyl-7β-[(2R)-2-((2R)-2-BOC-amino-2-diphenylmethoxycarbonyläthoxycarbonylamino)-2-(2-aminothiazol-4-yl)-acetamido]-3-cephem-4-carbonsäurediphenylmethylester werden in 5 ml $CH_2Cl_2$ und 2,5 ml Anisol mit 20 ml Trifluoressigsäure analog Beispiel 1c) umgesetzt und aufgearbeitet. Man erhält das Hydrat des 3-Acetoxymethyl-7β-[(2R)-2-((2R)-2-amino-2-carboxyäthoxycarbonylamino-2-(2-aminothiazol-4-yl)-acetamido]-3-cephem-4-carbonsäurenatriumsalzes; Zersetzung ab 165°C; $[\alpha]_D^{20°}$ = +52° ± 1° (0,84% in $H_2O$); IR: 3600-3100, 1760, 1720, 1680, 1615, 1527, 1460 $cm^{-1}$ (Nujol); UV: 252 (11500); $H_2O$).

9e) 3,95 g 3-Acetoxymethyl-7β-[(2R)-2-((2R)-2-BOC-amino-2-diphenylmethoxycarbonyläthoxycarbonylamino)-2-(2-aminothiazol-4-yl)-acetamido]-3-cephem-4-carbonsäurediphenylmethylester werden in 7 ml $CH_2Cl_2$ und 4,5 ml Anisol mit 25 ml Trifluoressigsäure analog Beispiel 1c) umgesetzt und aufgearbeitet. Man erhält das Hydrat des 3-Acetoxymethyl-7β-[(2S)-2-((2R)-2-amino-2-carboxyäthoxycarbonylamino-2-(2-aminothiazol-4-yl)-acetamido]-3-cephem-4-carbonsäurenatriumsalzes; Zersetzung ab 165°; $[\alpha]_D^{20°}$ = +86° ± 1° (0,85% in $H_2O$); IR: 3600-3100, 1760, 1720, 1680, 1615, 1527, 1460 $cm^{-1}$ (Nujol); UV: 252 (11200; $H_2O$).

9f) 2 g 3-Acetoxymethyl-7β-[(2R,S)-2-((2R)-2-amino-2-carboxyäthoxycarbonylamino)-2-(2-aminothiazol-4-yl)-acetamido]-3-cephem-4-carbonsäurenatriumsalz und 1,28 g 1-Dimethylaminoäthyl-5-mercapto-1H-tetrazol werden in 6 ml Wasser wie im Beispiel 1e) beschrieben umgesetzt, aufgearbeitet und chromatographiert. Man erhält die 3-[1-(2-Dimethylaminoäthyl)-1H-tetrazol-5-yl-thiomethyl]-7β-[(2R,S)-2-((2R)-2-amino-2-carboxyäthoxycarbonylamino)-2-(2-aminothiazol-4-yl)-acetamido]-3-cephem-4-carbonsäure; Zersetzung ab 160°; $[\alpha]_D^{20°}$ = +29° ± 1° (0,82% in $H_2O$); IR: 3600-2500 breit, 1770, 1712 (Schulter), 1680 (Schulter), 1615, 1508 $cm^{-1}$ (Nujol); UV: 254 (13500; $H_2O$).

9g) 2 g 3-Acetoxymethyl-7β-[(2R,S)-2-((2R)-2-amino-2-carboxyäthoxycarbonylamino)-2-(2-aminothiazol-4-yl)-acetamido]-3-cephem-4-carbonsäure-natriumsalz und 1,19 g 1-Carboxymethyl-5-mercapto-1H-tetrazol werden in 6 ml Wasser, wie im Beispiel 1e) beschrieben umgesetzt, aufgearbeitet und chromatographiert. Man erhält das Hydrat des 3-(1-Carboxymethyl-1H-tetrazol-5-yl-thiomethyl)-7β-[(2R,S)-2-((2R)-2-amino-2-carboxyäthoxycarbonylamino)-2-(2-aminothiazol-4-yl)-acetamido]-3-cephem-4-carbonsäure-dinatriumsalzes; Zersetzung ab 175°; $[\alpha]_D^{20°} = +11° \pm 1°$ (0,98% in $H_2O$); IR: 3600-2600 (breit), 1765, 1740-1570 (breit), 1520 $cm^{-1}$ (Nujol); UV: 258 (13500; $H_2O$).

9h) 2 g 3-Acetoxymethyl-7β-[(2R,S)-2-((2R)-2-amino-2-carboxyäthoxycarbonylamino)-2-(2-aminothiazol-4-yl)-acetamido]-3-cephem-4-carbonsäurenatriumsalz und 1,9 g 1-Sulfomethyl-5-mercapto-1H-tetrazol werden in 6 ml Wasser wie im Beispiel 1e) beschrieben umgesetzt, aufgearbeitet und chromatographiert. Man erhält das 3-(1-Sulfomethyl-1H-tetrazol-5-yl-thiomethyl)-7β-[(2R,S)-2-((2R)-2-amino-2-carboxyäthoxycarbonylamino)-2-(2-aminothiazol-4-yl)-acetamido]-3-cephem-4-carbonsäuredinatriumsalz als Hydrat; Zersetzung ab 165°; $[\alpha]_D^{20°} = +12° \pm 1°$ (0,90% in $H_2O$); IR: 3600-250 (breit), 1765, 1730 (Schulter), 1680 (Schulter), 1625, 1520 $cm^{-1}$ (Nujol); UV: 258 (14200; $H_2O$).

<u>Beispiel 10:</u>

10a) 14,52 g (2R,S)-2-((3R)-3-BOC-Amino-3-t.butoxycarbonylpropionamido)-2-(2-trichloräthoxycarbonylaminothiazol-4-yl)-essigsäure und 9,1 g 3-Acetoxymethyl-7β-amino-3-cephem-4-carbonsäurediphenylmethylester werden in 180 ml Tetrahydrofuran mit 1,8 g Hydroxybenztriazol und 5,1 g Dicyclohexylcarbodiimid analog zum Beispiel 8a) umgesetzt und aufgearbeitet. Nach Chromatographie an 900 g Kieselgel erhält man aus den Toluol-Essigester-(7:3)-Eluaten den 3-Acetoxymethyl-7β-[(2R,S)-2-((3R)-3-BOC-amino-3-t-butoxycarbonylpropionamido)-2-(2-trichloräthoxycarbonylaminotriazil-4-yl)-acetamido]-3-cephem-4-carbonsäurediphenylmethylester. $[\alpha]_D^{20°} = +15° \pm 1°$ (0,90% in $CHCl_3$); IR: 3395,

3300 (Schulter), 1780, 1750-1650 (breit), 1550 (Schulter), 1490 $cm^{-1}$ ($CH_2Cl_2$); UV: 258 (1600; $C_2H_5OH$).

10b) 6,75 g 3-Acetoxymethyl-7β-[(2R,S)-2-((3R)-3-BOC-amino-3-t. butoxycarbonylpropionamido)-2-(2-trichloräthoxycarbonylaminothiazol-4-yl)-acetamido]-3-cephem-4-carbonsäurediphenylmethylester werden mit 7,6 g Zinkstaub in 67 ml Essigsäure-Acetonitril-(1:1)-Gemisch analog Beispiel 8b) umgesetzt, aufgearbeitet und chromatographiert. Dabei erhält man den 3-Acetoxymethyl-7β-[(2R,S)-2-((3R)-3-BOC-amino-3-t. butoxycarbonylpropionamido)-2-(2-aminothiazol-4-yl)-acetamido]-3-cephem-4-carbonsäurediphenylmethylester; $[\alpha]_D^{20°}$ = -7° ± 1° (0,98% in $CHCl_3$); IR: 3480 (Schulter), 3390, 3310 (Schulter), 1780, 1740-1650 (breit), 1600, 1487 $cm^{-1}$ ($CH_2Cl_2$); UV: 258 (12600; $C_2H_5OH$).

10c) 2,5 g 3-Acetoxymethyl-7β-[(2R,S)-2-((3R)-3-BOC-amino-3-t. butoxycarbonylpropionamido)-2-(2-aminothiazol-4-yl)-acetamido-3-cephem-4-carbonsäurediphenylmethylester werden in 4,3 ml $CH_2Cl_2$ und 1,4 ml Anisol mit 30 ml Trifluoressigsäure analog Beispiel 1c) umgesetzt und aufgearbeitet, wobei jedoch die Reaktionsdauer auf 2 Std. verlängert wird. Man erhält das Hydrat des 3-Acetoxymethyl-7β-[(2R,S)-2-((3R)-3-amino-3-carboxypropionamido)-2-(2-aminothiazol-4-yl)-acetamido]-3-cephem-4-carbonsäurenatriumsalzes (Zers. ab 180°), das ohne weitere Charakterisierung weiterverarbeitet wird (Beispiel 10d).

10d) 1,45 g 3-Acetoxymethyl-7β-[(2R,S)-2-((3R)-3-amino-3-carboxypropionamido)-2-(2-aminothiazol-4-yl)-acetamido]-3-cephem-4-carbonsäurenatriumsalz und 0,76 g 1-Methyl-5-mercapto-1H-tetrazol-natriumsalz werden in 5 ml Wasser wie im Beispiel 1e) beschrieben umgesetzt, aufgearbeitet und chromatographiert. Man erhält das 3-(1-Methyl-1H-tetrazol-5-yl-thiomethyl)-7β-[(2R,S)-2-((3R)-3-amino-3-carboxypropionamido)-2-(2-aminothiazol-4-yl)-acetamido]-3-cephem-4-carbonsäurenatriumsalz als Hydrat. Zersetzung ab 180°C; $[\alpha]_D^{20°}$ = -9° ± 1° (0,70% in $H_2O$); IR: 3600-2400 (breit) 1765, 1710-1575 (breit), 1500 $cm^{-1}$ (Nujol); UV: 258 (12040; $H_2O$).

Das Ausgangsmaterial für die Beispiele 10a)-10d) kann wie folgt hergestellt werden:

10e) 7,04 g (3R)-3-BOC-Amino-5-t.butoxycarbonylpropionsäure werden in 150 ml Tetrahydrofuran gelöst, auf -20° abgekühlt und nacheinander mit 3,13 ml N-Methylmorpholin und 3,0 ml Chlorameisensäure-isobutylester versetzt. Dann rührt man 4 Std. bei -20°. Gleichzeitig schlämmt man 8,6 g (2R,S)-2-(2-Trichloräthoxycarbonylaminothiazol-4-yl)-glycin in 150 ml Tetrahydrofuran auf, versetzt sofort und nochmals nach 30 Minuten mit je 7 ml N,O-Bis-(Trimethylsilyl)-acetamid und rührt total 1 1/2 Std. bei Zimmertemperatur. Dabei entsteht eine klare Lösung die nach Ablauf der 4 Stunden Reaktionsdauer bei -20° zu der zuerst beschriebenen Reaktionslösung zugetropft wird. Anschliessend rührt man während weiteren 3 Std. bei 0°. Dann giesst man auf Eiswasser, stellt mit 2N Salzsäure auf pH 2, extrahiert mit Essigester und wäscht mit gesättigter wässriger NaCl-Lösung neutral. Die nach dem Trocknen über $Na_2SO_4$ und dem Eindampfen erhalten rohe (2R,S)-2-((3R)-3-BOC-Amino-3-t.butoxycarbonylpropionamido)-2-(2-trichloräthoxycarbonylaminothiazol-4-yl)-essigsäure wird direkt weiterverarbeitet (Beispiel 10a)).

<u>Beispiel 11:</u>

11a) 3,4 g (2R)-2-((4R)-4-BOC-Amino-4-t.butoxycarbonylbutyramido)-2-methylessigsäure und 3 g 3-Acetoxymethyl-7β-amino-3-cephem-4-carbonsäurediphenylmethylester werden analog Beispiel 4a) mit 0,92 ml N-Methylmorpholin und 0,88 ml Chlorameisensäureisobutylester in 65 ml Tetrahydrofuran umgesetzt und aufgearbeitet. Das erhaltene Rohprodukt chromatographiert man an 200 g Kieselgel (Eluiermittel:Methylenchlorid-Essigester (1:1), Fraktionengrösse 100 ml). Durch Vereinigen der produkthaltigen Fraktionen und Umfällen aus Methylenchlorid-Aether-Hexan erhält man den 3-Acetoxymethyl-7β-[(2R)-2-((4R)-4-BOC-amino-4-t.butoxycarbonylbutyramido)-2-methylacetamido]-3-cephem-4-carbonsäurediphenylmethylester; $[\alpha]_D^{20°} = +5° \pm 1°$ (0,87% in $CHCl_3$); IR: 3340,

1785, 1730, 1697, 1675, 1645, 1530 $cm^{-1}$ (Nujol); UV: 260 (7600; $C_2H_5OH$).

11b) 13,0 g 3-Acetoxymethyl-7β-[(2R)-2-((4R)-4-BOC-amino-4-t.butoxycarbonylbutyramido)-2-methylacetamido]-3-cephem-4-carbonsäurediphenylmethylester werden in 1300 ml Tetrahydrofuran wie im Beispiel 1a) beschrieben methoxyliert (0,45 g Lithium in 50 ml Methanol; 3,5 ml t.Butylhypochlorit, aufgeteilt in zwei Portionen zu 1,45 und eine Portion zu 0,6 ml), aufgearbeitet und chromatographiert (Eluiermittel: Aether-Essigester (9:1); Fraktionengrösse 150 ml). Man erhält den 3-Acetoxymethyl-7α-methoxy-7β-[(2R)-2-((4R)-4-BOC-amino-4-t.butoxycarbonylbutyramido)-2-methylacetamido]-3-cephem-4-carbonsäurediphenylmethylester; $[\alpha]_D^{20°}$ = +75° ± 1° (1,03% in $CHCl_3$); IR: 3340, 1785, 1730 (breit), 1655, 1520 $cm^{-1}$ (Nujol); UV: 247 (5600), 264 (5800), 269 (5800; $C_2H_5OH$).

11c) 2 g 3-Acetoxymethyl-7β-[(2R)-2-((4R)-4-BOC-amino-4-t.butoxycarbonylbutyramido)-2-methylacetamido]-3-cephem-4-carbonsäurediphenylmethylester werden in 3 ml $CH_2Cl_2$ und 0,43 ml Anisol mit 8 ml Trifluoressigsäure analog Beispiel 10c) umgesetzt und aufgearbeitet. Man erhält das Hydrat des 3-Acetoxymethyl-7β-[(2R)-2-((4R)-4-amino-4-carboxybutyramido)-2-methylacetamido]-3-cephem-4-carbonsäure-natriumsalzes; Zersetzung ab 190°; $[\alpha]_D^{20°}$ = +81° ± 1° (0,86% in $H_2O$); IR: 3600-2400 (breit), 1772, 1720-1600 (breit), 1550 (Schulter) $cm^{-1}$ (Nujol); UV: 263 (6020; $C_2H_5OH$).

11d) 6,7 g 3-Acetoxymethyl-7α-methoxy-7β-[(2R)-2-((4R)-4-BOC-amino-4-t.butoxycarbonylbutyramido)-2-methylacetamido]-3-cephem-4-carbonsäurediphenylmethylester werden in 10 ml $CH_2Cl_2$ und 2 ml Anisol mit 25 ml Trifluoressigsäure analog Beispiel 10c) umgesetzt und aufgearbeitet. Man erhält das Hydrat des 3-Acetoxymethyl-7α-methoxy-7β-[(2R)-2-((4R)-4-amino-4-carboxybutyramido)-2-methylacetamido]-3-cephem-4-carbonsäurenatriumsalzes; Zersetzung ab 170°;

$[\alpha]_D^{20°}$ = +145° ± 1° (0,87% in $H_2O$); IR: 3600-2400, 1769, 1720-1630 (breit), 1540 (Schulter) $cm^{-1}$ (Nujol); UV: 238 (5480), 264 (6460; $H_2O$).

11e) 1,7 g 3-Acetoxymethyl-7β-[(2R)-2-((4R)-4-amino-4-carboxy-butyramido)-2-methylacetamido]-3-cephem-4-carbonsäurenatriumsalz und 0,8 g 1-Methyl-5-mercapto-1H-tetrazol-natriumsalz werden in 10 ml Wasser wie im Beispiel 1e) beschrieben umgesetzt, aufgearbeitet und chromatographiert. Man erhält das 3-(1-Methyl-1H-tetrazol-5-ylthio-methyl)-7β-[(2R)-2-((4R)-4-amino-4-carboxybutyramido)-2-methylacet-amido]-3-cephem-4-carbonsäurenatriumsalz als Hydrat; Zersetzung ab 195°; $[\alpha]_D^{20°}$ = 11° ± 1° (0,95% in $H_2O$); IR: 3600-2400 (breit), 1768, 1750-1580 (breit), 1550 (Schulter) $cm^{-1}$ (Nujol); UV: 265 (6800; $H_2O$).

11f) 3,2 g 3-Acetoxymethyl-7α-methoxy-7β-[(2R)-2-((4R)-4-amino-4-carboxybutyramido)-2-methylacetamido]-3-cephem-4-carbonsäurenatriumsalz und 1,6 g 1-Methyl-5-mercapto-1H-tetrazol-natriumsalz werden in 20 ml Wasser wie im Beispiel 1e) beschrieben umgesetzt, aufgearbeitet und chromatographiert. Man erhält das 3-(1-Methyl-1H-tetrazol-5-yl-thiomethyl)-7α-methoxy-7β-[(2R)-2-((4R)-4-amino-4-carboxybutyramido)-2-methylacetamido]-3-cephem-4-carbonsäure-natriumsalz als Hydrat; Zersetzung ab 195°; $[\alpha]_D^{20°}$ = +89° ± 1° (0,82% in $H_2O$); IR: 3600-2400 (breit), 1769, 1750-1675 (breit), 1530 $cm^{-1}$ (Nujol); UV: 272 (3540; $H_2O$).

Das Ausgangsmaterial für Beispiele 11a)-11f) kann wie folgt hergestellt werden:

11g) 3 g (4R)-4-BOC-Amino-4-t.butoxycarbonylbuttersäure werden in 60 ml Tetrahydrofuran gelöst, auf -20° abgekühlt und nacheinander mit 1,27 ml N-Methylmorpholin und 1,22 ml Chlorameisensäure-isobutylester versetzt. Dann rührt man 4 Std. bei -20°. Gleichzeitig schlämmt man 0,81 g D-Alanin in 30 ml Acetonitril auf, versetzt mit 8 ml N,O-

Bis-(Trimethylsilyl)-acetamid und erhitzt 3 Std. am Rückfluss. Dabei entsteht eine klare Lösung, die am Rotverdampfer zur Trockene eingedampft wird. Den Eindampfrückstand löst man in 30 ml Tetrahydrofuran und tropft anschliessend diese Lösung bei -20° zu der zuerst beschriebenen Reaktionslösung. Darauf rührt man weitere 3 Std. bei 0°. Dann giesst man auf Eiswasser, stellt mit 2N Salzsäure auf pH 2, extrahiert mit Essigester und wäscht mit gesättigter wässriger NaCl-Lösung neutral. Die nach dem Trocknen über $Na_2SO_4$ und dem Eindampfen erhaltene rohe (2R)-2-((4R)-4-BOC-Amino-4-t.butoxycarbonylbutyramido-2-methylessigsäure wird direkt weiterverarbeitet (Beispiel 11a)).

Beispiel 12:

12a) 15 g 3-Acetoxymethyl-7β-[(2R)-2-amino-2-hydroxymethylacetyl-amino]-3-cephem-4-carbonsäurediphenylmethylester-p-toluolsulfonsäuresalz werden in 200 ml Tetrahydrofuran zusammen mit 4,1 ml Pyridin und 18,6 g (2R)-2-BOC-Amino-2-diphenylmethoxycarbonyläthoxycarbonylchlorid wie im Beispiel 1a) beschrieben umgesetzt, aufgearbeitet und chromatographiert. Man erhält den 3-Acetoxymethyl-7β-[(2R)-2-((2R)-2-BOC-amino-2-diphenylmethoxycarbonyläthoxycarbonylamino)-2-hydroxymethyl-acetamido[-3-cephem-4-carbonsäurediphenylmethylester; $[\alpha]_D^{20°}$ = +17° ± 1° (0,84% in $CHCl_3$); IR: 3370, 1780, 1740-1680 (breit), 1495 $cm^{-1}$ ($CH_2Cl_2$); UV: 258 (7800), 264 (7800; $C_2H_5OH$).

12b) 11,3 g 3-Acetoxymethyl-7β-[(2R)-2-((2R)-2-BOC-amino-2-diphenylmethoxycarbonyläthoxycarbonylamino)-2-hydroxymethylacetamido]-3-cephem-4-carbonsäure-diphenylmethylester werden in 130 ml Tetrahydrofuran und 1,424 ml Pyridin mit 2,44 ml 2,2,2-Trichloräthylchlorformiat analog Beispiel 1a) umgesetzt und aufgearbeitet. Nach Umfällen des Rohproduktes aus Methylenchlorid-Aether-Hexan erhält man den 3-Acetoxymethyl-7β-[(2R)-2-((2R)-2-BOC-amino-2-diphenylmethoxycarbonyläthoxycarbonylamino)-2-trichloräthoxycarbonyloxymethylacetamido]-3-cephem-4-carbonsäure-diphenylmethylester; $[\alpha]_D^{20°}$ = +5° ± 1° (0,79% in $CHCl_3$); IR: 3370, 1780, 1760, 1740-1700 (breit), 1690 (Schulter), 1595 $cm^{-1}$ ($CH_2Cl_2$); UV: 258 (8000), 264 (8000; $C_2H_5OH$).

12c) 12,95 g 3-Acetoxymethyl-7β-[(2R)-2-((2R)-2-BOC-amino-2-diphenylmethoxycarbonyläthoxycarbonylamino)-2-trichloräthoxycarbonyloxy)-methylacetamido]-3-cephem-4-carbonsäurediphenylmethylester werden in 1295 ml Tetrahydrofuran wie im Beispiel 1b) beschrieben methoxyliert (0,35 g Lithium in 62,3 ml Methanol; 1,985 ml t-Butylhypochlorit, aufgeteilt in 2 Portionen zu 0,775 ml und 1 Portion zu 0,435 ml), aufgearbeitet und chromatographiert. Dabei eluiert man mit Toluol-Essigester-(9:1)-Gemisch den 3-Acetoxymethyl-7α-methoxy-7β-[(2R)-2-((2R)-2-BOC-amino-2-diphenylmethoxycarbonyläthoxycarbonylamino)-2-trichloräthoxycarbonyloxymethyl-acetamido]-3-cephem-4-carbonsäurediphenylmethylester; $[\alpha]_D^{20°}$ = +46° ± 1° (0,90% in $CHCl_3$); IR: 3380, 1780 (Schulter), 1770-1695 (breit), 1490 $cm^{-1}$ ($CH_2Cl_2$); UV: 247 (6600), 251 (6700), 257 (6900), 263 (7000), 267 (6800; $C_2H_5OH$).

Aus den Toluol-Essigester-(85:15)-Fraktionen isoliert man den 3-Acetoxymethyl-7α-methoxy-7β-[(2R)-2-((2R)-2-BOC-amino-2-diphenylmethoxycarbonyläthoxycarbonylamino)-2-methoxymethylacetamido]-3-cephem-4-carbonsäurediphenylmethylester; $[\alpha]_D^{20°}$ = +41° ± 1° (1,07% in $CHCl_3$); IR: 3375, 1780, 1760-1690 (breit), 1590 $cm^{-1}$ ($CH_2Cl_2$); UV: 247 (5400), 251 (5500), 257 (5700), 263 (5700), 267 (5600; $C_2H_5OH$).

12d) 5.2 g 3-Acetoxymethyl-7α-methoxy-7β-[(2R)-2-((2R)-2-BOC-amino-2-diphenylmethoxycarbonyläthoxycarbonylamino)-2-methoxymethylacetamido]-3-cephem-4-carbonsäurediphenylmethylester werden in 10 ml $CH_2Cl_2$ und 3,78 ml Anisol mit 49,66 ml Trifluoressigsäure analog Beispiel 1c) umgesetzt und aufgearbeitet. Man erhält das 3-Acetoxymethyl-7α-methoxy-7β-[(2R)-2-((2R)-2-amino-2-carboxyäthoxycarbonylamino)-2-methoxymethylacetamido]-3-cephem-4-carbonsäure-natriumsalz als Hydrat; Zersetzung ab 155°; $[\alpha]_D^{20°}$ = +120° ± 1° (0,80% in $H_2O$); IR: 3600-2500 (breit), 1760, 1730, 1683, 1632, 1607, 1512, 1460 $cm^{-1}$ (Nujol); UV: 240 (6000), 264 (7200; $H_2O$).

12e) 1,75 g 3-Acetoxymethyl-7α-methoxy-7β-[(2R)-2-((2R)-2-amino-2-carboxyäthoxycarbonylamino)-2-methoxymethylacetamido]-3-cephem-4-carbonsäure-natriumsalz werden mit 0,93 g 1-Methyl-5-mercapto-1H-tetrazol-natriumsalz wie im Beispiel 1e) beschrieben umgesetzt, aufgearbeitet und chromatographiert. Man erhält das 3-(1-Methyl-1H-tetrazol-5-ylthiomethyl)-7α-methoxy-7β-[(2R)-2-((2R)-2-amino-2-carboxyäthoxycarbonylamino)-2-methoxymethylacetamido]-3-cephem-4-carbonsäure-natriumsalz als Hydrat; Zersetzung ab 135°; $[\alpha]_D^{20°} = +62° \pm 1°$ (0,75% in $H_2O$); IR: 3600-2500, 1760, 1710, 1673, 1520, 1462 $cm^{-1}$ (Nujol); UV: 240 (7100), 268 (8200; $H_2O$).

12f) 4,8 g 3-Acetoxymethyl-7α-methoxy-7β-[(2R)-2-((2R)-2-BOC-amino-2-diphenylmethoxycarbonyläthoxycarbonylamino)-2-trichloräthoxycarbonyloxymethylacetamido]-3-cephem-4-carbonsäurediphenylmethylester werden mit 7,2 g Zinkstaub in 55 ml Acetonitril-Essigsäure-(1:1) wie im Beispiel 8b) reduziert, aufgearbeitet und chromatographiert. Dabei erhält man den 3-Acetoxymethyl-7α-methoxy-7β-[(2R)-2-((2R)-2-BOC-amino-2-diphenylmethoxycarbonyläthoxycarbonylamino)-2-hydroxymethylacetamido]-3-cephem-4-carbonsäure-diphenylmethylester; $[\alpha]_D^{20°} = +46° \pm 1°$ (0,78% in $CHCl_3$); IR: 3380, 1780, 1740-1700 (breit), 1690 (Schulter), 1590 $cm^{-1}$ ($CH_2Cl_2$); UV: 251 (5200), 257 (5300), 263 (5400), 267 (5200; $C_2H_5OH$).

12g) 4,1 g 3-Acetoxymethyl-7α-methoxy-7β-[(2R)-2-((2R)-2-BOC-amino-2-diphenylmethoxycarbonyläthoxycarbonylamino)-2-hydroxymethylacetamido]-3-cephem-4-carbonsäurediphenylmethylester werden in 7,5 ml $CH_2Cl_2$ und 2,98 ml Anisol mit 39,15 ml Trifluoressigsäure analog Beispiel 1c) umgesetzt und aufgearbeitet. Man erhält das 3-Acetoxymethyl-7α-methoxy-7β-[(2R)-2-((2R)-2-amino-2-carboxyäthoxycarbonylamino)-2-hydroxymethylacetamido]-3-cephem-4-carbonsäure-natriumsalz als Hydrat; Zersetzung ab 155°; $[\alpha]_D^{20°} = +143° \pm 1°$ (0,76% in $H_2O$); IR: 3600-2500 (breit), 1770, 1730, 1695, 1611, 1532, 1467 $cm^{-1}$ (Nujol); UV: 240 (6400), 266 (7700; $H_2O$).

12h) 1,5 g 3-Acetoxymethyl-7α-methoxy-7β-[(2R)-2-((2R)-2-amino-2-carboxyäthoxycarbonylamino)-2-hydroxymethylacetamido]-3-cephem-4-carbonsäure-natriumsalz werden mit 0,82 g 1-Methyl-5-mercapto-1H-tetrazol-natriumsalz wie im Beispiel 1e) beschrieben umgesetzt, aufgearbeitet und chromatographiert. Man erhält das 3-(1-Methyl-1H-tetrazol-5-ylthiomethyl)-7α-methoxy-7β-[(2R)-2-((2R)-2-amino-2-carboxyäthoxycarbonylamino)-2-hydroxymethylacetamido]-3-cephem-4-carbonsäure-natriumsalz als Hydrat; Zersetzung ab 150°; $[\alpha]_D^{20°} = +75° \pm 1°$ (0,93% in $H_2O$); IR: 3600-2500 (breit), 1770, 1725, 1692, 1620, 1533, 1466 $cm^{-1}$ (Nujol); UV: 238 (8000), 270 (9200; $H_2O$).

Das Ausgangsmaterial für die Beispiele 12a)-12h) kann wie folgt hergestellt werden:

12i) 10,4 g N-BOC-(D)-Serin und 20 g 3-Acetoxymethyl-7β-amino-3-cephem-4-carbonsäurediphenylmethylester werden in 240 ml Dioxan und 160 ml Tetrahydrofuran mit 3,92 g Hydroxybenztriazol und 3 x 3,4 g Dicyclohexylcarbodiimid analog Beispiel 8a) umgesetzt, aufgearbeitet und chromatographiert. Man erhält den 3-Acetoxymethyl-7β-[(2R)-2-BOC-amino-2-hydroxymethylacetamido]-3-cephem-4-carbonsäure-diphenylmethylester; $[\alpha]_D^{20°} = +13° \pm 1°$ (1,03% in $CHCl_3$); IR: 3500, 3370, 1780, 1720, 1690, 1488 $cm^{-1}$ ($CH_2Cl_2$); UV: 260 (7200; $C_2H_5OH$).

12j) 18 g 3-Acetoxymethyl-7β-[(2R)-2-BOC-amino-2-hydroxymethylacetamido]-3-cephem-4-carbonsäurediphenylmethylester werden mit 10,9 g p-Toluolsulfonsäuremonohydrat in 100 ml Acetonitril wie im Beispiel 1m) umgesetzt und aufgearbeitet. Man erhält das 3-Acetoxymethyl-7β-[(2R)-2-amino-2-hydroxymethylacetamido]-3-cephem-4-carbonsäure-diphenylmethylester-p-toluolsulfonsäuresalz; $[\alpha]_D^{20°} = +22° \pm 1°$ (0,74% in $CH_3OH$); IR: 3600-2500 (breit), 1785, 1732, 1693, 1550, 1490, 1461 $cm^{-1}$ (Nujol); UV: 260 (7200; $C_2H_5OH$).

Beispiel 13:

13a) 3,412 g der gemäss Beispiel 4d) erhältlichen (2R)-2-((2R)-2-BOC-Amino-2-diphenylmethoxycarbonyläthoxycarbonylamino)-2-phenylessigsäure und 2,45 g 3-(1-Methyl-1H-tetrazol-5-ylthiomethyl)-7β-amino-3-cephem-4-carbonsäurediphenylmethylester werden analog zu Beispiel 4a) umgesetzt (0,72 ml N-Methylmorpholin + 0,68 ml Chlorameisensäure-isobutylester in 90 ml Tetrahydrofuran), aufgearbeitet und chromatographiert (200 g Kieselgel; Eluiermittel: Toluol-Essigester (4:1), Fraktionengrösse 25 ml). Durch Vereinigen der produkthaltigen Fraktionen und Umfällen aus Methylenchlorid-Hexan erhält man den 3-(1-Methyl-1H-tetrazol-5-ylthiomethyl)-7β-[(2R)-2-((2R)-2-BOC-amino-3-diphenylmethoxycarbonyläthoxycarbonylamino)-2-phenylacetamido]-3-cephem-4-carbonsäurediphenylmethylester; $[\alpha]_D^{20°} = -94° \pm 1°$ (1,11% in $CHCl_3$); IR: 3360, 1790, 1728, 1705 (Schulter), 1500 $cm^{-1}$ (Nujol); UV: 262 (9000), 267 (8900; $C_2H_5OH$).

13b) 2 g 3-(1-Methyl-1H-tetrazol-5-ylthiomethyl)-7β-[(2R)-2-((2R)-2-BOC-amino-2-diphenylmethoxycarbonyläthoxycarbonylamino)-2-phenylacetamido]-3-cephem-4-carbonsäurediphenylmethylester werden in 4 ml Trifluoressigsäure analog zu Beispiel 1c) umgesetzt und aufgearbeitet. Man erhält das 3-(1-Methyl-1H-tetrazol-5-ylthiomethyl)-7β-[(2R)-2-((2R)-2-amino-2-carboxyäthoxycarbonylamino)-2-phenylacetamido)-3-cephem-4-carbonsäurenatriumsalz als Hydrat; Zersetzung ab 180°; $[\alpha]_D^{20} = -26° \pm 1°$ (0,91% in $H_2O$); IR: 3600-2500, 1770, 1718 (Schulter), 1682 (Schulter), 1611, 1532, 1465 $cm^{-1}$ (Nujol); UV: 265 (9600; $H_2O$).

Beispiel 14:

14a) 4 g (2S)-2-((2R)-2-BOC-Amino-2-diphenylmethoxycarbonyläthoxycarbonylamino-2-phenylessigsäure und 3,12 g 3-(1-Methyl-1H-tetrazol-5-ylthiomethyl)-7β-amino-2-cephem-4-carbonsäurediphenylmethylester werden analog Beispiel 4a) umgesetzt (0,92 ml N-Methylmorpholin + 0,87 ml Chlorameisensäureisobutylester in 150 ml Tetrahydrofuran), aufgearbeitet, chromatographiert und umgefällt. Man erhält den 3-(1-Methyl-1H-tetrazol-5-ylthiomethyl)-7β-[(2S)-2-((2R)-2-BOC-amino-2-diphenylmethoxycarbonyläthoxycarbonylamino)-2-phenylacetamido]-3-cephem-4-carbonsäurediphenylmethylester; $[\alpha]_D^{20°}$ = -65° ± 1° (1,05% in $CHCl_3$); IR: 3355, 1790, 1727, 1692 (Schulter), 1500, 1458 $cm^{-1}$ (Nujol); UV: 264 (9000), 268 (8900; $C_2H_5OH$).

14b) 2 g 3-(1-Methyl-1H-tetrazol-5-ylthiomethyl)-7β-[(2S)-2-((2R)-2-BOC-amino-2-diphenylmethoxycarbonyläthoxycarbonylamino)-2-phenylacetamido]-3-cephem-4-carbonsäurediphenylmethylester werden analog Beispiel 1c) umgesetzt und aufgearbeitet. Man erhält das 3-(1-Methyl-1H-tetrazol-5-ylthiomethyl)-7β-[(2S)-2-((2R)-2-amino-2-carboxyäthoxycarbonylamino)-2-phenylacetamido)-3-cephem-4-carbonsäure-natriumsalz als Hydrat; Zersetzung ab 178°; $[\alpha]_D^{20°}$ = +44° ± 1° (1,03% in $H_2O$); IR: 3600-2500, 1770, 1721 (Schulter), 1682 (Schulter), 1612, 1531, 1463 $cm^{-1}$ (Nujol); UV: 265 (9500); $H_2O$).

Das Ausgangsmaterial für Beispiel 14a)-14b) kann wie folgt hergestellt werden:

14c) 1,25 g L-Phenylglycin und 3,94 g (2R)-2-BOC-Amino-2-diphenylmethoxycarbonyläthoxycarbonylchlorid werden wie im Beispiel 4d) beschrieben umgesetzt und aufgearbeitet. Man erhält die (2S)-2-((2R)-2-BOC-Amino-2-diphenylmethoxycarbonyläthoxycarbonylamino)-2-phenylessigsäure, die ohne Charakterisierung weiterverarbeitet wird (Beispiel 14a).

Beispiel 15:

15a) 15 g (2R,S)-2-((2R)-2-BOC-Amino-2-diphenylmethoxycarbonyläthoxycarbonylamino)-2-(2-BOC-aminothiazol-4-yl)-essigsäure und 10 g 3-(1-Methyl-1H-tetrazol-5-ylthiomethyl)-7β-amino-3-cephem-4-carbonsäurediphenylmethylester werden in 150 ml Tetrahydrofuran analog zu

Beispiel 8a) umgesetzt (1,76 g Hydroxybenztriazol + 3 x 1,63 g Dicyclohexylcarbodiimid in je 50 ml Tetrahydrofuran) und aufgearbeitet. Das anfallende Rohprodukt wird in 5 Portionen an je 450 g Kieselgel auf Stufensäulen chromatographiert (Eluiermittel:Toluol-Essigester-85:5-Gemisch; Fraktionen à 25 ml). Dabei eluiert man zuerst den 3-(1-Methyl-1H-tetrazol-5-ylthiomethyl)-7β-[(2R)-2-((2R)-2-BOC-amino-2-diphenylmethoxycarbonyläthoxycarbonylamino)-2-(2-BOC-aminothiazol-4-yl)-acetamido]-3-cephem-4-carbonsäurediphenylmethylester, der aus $CH_2Cl_2$-Aether-Hexan umgefällt wird. Die Konfigurationszuordnung am α-Kohlenstoffatom des Acetylrestes in der 7β-Acylseitenkette erfolgt anhand der im Beispiel 9a) genannten Methoden. $[\alpha]_D^{20°} = -68° \pm 1°$ (1,03% in $CHCl_3$); IR: 3400, 1784, 1730-1690 (breit), 1520 (Schulter), 1490 $cm^{-1}$ ($CH_2Cl_2$); UV: 260 (17000; $C_2H_5OH$).

Die anschliessend eluierten Fraktionen bestehen aus einem binären Gemisch der obigen Verbindung mit dem entsprechenden (2S)-Isomeren, das durch nochmalige Chromatographie weiter aufgetrennt werden kann. Zuletzt wird reiner 3-(1-Methyl-1H-tetrazol-5-ylthiomethyl)-7β-[(2S)-2-((2R)-2-BOC-amino-2-diphenylmethoxycarbonyläthoxycarbonylamino)-2-(2-BOC-aminothiazol-4-yl)-acetamido]-3-cephem-4-carbonsäurediphenylmethylester eluiert, der sich aus $CH_2Cl_2$-Aether-Hexan umfällen lässt. Die Konfigurationszuordnung am α-Kohlenstoffatom des Acetylrestes in der 7β-Acylseitenkette erfolgt anhand der im Beispiel 9a) genannten Methoden. $[\alpha]_D^{20°} = -36° \pm 1°$ (0,82% in $CHCl_3$); IR: 3400, 1786, 1730-1690 (breit), 1540 (Schulter), 1495 $cm^{-1}$ ($CH_2Cl_2$); UV: 260 (17400; $C_2H_5OH$).

15b) 2,8 g 3-(1-Methyl-1H-tetrazol-5-ylthiomethyl)-7β-[(2R)-2-((2R)-2-BOC-amino-2-diphenylmethoxycarbonyläthoxycarbonylamino)-2-(2-BOC-aminothiazol-4-yl)-acetamido]-3-cephem-4-carbonsäurediphenylmethylester werden in 4,8 ml $CH_2Cl_2$ und 2,4 ml Anisol mit 19,6 ml Trifluoressigsäure analog Beispiel 1c) umgesetzt (Reaktionsdauer: 1 Std.) und aufgearbeitet. Man erhält das Hydrat des 3-(1-Methyl-1H-tetrazol-5-ylthiomethyl)-7β-[(2R)-2-((2R)-2-amino-2-carboxyäthoxycarbonyl-

amino)-2-(2-aminothiazol-4-yl)-acetamido-3-cephem-4-carbonsäure-natriumsalzes; Zersetzung ab 168°; $[\alpha]_D^{20°}$ = -1° ± 1° (0,93% in $H_2O$); IR: 3600-2500 (breit), 1770, 1715 (Schulter), 1680 (Schulter), 1622, 1523, 1465 $cm^{-1}$ (Nujol); UV: 258 (13000; $H_2O$).

15c): 1,83 g 3-(1-Methyl-1H-tetrazol-5-ylthiomethyl)-7β-[(2S)-2-((2R)-2-BOC-amino-2-diphenylmethoxycarbonyläthoxycarbonylamino)-2-(2-BOC-aminothiazol-4-yl)-acetamido]-3-cephem-4-carbonsäurediphenylmethylester werden in 3,2 ml $CH_2Cl_2$ und 1,6 ml Anisol mit 12,8 ml Trifluoressigsäure wie im Beispiel 1c) umgesetzt und aufgearbeitet. Man erhält das Hydrat des 3-(1-Methyl-1H-tetrazol-5-ylthiomethyl)-7β-[(2S)-2-((2R)-2-amino-2-carboxyäthoxycarbonylamino)-2-(2-aminothiazol-4-yl)-acetamido-3-cephem-4-carbonsäure-natriumsalzes; Zersetzung ab 170°; $[\alpha]_D^{20°}$ = +52° ± 1° (0,75% in $H_2O$); IR: 3600-2500 (breit), 1770, 1719 (Schulter), 1625, 1525, 1467 $cm^{-1}$ (Nujol); UV: 255 (12100; $H_2O$).

Beispiel 16:

3,4 g des gemäss Beispiel 8c) erhältlichen 3-Acetoxymethyl-7β-[(2R,S)-2-((2R)-2-amino-2-carboxyäthoxycarbonylamino)-2-(2-aminothiazol-4-yl)-acetamido]-3-cephem-4-carbonsäurenatriumsalzes, 1,046 g Isonicotinamid, 9,48 g Natriumjodid und 1,032 g Trichloressigsäure werden in 6,3 ml Wasser wie im Beispiel 1i) beschrieben umgesetzt, aufgearbeitet und chromatographiert. Man erhält die 3-(4-Carbamoylpyridinomethyl)-7β-[(2R,S)-2-((2R)-2-amino-2-carboxyäthoxycarbonylamino)-2-(2-aminothiazol-4-yl)-acetamido]-3-cephem-4-carbonsäure als Hydrat; Zersetzung ab 160°; $[\alpha]_D^{20°}$ = +10° ± 1° (0,84% in $H_2O$); IR: 3600-2500, 1772, 1620 (Schulter), 1687 (breit), 1520 $cm^{-1}$ (Nujol); UV: 258 (14300; $H_2O$).

Beispiel 17:

17a) 3 g (2R,S)-2-((4R)-4-BOC-Amino-4-t.butoxycarbonylburyramido)-2-(2-BOC-aminothiazol-4-yl)-essigsäure und 2,47 g 3-(1-Methyl-1H-tetrazol-5-ylthiomethyl)-7β-amino-3-cephem-4-carbonsäurediphenylmethylester werden in 40 ml Tetrahydrofuran analog Beispiel 8a) umgesetzt (0,43 g Hydroxybenztriazol + 3 x 0,38 g Dicyclohexylcarbodiimid in je 50 ml Tetrahydrofuran) und aufgearbeitet. Nach Chromatographie des Rohproduktes an 200 g Kieselgel (Eluiermittel:Aether-Essigester 4:1; Fraktionengrösse 100 ml) erhält man den 3-(1-Methyl-1H-tetrazol-5-ylthiomethyl)-7β-[(2R,S)-2-((4R)-4-BOC-amino-4-t.butoxycarbonylbutyramido)-2-(2-BOC-aminothiazol-4-yl)-acetamido]-3-cephem-4-carbonsäurediphenylmethylester, der aus $CH_2Cl_2$-Aether-Hexan umgefällt wird; $[\alpha]_D^{20°}$ = -88° ± 1° (0,91% in $CHCl_3$); IR: 3430, 3315, 1790, 1722, 1688, 1547, 1503 $cm^{-1}$ ($CH_2Cl_2$); UV: 260 (16200; $C_2H_5OH$).

17b) 3,8 g 3-(1-Methyl-1H-tetrazol-5-ylthiomethyl)-7β-[(2R,S)-2-((4R)-4-BOC-amino-4-t.butoxycarbonylbutyramido)-2-(2-BOC-aminothiazol-4-yl)-acetamido]-3-cephem-4-carbonsäurediphenylmethylester werden in 5 ml $CH_2Cl_2$ und 1,1ml Anisol mit 20 ml Trifluoressigsäure wie im Beispiel 1c) beschrieben umgesetzt und aufgearbeitet. Man erhält das Hydrat des 3-(1-Methyl-1H-tetrazol-5-ylthiomethyl)-7β-[(2R,S)-2-((4R)-4-amino-4-carboxybutyramido)-2-(2-aminothiazol-4-yl)-acetamido]-3-cephem-4-carbonsäurenatriumsalzes; Zersetzung ab 210°; $[\alpha]_D^{20°}$ = -10° ± 1° (1,15% in $H_2O$); IR: 3600-2500, 1770, 1718, 1530 $cm^{-1}$ (Nujol); UV: 258 (13400; $H_2O$).

Das Ausgangsmaterial für Beispiel 17a)-17b) kann wie folgt hergestellt werden:

17c) 3 g (4R)-4-BOC-Amino-4-t.butoxycarbonylbuttersäure werden in 200 ml Tetrahydrofuran gelöst, auf-20° abgekühlt und nacheinander mit 1,35 ml N-Methylmorpholin und 1,3 ml Chlorameisensäureisobutylester versetzt. Man rührt 3 Std. bei -20°, fügt 2,9 g (2R,S)-2-(2-BOC-aminothiazol-4-yl)-glycinmethylester zu, rührt weitere 3 Std.

bei 0° und arbeitet wie im Beispiel 1ℓ) beschrieben auf. Das Rohprodukt chromatographiert man an 200 g Kieselgel (Eluiermittel:Aether; Fraktionengrösse 100 ml). Vereinigung der produkthaltigen Fraktionen liefert den (2R,S)-2-((4R)-4-BOC-Amino-4-t.butoxycarbonylbutyramido)-2-(2-BOC-aminothiazol-4-yl)-essigsäuremethylester, der ohne Charakterisierung weiterverarbeitet wird (Beispiel 17d).

17d) 2,7 g (2R,S)-2-((4R)-4-BOC-Amino-4-t.butoxycarbonylbutyramido)-2-(2-BOC-aminothiazol-4-yl)-essigsäuremethylester werden in 50 ml Methanol mit 50 ml 1N wässriger NaOH versetzt und 30 Min. bei Zimmertemperatur gerührt. Dann extrahiert man mit Essigester und wäscht 2 x mit Wasser nach. Die Essigesterphase wird eliminiert. Die vereinigten Wasseranteile werden bei 0° mit 4N HCl auf pH 3 gestellt, mit Essigester extrahiert und 4 x mit gesättigter wässriger NaCl-Lösung gewaschen. Die nach dem Trocknen und EIndampfen der organischen Phase anfallende rohe (2R,S)-2-((4R)-4-BOC-Amino-4-t.butoxycarbonylbutyramido)-2-(2-BOC-aminothiazol-4-yl)-essigsäure wird ohne Charakterisierung weiterverarbeitet (Beispiel 17a)).

Beispiel 18:

18a) 13,7 g 3-Acetoxymethyl-7β-(4-aminobutyramido)-3-cephem-4-carbonsäure-diphenylmethylester-p-toluolsulfonsäuresalz werden in 350 ml Tetrahydrofuran zusammen mit 5 ml Pyridin und 18,8 g (2R)-2-BOC-Amino-2-diphenylmethoxycarbonyläthoxycarbonylchlorid analog Beispiel 1a) umgesetzt, aufgearbeitet und chromatographiert. Nach Vereinigung der produkthaltigen Fraktionen erhält man den 3-Acetoxymethyl-7β-[4-((2R)-2-BOC-amino-2-diphenylmethoxycarbonyläthoxycarbonylamino)-butyramido]-3-cephem-4-carbonsäurediphenylmethylester; IR: 3390, 1782, 1740-1690 (breit), 1511 (Schulter), 1496 $cm^{-1}$ ($CH_2Cl_2$); UV: 258 (7900), 264 (7750; EtOH).

18b) 9,2 g 3-Acetoxymethyl-7β-[4-((2R)-2-BOC-amino-2-diphenylmethoxycarbonyläthoxycarbonylamino)-butyramido]-3-cephem-4-carbonsäure-diphenylmethylester werden in 920 ml Tetrahydrofuran analog Beispiel

1b) methoxyliert (0,28 g Lithium in 35 ml Hethanol; 2,2 ml t-Butylhypochlorit, aufgeteilt in 2 Portionen zu 0,85 und 1 Portion zu 0,5 ml), aufgearbeitet und chromatographiert. Man erhält den 3-Acetoxymethyl-7α-methoxy-7β-[4-((2R)-2-BOC-amino-2-diphenylmethoxycarbonyläthoxycarbonylamino)-butyramido]-3-cephem-4-carbonsäurediphenylmethylester; $[\alpha]_D^{20°} = +47° \pm 1°$ (1,21% in $CHCl_3$); IR: 3465, 1790, 1730, 1695 (Schulter), 1498 $cm^{-1}$ ($CH_2Cl_2$); UV: 246 (5100), 251 (5200), 258 (5200), 263 (5400; $CH_2Cl_2$).

18c) 10,1 g 3-Acetoxymethyl-7α-methoxy-7β-[4-((2R)-2-BOC-amino-2-diphenylmethoxycarbonyläthoxycarbonylamino)-butyramido]-3-cephem-4-carbonsäurediphenylmethylester werden in 10 ml $CH_2Cl_2$ und 2,6 ml Anisol mit 25 ml Trifluoressigsäure analog Beispiel 1c) umgesetzt und aufgearbeitet. Man erält das 3-Acetoxymethyl-7α-methoxy-7β-[4-((2R)-2-amino-2-carboxyäthoxycarbonylamino)-butyramido]-3-cephem-4-carbonsäurenatriumsalz als Hydrat; Zersetzung ab 180°; $[\alpha]_D^{20°} = +126° \pm 1°$ (0,75% in $H_2O$); IR: 3600-2400 (breit), 1770, 1725, 1612, 1532 $cm^{-1}$ (Nujol); UV: 240 (5600), 265 (6600; $H_2O$).

18d) 7,2 g 3-Acetoxymethyl-7β-[4-((2R)-2-BOC-amino-2-diphenylmethoxycarbonyläthoxycarbonylamino)-butyramido]-3-cephem-4-carbonsäurediphenylmethylester werden in 10 ml $CH_2Cl_2$ und 1,9 ml Anisol mit 12 ml $CF_3COOH$ analog Beispiel 1c) umgesetzt und aufgearbeitet. Man erhält das 3-Acetoxymethyl-7β-[4-((2R)-2-amino-2-carboxyäthoxycarbonylamino)-butyramido]-3-cephem-4-carbonsäurenatriumsalz als Hydrat; Zersetzung ab 180°; IR: 3600-2400 (breit), 1765, 1712, 1656, 1611, 1539 $cm^{-1}$ (Nujol); UV: 257 (6800; $H_2O$).

18e) 2,5 g 3-Acetoxymethyl-7α-methoxy-7β-[4-((2R)-2-amino-2-carboxyäthoxycarbonylamino)-butyramido]-3-cephem-4-carbonsäurenatriumsalz werden mit 1,2 g 1-Methyl-5-mercapto-1H-tetrazol-natriumsalz analog Beispiel 1e) umgesetzt. Man erhält das 3-(1-Methyl-1H-tetrazol-5-ylthiomethyl)-7α-methoxy-7β-[4-((2R)-2-amino-2-carboxyäthoxycarbonylamino)-buyramido]-3-cephem-4-carbonsäurenatriumsalz als Hydrat; Zersetzung ab 185°; $[\alpha]_D^{20°} = +72° \pm 1°$ (0,92% in $H_2O$); IR: 3600-2400

(breit), 1772, 1712, 1610 (breit) 1535 $cm^{-1}$ (Nujol); UV: 238 (7000), 270 (8700; $H_2O$).

18f) 2,5 g 3-Acetoxymethyl-7β-[4-((2R)-2-amino-2-carboxyäthoxy-carbonylamino)-butyramido]-3-cephem-4-carbonsäurenatriumsalz werden mit 1,3 g 1-Methyl-5-mercapto-1H-tetrazol-natriumsalz analog Beispiel 1e) umgesetzt. Man erhält das 3-(1-Methyl-1H-tetrazol-5-ylthiomethyl)-7β-[4-((2R)-2-amino-2-carboxyäthoxycarbonylamino)-butyramido]-3-cephem-4-carbonsäurenatriumsalz als Hydrat; Zersetzung ab 190°; $[\alpha]_D^{20°}$ = +1° ± 1° (1,04% in $H_2O$); IR: 3600-2400, 1770, 1711, 1665-1600 (breit), 1540 $cm^{-1}$ (Nujol); UV: 267 (9700); $H_2O$).

Das Ausgangsmaterial für Beispiel 18a)-18f) kann wie folgt hergestellt werden:

18g) 30,5 g N-BOC-4-Aminobuttersäure und 65,7 g 3-Acetoxymethyl-7β-amino-3-cephem-4-carbonsäurediphenylmethylester werden analog Beispiel 1ℓ) umgesetzt (20,5 ml N-Methylmorpholin; 20 ml Chlorameisensäure-isobutylester; 1100 ml Tetrahydrofuran) und aufgearbeitet. Durch Kristallisation des Rohproduktes aus Aether erhält man den 3-Acetoxymethyl-7β-(4-BOC-aminobutyramido)-3-cephem-4-carbonsäurediphenylmethylester; IR: 3385, 1780, 1740-1690 (breit), 1512 (Schulter), 1492 $cm^{-1}$ ($CH_2Cl_2$); UV: 262 (7900; EtOH).

18h) 87 g 3-Acetoxymethyl-7β-(4-BOC-aminobutyramido)-3-cephem-4-carbonsäurediphenylmethylester werden mit 53 g p-Toluolsulfonsäuremonohydrat in 725 ml Acetonitril analog Beispiel 1m) umgesetzt und aufgearbeitet. Man erhält das 3-Acetoxymethyl-7β-(4-aminobutyramido)-3-cephem-4-carbonsäure-diphenylmethylester-p-toluolsulfonsäuresalz; IR: 3600-2400 (breit), 1781, 1727, 1690, 1550 (breit), 1485 $cm^{-1}$ (Nujol); UV: 260 (6900; EtOH).

Beispiel 19:

19a) 7,5 g (2R,S)-2-((2R)-2-BOC-Amino-2-diphenylmethoxycarbonyläthoxycarbonylamino)-2-(5-BOC-amino-1,2,4-thiadiazol-3-yl)-essigsäure und 5 g 3-(1-Methyl-1H-tetrazol-5-ylthiomethyl)-7β-amino-3-cephem-4-carbonsäurediphenylmethylester werden in 75 ml Tetrahydrofuran analog Beispiel 8a) umgesetzt, aufgearbeitet und umgefällt. Dabei erhält man den 3-(1-Methyl-1H-tetrazol-5-ylthiomethyl)-7β-[(2R,S)-2-((2R)-2-BOC-amino-2-diphenylmethoxycarbonyläthoxycarbonylamino)-2-(5-BOC-amino-1,2,4-thiadiazol-3-yl)-acetamido]-3-cephem-4-carbonsäurediphenylmethylester; IR: 3400, 1782, 1735-1690 (breit), 1521 (Schulter), 1490 $cm^{-1}$ ($CH_2Cl_2$); UV: 260 (16800); EtOH).

19b) 5,6 g 3-(1-Methyl-1H-tetrazol-5-ylthiomethyl)-7β-[(2R,S)-2-((2R)-2-BOC-amino-2-diphenylmethoxycarbonyläthoxycarbonylamino)-2-(5-BOC-amino-1,2,4-thiadiazol-3-yl)-acetamido]-3-cephem-4-carbonsäurediphenylmethylester werden in 9,6 ml $CH_2Cl_2$ und 4,8 ml Anisol mit 40 ml Trifluoressigsäure analog Beispiel 1c) umgesetzt (Reaktionsdauer 1 Std.) und aufgearbeitet. Man erhält das Hydrat des 3-(1-Methyl-1H-tetrazol-5-ylthiomethyl)-7β-[(2R,S)-2-((2R)-2-amino-2-carboxyäthoxycarbonylamino)-2-(5-amino-1,2,4-thiadiazol-3-yl)-acetamido]-3-cephem-4-carbonsäurenatriumsalzes; IR: 3600-2400 (breit), 1771, 1720-1680 (breit), 1620, 1520, 1465 $cm^{-1}$ (Nujol); UV: 258 (12900); $H_2O$).

Das Ausgangsmaterial für Beispiel 19a)-19b) kann wie folgt hergestellt werden:

19c) 14 g 2-(5-BOC-Amino-1,2,5-thiadiazol-3-yl)-2-Z-methoxyimino-essigsäuremethylester werden in 550 ml Methanol-Essigsäure-(1:1)-Gemisch in Gegenwart von 10 g 5-proz. Pd/C erschöpfend hydriert. Dann nutscht man vom Katalysator ab und dampft im Vakuum zur Trockene ein. Der Rückstand wird in Wasser gelöst, mit 2N wässriger NaOH auf pH 7 gestellt, mit Essigester extrahiert und dreimal mit Wasser gewaschen. Darauf wird die organische Phase dreimal mitje 50 ml 2N HCl extrahiert.

Die Essigesterphase wird eliminiert und die Wasseranteile werden unter Eiskühlung mit 2N NaOH wiederum auf pH 7 gestellt. Darauf extrahiert man erneut mit Essigester, wäscht mit gesättigter NaCl-Lösung neutral, trocknet über $Na_2SO_4$ und dampft im Vakuum ein. Dabei erhält man den (2R,S)-2-(5-BOC-Amino-1,2,4-thiadiazol-3-yl)-2-amino-essigsäuremethylester, der direkt weiterverarbeitet wird.

19d) 7,6 g (2R,S)-2-(-5-BOC-Amino-1,2,4-thiadiazol-3-yl)-2-amino-essigsäuremethylester werden in einem Gemisch von 25 ml Methanol und 71 ml 0,75 N Natronlauge 1 Std. bei Zimmertemperatur gerührt. Dann stellt man mit 1N HCl auf pH 7 und dampft im Vakuum zur Trockene ein. Den Eindampfrückstand löst man in 50 ml Wasser und stellt mit 2N NaOH auf pH 9,5. Darauf tropft man bei 0° im Verlaufe von 15 Minuten unter Rühren eine Lösung von 9,2 g (2R)-2-BOC-Amino-2-diphenylmethoxycarbonyläthoxycarbonylchlorid in 60 ml Acetonitril zu, wobei das pH bei 9,5 durch Zugabe von 2N NaOH (Titrator) konstant gehalten wird. Dann rührt man wiederum bei pH 9,5 und 0° 2 Std. nach. Darauf wird mit 2N HCl unter Eiskühlung auf pH 3 gestellt, mit Essigester extrahiert, mit gesättigter wässriger NaCl-Lösung neutral gewaschen, über $Na_2SO_4$ getrocknet und eingedampft. Dabei wird die (2R,S)-2-((2R)-2-BOC-Amino-2-diphenylmethoxycarbonyläthoxycarbonylamino)-2-(5-BOC-amino-1,2,4-thiadiazol-3-yl)-essigsäure erhalten, die direkt weiterverarbeitet wird.

Beispiel 20:

20a) Eine auf 0° gekühlte Lösung von 6,7 g (2R,S)-2-((2R)-2-BOC-Amino-2-diphenylmethoxycarbonyläthoxycarbonylamino)-2-(2-BOC-amino-thiazol-4-yl)-essigsäure und 1,35 g 1-Hydroxybenztriazol in 75 ml Tetrahydrofuran wird unter Rühren innerhalb 10 Minuten mit 2,27 g Dicyclohexylcarbodiimid, gelöst in 25 ml Tetrahydrofuran, versetzt. Nach zwei Stunden werden zu dieser Suspension 3,57 g 3-Methoxy-7β-amino-3-cephem-4-carbonsäurediphenylmethylester gegeben. Nach einer Reaktionszeit von einer Stunde bei 0° und 3 Stunden bei Zimmertemperatur wird der gebildete Niederschlag abfiltriert, und das Lösungsmittel abdestilliert. Darauf wird der Rückstand in Essigester (500 ml) aufgenommen und je dreimal mit 50 ml Eiswasser, 1N Salzsäure und gesättigter Natriumchloridlösung gewaschen. Man trocknet über Natriumsulfat, befreit das Rohprodukt vom Lösungsmittel und reinigt an Kieselgel mit Methylenchlorid-Essigester (4:1) als Eluiermittel, worauf der 3-Methoxy-7β-[(2R,S)-2-((2R)-BOC-amino-2-diphenylmethoxycarbonyläthoxycarbonylamino)-2-(2-BOC-aminothiazol-4-yl)-acetamido]-3-cephem-4-carbonsäurediphenylmethylester erhalten wird; DC (Silicagel, Identifikation mit Jod): $R_f$ ~0.58 (Methylenchlorid-Essigester 1:1).

20b) Analog Beispiel 20a) erhält man durch Behandeln von 6.7 g (2R,S)-2-((2R)-2-BOC-Amino-2-diphenylmethoxycarbonyläthoxycarbonylamino)-2-(2-BOC-aminothiazol-4-yl)-essigsäure und 3,29 g 7β-Amino-3-cephem-4-carbonsäurediphenylmethylester mit 1,35 g 1-Hydroxybenztriazol und 2,27 g Dicyclohexylcarbodiimid in Tetrahydrofuran (100 ml) als Lösungsmittel den 7β-[(2R,S)-2-((2R)-BOC-Amino-2-diphenylmethoxycarbonyläthoxycarbonylamino)-2-(2-BOC-aminothiazol-4-yl)-acetamido]-3-cephem-4-carbonsäurediphenylmethylester; DC (Silicagel, Identifikation mit Jod); $R_f$ ~0.68 (Methylenchlorid-Essigester 1:1).

20c) Analog Beispiel 20b) erhält man durch Behandeln von 4,02 g (2R,S)-2-((2R)-2-BOC-Amino-2-diphenylmethoxycarbonyläthoxycarbonylamino)-2-(2-BOC-amino-thiazol-4-yl)-essigsäure und 2,42 g 3-Carbamoyloxymethyl-7β-amino-3-cephem-4-carbonsäurediphenylmethylester mit 0,81 g 1-Hydroxybenztriazol und 1,24 g Dicyclohexylcarbodimid in Tetrahydrofuran (65 ml) als Lösungsmittel den 3-Carbamoyloxymethyl-7β-[(2R,S)-2-((2R)-BOC-amino-2-diphenylmethoxycarbonylamino)-2-(2-BOC-aminothiazol-4-yl)-acetamido]-3-cephem-4-carbonsäurediphenylmethylester; DC (Silicagel, Identifikation mit Jod): $R_f$ ~0.48 (Methylenchlorid-Essigester 1:1).

20d) Eine Lösung von 3,70 g des gemäss Beispiel 20a) erhältlichen 3-Methoxy-7β-[(2R,S)-2-((2R)-BOC-amino-2-diphenylmethoxycarbonyläthoxycarbonylamino)-2-(2-BOC-amino-thiazol-4-yl)-acetamido]-3-cephem-4-carbonsäurediphenylmethylesters und 3,7 ml Anisol in 18,5 ml Methylenchlorid wird mit 18,5 ml Trifluoressigsäure versetzt, während einer Stunde bei Zimmertemperatur gerührt und anschliessend bei 0° innerhalb 15 Min. mit Diäthyläther (200 ml) versetzt. Der so erhaltene Niederschlag des Trifluoressigsäuresalzes wird nach Filtration in Wasser (12 ml) gelöst und mit Essigester (3 x 5 ml) extrahiert. Die saure wässerige Phase (pH 1.5) wird bei 0° durch Zugabe von 2N Natronlauge auf pH 7 gebracht und mit Aethanol (45 ml) versetzt. Der gebildete Niederschlag wird abfiltriert, zur Entfernung des organischen Lösungsmittes in Wasser aufgenommen und am Rotationsverdampfer eingeengt. Nach Trocknen (16 Stunden, Zimmertemperatur, 0.05 Torr) wird das 3-Methoxy-7β-[(2R,S)-2-((2R)-amino-2-carboxyäthoxycarbonylamino)-2-(2-amino-thiazol-4-yl)-acetamido]-3-cephem-4-carbonsäurenatriumsalz in Form eines Dihydrates erhalten. Smp. ab 210° (unter Zersetzung); DC (Silicagel OPTI-UPC$_{12}$) Identifikation mit Ninhydrin): $R_f$~0.33 (Wasser).

20e) Analog Beispiel 20d) erhält man durch Behandeln von 8,2 g des gemäss Beispiel 20b) erhältlichen 7β-[(2R,S)-2-((2R)-BOC-Amino-2-diphenylmethoxycarbonyläthoxycarbonylamino)-2-(2-BOC-aminothiazol-4-yl)-acetamido]-3-cephem-4-carbonsäurediphenylmethylesters in Gegenwart von 8 ml Anisol mit 40 ml Trifluoressigsäure in 40 ml Methylenchlorid 7β-[(2R,S)-2-((2R)-2-Amino-2-carboxyäthoxycarbonylamino)-2-(2-aminothiazol-4-yl)-acetamido]-3-cephem-4-carbonsäurenatriumsalz in Form eines Trihydrates; Smp. ab 200° (unter Zersetzung); $[\alpha]_D^{20°}$ = +86° ± 1° (1.195% in 0.1N Salzsäure); UV: 252 nm (13100) in 0.1N Salzsäure; DC (Silicagel, OPTI-UP $C_{12}$, Identifikation mit Ninhydrin): $R_f$ ~ 0.78 (Wasser-Acetonitril 9:1).

20f) Analog Beispiel 20d) erhält man durch Behandeln von 3,9 g des gemäss Beispiel 20c) erhältlichen 3-Carbamoyloxymethyl-7β-[(2R,S)-2-((2R)-BOC-amino-2-diphenylmethoxycarbonyläthoxycarbonylamino)-2-(2-BOC-amino-thiazol-4-yl)-acetamido-3-cephem-4-carbonsäurediphenylmethylesters in Gegenwart von 4 ml Anisol mit 20 ml Trifluoressigsäure in 20 ml Methylenchlorid 3-Carbamoylmethyl-7β-[(2R,S)-(2-((2R)-amino-2-carboxyäthoxycarbonylamino)-2-(2-amino-thiazol-4-yl)-acetamido]-3-cephem-4-carbonatsäurenatriumsalz in Form eines Dihydrates; Smp. ab 200° (unter Zersetzung); $[\alpha]_D^{20°} = +54° \pm 1°$ (in 0,1N Salzsäure); UV: 252 nm (14900) in 0.1N Salzsäure; DC (Silicagel, OPTI-UP $C_{12}$, Identifikation mit Ninhydrin) $R_f$ ~0.77 (Wasser-Acetonitril 9:1).

Das Ausgangsmaterial für Beispiel 20a)-20c) kann wie folgt hergestellt werden:

20g) Ein Gemisch von 43,1 g 2-(2-Amino-thiazol-4-yl)-2-syn-methoximinoessigsäuremethylester und 131 g Di-tert.-butyl-dicarbonat wird 2 Stunden unter destillativer Entfernung des sich bildenden tert.-Butanols bei 110° erhitzt. Darauf werden nochmals 43,6 g Di-tert.-butyl-dicarbonat zugegeben. Nach einer Reaktionszeit von insgesamt 4 Stunden wird das Rohprodukt an Kieselgel mit Hexan-Diäthyläther (1:1) als Eluiermittel gereinigt, woraus 2-(2-BOC-Aminothiazol-4-yl)-2-syn-methoximino-essigsäuremethylester als amorphes Pulver erhalten wird. DC (Silicagel, Identifikation mit Jod): $R_f$ 0.67 (Diäthyläther); IR ($CH_2Cl_2$) u.a. charakteristische Absorptionsbanden bei 3395, 1730, 1645, 1155 und 942 $cm^{-1}$.

20h) Eine Lösung von 26.8 g 2-(2-BOC-Amino-thiazol-4-yl)-2-syn-methoximino-essigsäuremethylester in 1,1 Liter Essigsäure-Methanol (1:1) wird in Gegenwart von 13,4 g Palladium-Kohle (10%-ig) als Katalysator hydriert. Nach Aufnahme der theoretischen Menge Wasserstoff wird das Reaktionsgemisch über Celite filtriert und unter vermindertem Druck von Lösungsmittel befreit. Der Rückstand wird in Essigester

(1 Liter) aufgenommen und je zweimal mit 5%-iger Natriumhydrogencarbonat und Natriumchloridlösung gewaschen. Nach Trocknung der organischen Phase mit Natriumsulfat und Entfernung des Lösungsmittels erhält man den dünnschichtchromatographisch einheitlichen 2-(2R,S)-Amino-2-(2-BOC-amino-thiazol-4-yl)-essigsäuremethylester, der direkt im nächsten Reaktionsschritt eingesetzt wird. DC (Silicagel, Identifikatoin mit Jod); $R_f$ ~0.60 (sek. Butanol-Essigsäure-Wasser, 67:10:23).

20i) Eine Lösung von 28,94 g 2-(2R,S)-Amino-2-(2-BOC-amino-thiazol-4-yl)-essigsäuremethylester in 650 ml Aethanol wird unter Rühren bei Zimmertemperatur mit 32,4 g Kaliumhydroxid, gelöst in 276 ml Wasser, versetzt. Nach 1,5 Stunden wird das auf 0° gekühlte Reaktionsgemisch mit 4N Salzsäure auf pH 7 gestellt und unter vermindertem Druck auf ca. 350 ml eingeengt. Nach Ansäuern dieser Lösung auf pH 4,5 mit 4N Salzsäure wird der entstandene Niederschlag abfiltriert, und über Phosphorpentoxid getrocknet (16 Stunden, Zimmertemperatur, 0,01 Torr). Die so erhaltene 2-(2R,S)-Amino-2-(2-BOC-amino-thiazol-4-yl)-essigsäure enthält 0,5 Mol Wasser· Smp. 159-162° (unter Zersetzung); DC (Silicagel, Identifikation mit Ninhydrin): $R_f$ ~0,48 (sek. Butanol-Essigsäure-Wasser 67:10:23).

20k) Eine auf 0° gekühlte Suspension von 10,92 g 2-(2R,S)-Amino-2-(2-BOC-amino-thiazol-4-yl)-essigsäure in 300 ml Acetonitril-Wasser (1:1) wird mit 1N Natronlauge auf pH 9.5 gestellt und innerhalb 10 Minuten mit 19.07 g (2R)-2-BOC-Amino-2-diphenylmethoxycarbonyläthoxycarbonylchlorid, gelöst in 100 ml Acetonitril, versetzt. Der pH-Wert wird während der gesamten Reaktionszeit von 3 Stunden durch tropfenweise Zugabe von 1N Natronlauge bei pH 9.5 konstant gehalten. Darauf wird das Reaktonsgemisch mit 2N Salzsäure auf pH 2 gebracht, mit Essigester verdünnt und mit Eiswasser gewaschen. Nach Trocknen der organischen Phase über Natriumsulfat wird das Lösungsmittel abdestilliert, und der Rückstand an Silicagel mit Methylenchlorid-Essigester (4:1) als Eluiermittel gereinigt, woraus die (2R,S)-2-((2R)-2-BOC-Amino-2-diphenylmethoxycarbonyläthoxycarbonylamino)-2-(2-BOC-amino-thiazol-4-yl)-essigsäure erhalten wird. DC (Silicagel, Identifikation mit Jod) $R_f$ ~0.75 (Chloroform-Methanol-Essigsäure 75:22:13).

Beispiel 21: Analog den vorangehenden Beispielen können ausgehend von entsprechenden Ausgangsmaterialien und Zwischenprodukten die folgenden Verbindungen hergestellt werden:

3-(4-Methyl-5,6-dioxo-1,4,5,6-tetrahydro-as-triazin-3-ylthiomethyl)-7β-[2-((2R)-2-amino-2-carboxyäthoxycarbonylamino)-acetamido]-3-cephem-4-carbonsäure; IR: 3600-2400 (breit), 1770, 1718, 1648 (breit), 1570, 1510 $cm^{-1}$ (Nujol); UV: 234 (16800), 278 (24100; $H_2O$).

3-Pyridiniomethyl-7β-[2-((2R)-2-amino-2-carboxyäthoxycarbonylamino)-acetamido]-3-cephem-4-carbonsäure; IR: 3600-2400 (breit), 1770, 1725, 1655 (breit), 1570, 1510 $cm^{-1}$ (Nujol); UV 234 (16900), 277 (23600; $H_2O$).

3- Pyridiniomethyl-7β-[(2S)-2-((2R)-2-amino-2-carboxyäthoxycarbonyl-amino)-2-(2-aminothiazol-4-yl)-acetamido]-3-cephem-4-carboxylat; IR: 3600-2450 (breit), 1770, 1720 (Schulter), 1690 (breit), 1615 (breit), 1520 $cm^{-1}$ (Nujol); UV: 259 (14100; $H_2O$).

3-(2-Methyl-5,6-dioxo-1,4,5,6-tetrahydro-as-triazin-2-ylthiomethyl)-7β-[(2S)-2-((2R)-2-amino-2-carboxyäthoxycarbonylamino)-2-(2-amino-thiazol-4-yl)-acetamido]-3-cephem-4-carbonsäure; IR: 3600-2390 (breit), 1772, 1720, 1650, (breit), 1575, 1510 $cm^{-1}$ (Nujol); UV: 279 (21200; $H_2O$).

3-(4-Methyl-5,6-dioxo-1,4,5,6-tetrahydro-as-triazin-3-ylthiomethyl)-7β-[(2S)-2-((2R)-2-amino-2-carboxyäthoxycarbonylamino)-2-(2-amino-thiazol-4-yl)-acetamido]-3-cephem-4-carbonsäure; IR: 3600-2400, 1775, 1722, 1655 (breit), 1570, 1510 $cm^{-1}$ (Nujol); UV: 278 (20900; $H_2O$).

7β-[(2S)-2-((2R)-2-amino-2-carboxyäthoxycarbonylamino)-2-(5-amino-1,2,4-thiadiazol-3-yl)-acetamido]-3-cephem-4-carbonsäure; IR: 3600-2500 (breit), 1770, 1715, 1680, 1610, 1520, 1460 $cm^{-1}$ (Nujol); UV: 256 (11000; $H_2O$).

3-Acetoxymethyl-7β-[(2S)-2-((2R)-2-amino-2-carboxyäthoxycarbonylamino)-2-(5-amino-1,2,4-thiadiazol-3-yl)-acetamido]-3-cephem-4-carbonsäure; IR: 3600-2500, 1775, 1725, 1715, 1683, 1610, 1520 $cm^{-1}$ (Nujol); UV: 252 (12000; $H_2O$).

3-(4-Carbamoylpyridiniomethyl)-7β-[(2S)-2-((2R)-2-amino-2-carboxyäthoxycarbonylamino)-2-(5-amino-1,2,4-thiadiazol-3-yl)-acetamido]-3-cephem-4-carboxylat; IR: 3600-2400 (breit), 1772, 1720 (Schulter), 1690 (breit), 1615 (breit), 1520 $cm^{-1}$ (Nujol); UV: 259 (13900; $H_2O$).

3-Carbamoyloxymethyl-7β-[(2S)-2-((2R)-2-amino-2-carboxyäthoxycarbonylamino)-2-(5-amino-1,2,4-thiadiazol-3-yl)-acetamido]-3-cephem-4-carbonsäure; IR: 3600-2400 (breit), 1769, 1720, 1690 (breit), 1620 (breit), 1620 (breit), 1520 $cm^{-1}$ (Nujol); UV: 252 (14000; $H_2O$).

3-Pyridiniomethyl-7β-[(2S)-2-((2R)-2-amino-2-carboxyäthoxycarbonylamino)-2-(5-amino-1,2,4-thiadiazol-3-yl)-acetamido]-3-cephem-4-carboxylat; IR: 3600-2400 (breit), 1770, 1720, 1690 (breit) 1610 (breit), 1515 $cm^{-1}$ (Nujol); UV: 259 (13300; $H_2O$).

3-(1-Carboxymethyl-1H-tetrazol-5-ylthiomethyl)-7β-[(2S)-2-((2R)-2-amino-2-carboxyäthoxycarbonylamino)-2-(5-amino-1,2,4-thiadiazol-3-yl)-acetamido]-3-cephem-4-carbonsäure; IR: 3600-2600 (breit), 1765, 1740-1570 (breit), 1520 $cm^{-1}$ (Nujol); UV: 258 (13400; $H_2O$).

3-(1-Sulfomethyl-1H-tetrazol-5-ylthiomethyl)-7β-[(2S)-2-((2R)-2-amino-2-carboxyäthoxycarbonylamino)-2-(5-amino-1,2,4-thiadiazol-3-yl)-acetamido]-3-cephem-4-carbonsäure; IR: 3600-2500 (breit), 1765, 1728 (Schulter), 1681 (Schulter), 1625, 1520 $cm^{-1}$ (Nujol); UV: 258 (14000; $H_2O$).

3-(2-Methyl-5,6-dioxo-1,4,5,6-tetrahydro-as-triazin-3-ylthio)-7β-[(2S)-2-((2R)-2-amino-2-carboxyäthoxycarbonylamino)-2-(5-amino-1,2,4-thiadiazol-3-yl)-acetamido]-3-cephem-4-carbonsäure; IR: 3600-2400 (breit), 1770, 1720, 1650 (breit) 1575 $cm^{-1}$ (Nujol); UV: 278 (20900; $H_2O$).

3-(4-Methyl-5,6-dioxo-1,4,5,6-tetrahydro-as-triazin-3-ylthiomethyl)-7β-[(2S)-2-((2R)-2-amino-2-carboxyäthoxycarbonylamino)-2-(5-amino-1,2,4-thiadiazol-3-yl)-acetamido]-3-cephem-4-carbonsäure; IR: 3600-2400 (breit), 1771, 1715 (Schulter), 1650 (breit) $cm^{-1}$ (Nujol); UV: 279 (19200; $H_2O$).

3-[1-(2-Dimethylaminoäthyl)-1H-tetrazol-5-ylthiomethyl]-7β-[(2S)-2-((2R)-2-amino-2-carboxyäthoxycarbonylamino)-2-(5-amino-1,2,4-thiadiazol-3-yl)-acetamido]-3-cephem-4-carbonsäure; IR: 3600-2500 (breit), 1770, 1712 (Schulter), 1680 (Schulter), 1615, 1508 $cm^{-1}$ (Nujol); UV: 254 (13900; $H_2O$).

7β-[(2S)-2-((3R)-3-amino-3-carboxypropionamido)-2-(2-amino-thiazol-4-yl)-acetamido]-3-cephem-4-carbonsäure; IR: 3600-2400 (breit), 1765, 1710-1575 (breit), 1500 $cm^{-1}$ (Nujol); UV: 259 (12000; $H_2O$).

3-(4-Carbamoylpyridiniomethyl)-7β-[(2S)-2-((3R)-3-amino-3-carboxypropionamido)-2-(2-amino-thiazol-4-yl)-acetamido]-3-cephem-4-carboxylat; IR: 3600-2400 (breit), 1770, 1719, 1685 (breit), 1620 (breit), 1520 $cm^{-1}$ (Nujol); UV: 252 (14000, $H_2O$.

3-Carbamoyloxymethyl-7β-[(2S)-2-((3R)-3-amino-3-carboxypropionamido)-2-(2-amino-thiazol-4-yl)-acetamido]-3-cephem-4-carbonsäure; IR: 3600-2400 (breit), 1772, 1721, 1690 (breit), 1620 (breit), 1520 $cm^{-1}$ (Nujol); UV: 252 (13900; $H_2O$).

3-Pyridiniomethyl-7β-[(2S)-2-((3R)-3-amino-3-carboxypropionamido)-2-(2-amino-thiazol-4-yl)-acetamido]-3-cephem-4-carboxylat. IR: 3600-2400 (breit), 1770, 1720, 1690 (breit), 1620 (breit) $cm^{-1}$ (Nujol); UV: 251 (13800; $H_2O$).

3-(1-Carboxymethyl-1H-tetrazol-5-ylthiomethyl)-7β-[(2S)-2-((3R)-3-amino-3-carboxypropionamido)-2-(2-amino-thiazol-4-yl)-acetamido]-3-cephem-4-carbonsäure; IR: 3600-2600 (breit), 1768, 1740-1570 (breit), 1520 $cm^{-1}$ (Nujol); UV: 258 (14800; $H_2O$).

3-(1-Sulfomethyl-1H-tetrazol-5-ylthiomethyl)-7β-[(2S)-2-((3R)-3-amino-3-carboxypropionamido)-2-(2-amino-thiazol-4-yl)-acetamido]-3-cephem-4-carbonsäure; IR: 3600-2500 (breit), 1766, 1728 (Schulter), 1680 (Schulter), 1625, 1520 $cm^{-1}$ (Nujol); UV: 258 (13500; $H_2O$).

3-(2-Methyl-5,6-dioxo-1,4,5,6-tetrahydro-as-triazin-3-ylthiomethyl)-7β-[(2S)-2-((3R)-3-amino-3-carboxypropionamido)-2-(2-amino-thiazol-4-yl)-acetamido]-3-cephem-4-carbonsäure; IR: 3600-2400 (breit), 1770, 1720, 1650 (breit), 1575 $cm^{-1}$ (Nujol); UV: 278 (20100; $H_2O$).

3-[1-(2-Dimethylaminoäthyl)-1H-tetrazol-5-yl-thiomethyl]-7β-[(2S)-2-((3R)-3-amino-3-carboxypropionamido)-2-(2-amino-thiazol-4-yl)-acetamido]-3-cephem-4-carbonsäure; IR: 3600 (breit), 1770, 1712 (Schulter), 1680 (Schulter), 1615, 1508 $cm^{-1}$ (Nujol); UV: 254 (14000; $H_2O$).

7β-[(2S)-2-((4R)-4-amino-4-carboxybutyramido)-2-(2-amino-thiazol-4-yl)-acetamido]-3-cephem-4-carbonsäure; IR: 3600-2400 (breit), 1766, 1710-1570 (breit), 1500 $cm^{-1}$ (Nujol); UV: 258 (11400; $H_2O$).

3-Acetoxymethyl-7β-[(2S)-2-((4R)-4-amino-4-carboxybutyramido)-2-(2-amino-thiazol-4-yl)-acetamido]-3-cephem-4-carbonsäure; IR: 3600-2400 (breit), 1768, 1725-1570 (breit), 1500 $cm^{-1}$ (Nujol); UV: 259 (14700; $H_2O$).

3-(4-Carbamoylpyridiniomethyl)-7β-[(2S)-2-((4R)-4-amino-4-carboxy-butyramido)-2-(2-amino-thiazol-4-yl)-acetamido]-3-cephem-4-carboxylat; IR: 3600-2400 (breit), 1771, 1720, 1680 (breit), 1620 (breit), 1520 $cm^{-1}$ (Nujol); UV: 253 (12900; $H_2O$).

3-Carbamoyloxymethyl-7β-[(2S)-2-((4R)-4-amino-4-carboxybutyramido)-2-(2-amino-thiazol-4-yl)-acetamido]-3-cephem-4-carbonsäure; IR: 3600-2400 (breit), 1771, 1720, 1690 (breit), 1620 (breit), 1515 $cm^{-1}$ (Nujol). UV: 252 (13000; $H_2O$).

3-Pyridiniomethyl-7β-[(2S)-2-((4R)-4-amino-4-carboxybutyramido)-2-(2-amino-thiazol-4-yl)-acetamido]-3-cephem-4-carboxylat; IR: 3600-2400 (breit, 1769, 1722, 1690 (breit), 1615 (breit) $cm^{-1}$ (Nujol); UV: 252 (11900; $H_2O$).

3-(1-Carboxymethyl-1H-tetrazol-4-ylthiomethyl)-7β-[(2S)-2-((4R)-4-amino-4-carboxybutyramido)-2-(2-amino-thiazol-4-yl)-acetamido]-3-cephem-4-carbonsäure; IR: 3600-2600, 1769, 1740-1570 (breit), 1520 $cm^{-1}$ (Nujol); UV: 258 (14500; $H_2O$).

3-(1-Sulfomethyl-1H-tetrazol-5-ylthiomethyl)-7β-[(2S)-2-((4R)-4-amino-4-carboxybutyramido)-2-(2-amino-thiazol-4-yl)-acetamido]-3-cephem-4-carbonsäure; IR: 3600-2500 (breit), 1768, 1727 (Schulter), 1680 (Schulter), 1625, 1520 $cm^{-1}$ (Nujol); UV: 258 (13700; $H_2O$).

3-(2-Methyl-5,6-dioxo-1,4,5,6-tetrahydro-as-triazin-3-ylthiomethyl)-7β-[(2S)-2-((4R)-4-amino-4-carboxybutyramido)-2-(2-aminothiazol-4-yl)-acetamido]-3-cephem-4-carbonsäure; IR: 3600-2400 (breit), 1769, 1718, 1655 (breit), 1575 $cm^{-1}$ (Nujol); UV: 278 (20000; $H_2O$).

3-[1-(2-Dimethylaminoäthyl)-1H-tetrazol-5-yl-thiomethyl]-7β-[(2S)-2-((4R)-4-amino-4-carboxybutyramido)-2-(2-amino-thiazol-4-yl)-acetamido]-3-cephem-4-carbonsäure; IR: 3600 (breit), 1771, 1715 (Schulter), 1680, 1615, 1510 $cm^{-1}$ (Nujol); UV: 254 (13300; $H_2O$).

7β-[(2S)-2-((3R)-3-amino-3-carboxypropionamido)-2-(5-amino-1,2,4-thiadiazol-3-yl)-acetamido]-3-cephem-4-carbonsäure; IR: 3600-2400 (breit), 1766, 1712-1577 (breit), 1500 $cm^{-1}$ (Nujol); UV: 258 (11800; $H_2O$).

3-Acetoxymethyl-7β-[(2S)-2-((3R)-3-amino-3-carboxypropionamido-2-(5-amino-1,2,4-thiadiazol-3-yl)-acetamido]-3-cephem-4-carbonsäure; IR: 3600-2400 (breit), 1771, 1720, 1690 (breit), 1615 (breit), 1515 $cm^{-1}$ (Nujol); UV: 253 (14200; $H_2O$).

3-(4-Carbamoylpyridiniomethyl)-7β-[(2S)-2-((3R)-3-amino-3-carboxypropionamido)-2-(5-amino-1,2,4-thiadiazol-3-yl)-acetamido]-3-cephem-4-carboxylat; IR: 3600-2400 (breit), 1769, 1718, 1680 (breit), 1620 (breit), 1515 $cm^{-1}$;UV: 252 (13500; $H_2O$).

3-Carbamoyloxymethyl-7β-[(2S)-2-((3R)-3-amino-3-carboxypropionamido)-2-(5-amino-1,2,4-thiadiazol-3-yl)-acetamido]-3-cephem-4-carbonsäure; IR: 3600-2400 (breit), 1771, 1720, 1690 (breit), 1620 (breit), 1518 $cm^{-1}$ (Nujol); UV: 252 (13900; $H_2O$).

3-Pyridiniomethyl-7β-[(2S)-2-((3R)-3-amino-3-carboxypropionamido)-2-(5-amino-1,2,4-thiadiazol-3-yl)-acetamido]-3-cephem-4-carbon-
IR: 3600-2400 (breit), 1769, 1720, 1690 (breit), 1620 (breit) $cm^{-1}$ (Nujol); UV: 251 (13700; $H_2O$).

3-(1-Methyl-1H-tetrazol-5-ylthiomethyl)-7β-[(2S)-2-((3R)-3-amino-3-carboxypropionamido)-2-(5-amino-1,2,4-thiadiazol-3-yl)-acetamido]-3-cephem-4-carbonsäure; IR: 3600-2600 (breit), 1769, 1740-1570 (breit), 1520 $cm^{-1}$ (Nujol); UV: 259 (14200; $H_2O$).

3-(1-Carboxymethyl-1H-tetrazol-5-ylthiomethyl)-7β-[(2S)-2-((3R)-3-amino-3-carboxypropionamido)-2-(5-amino-1,2,4-thiadiazol-3-yl)-acetamido]-3-cephem-4-carbonsäure; IR: 3600-2600 (breit), 1770, 1740-1570 (breit), 1520 $cm^{-1}$ (Nujol); UV: 259 (12850; $H_2O$).

3-(1-Sulfomethyl-1H-tetrazol-5-ylthiomethyl)-7β-[(2S)-((3R)-3-amino-3-carboxypropionamido)-2-(5-amino-1,2,4-thiadiazol-3-yl)-acetamido-3-cephem-4-carbonsäure; IR: 3600-2500 (breit), 1768, 1730 (Schulter), 1685 (Schulter), 1625, 1519 $cm^{-1}$ (Nujol); UV: 258 (14200; $H_2O$).

3-(2-Methyl-5,6-dioxo-1,4,5,6-tetrahydro-as-triazin-3-ylthiomethyl)-7β-[(2S)-2-((3R)-3-amino-3-carboxypropionamido)-2-(5-amino-1,2,4-thiadiazol-2-yl)-acetamido]-3-cephem-4-carbonsäure; IR: 3600-2400 (breit), 1768, 1722, 1650 (breit), 1575 $cm^{-1}$ (Nujol); UV: 279 (22000; $H_2O$).

3-[1-(2-Dimethylaminoäthyl)-1H-tetrazol-5-ylthiomethyl]-7β-[(2S)-2-((3R)-3-amino-3-carboxypropionamido)-2-(5-amino-1,2,4-thiadiazol-3-yl)-acetamido]-3-cephem-4-carbonsäure; IR: 3600-2600 (breit) 1771, 1712 (Schulter), 1680 (Schulter), 1617, 1510 $cm^{-1}$ (Nujol); UV: 255 (13000; $H_2O$).

7β-[(2S)-2-((4R)-4-amino-4-carboxybutyramido)-2-(5-amino-1,2,4-thiadiazol-3-yl)-acetamido]-3-cephem-4-carbonsäure; IR: 3600-2450 (breit), 1767, 1710-1570 (breit), $cm^{-1}$ (Nujol); UV: 257 (12400; $H_2O$).

3-Acetoxymethyl-7β-[(2S)-2-((4R)-4-amino-4-carboxybutyramido)-2-(5-amino-1,2,4-thiadiazol-3-yl)-acetamido]-3-cephem-4-carbonsäure; IR: 3600-2400 (breit), 1767, 1720-1570 (breit), 1502 $cm^{-1}$ (Nujol); UV: 259 (14400); $H_2O$).

3-(4-Carbamoylpyridiniomethyl)-7β-[(2S)-2-((4R)-4-amino-4-carboxybutyramido)-2-(5-amino-1,2,4-thiadiazol-3-yl]-acetamido]-3-cephem-4-carboxylat; IR: 3600-2420 (breit), 1770, 1720, 1680 (breit), 1620 (breit), 1520 $cm^{-1}$ (Nujol); UV: 254 (13100; $H_2O$).

3-Carbamoyloxymethyl-7β-[(2S)-2-((4R)-4-amino-4-carboxybutyramido)-2-(5-amino-1,2,4-thiadiazol-3-yl)-acetamido]-3-cephem-4-carbonsäure; IR: 3600-2400 (breit), 1771, 1720, 1690 (breit), 1615 (breit), 1520 $cm^{-1}$ (Nujol); UV: 252 (14200; $H_2O$).

3-Pyridiniomethyl-7β-[(2S)-2-((4R)-4-amino-4-carboxybutyramido)-2-(5-amino-1,2,4-thiadiazol-3-yl)-acetamido]-3-cephem-4-carboxylat; IR: 3600-2400 (breit), 1770, 1724, 1695 (breit), 1625 (breit) $cm^{-1}$ (Nujol); UV: 251 (12900; $H_2O$).

3-(1-Methyl-1H-tetrazol-5-ylthiomethyl)-7β-[(2S)-2-((4R)-4-amino-4-carboxybutyramido)-2-(5-amino-1,2,4-thiadiazol-3-yl)-acetamido]-3-cephem-4-carbonsäure; IR: 3600-2600 (breit), 1770, 1740-1570 (breit), 1520 $cm^{-1}$ (Nujol); UV: 259 (13950; $H_2O$).

3-(1-Carboxymethyl-1H-tetrazol-5-ylthiomethyl)-7β-[(2S)-2-((4R)-4-amino-4-carboxybutyramido)-2-(5-amino-1,2,4-thiadiazol-3-yl)-acetamido]-3-cephem-4-carbonsäure; IR: 3600-2600 (breit), 1772, 1740-1570 (breit), 1520 $cm^{-1}$ (Nujol); UV: 258 (12800; $H_2O$).

3-(1-Sulfomethyl-1H-tetrazol-5-ylthiomethyl)-7β-[(2S)-2-((4R)-4-amino-4-carboxybutyramido)-2-(5-amino-1,2,4-thiadiazol-3-yl)-acetamido]-3-cephem-4-carbonsäure; IR: 3600-2500 (breit), 1768, 1725

(Schulter), 1680 (Schulter), 1625, 1519 $cm^{-1}$ (Nujol); UV: 258 (14000; $H_2O$).

3-(2-Methyl-5,6-dioxo-1,4,5,6-tetrahydro-as-triazin-3-ylthiomethyl)-7β-[(2S)-2-((4R)-4-amino-4-carboxybutyramido)-2-(5-amino-1,2,4-thiadiazol-2-]1)-acetamido]-3-cephem-4-carbonsäure; IR: 3600-2400 (breit), 1769, 1725, 1650 (breit), 1575 $cm^{-1}$ (Nujol); UV: 279 (21300; $H_2O$).

3-[1-(2-Dimethylaminoäthyl)-1H-tetrazol-5-ylthiomethyl]-7β-[(2S)-2-((4R)-4-amino-4-carboxybutyramido)-2-(5-amino-1,2,4-thiadiazol-3-yl)-acetamido]-3-cephem-4-carbonsäure; IR: 3600-2600 (breit), 1769, 1712 (Schulter), 1680 (Schulter), 1615, 1512 $cm^{-1}$ (Nujol); UV: 256 (12910; $H_2O$),
sowie die entsprechenden 7α-Methoxy-Verbindungen und die entsprechenden R- und R,S-Derivate und ihre Salze, z.B. die Natriumsalze.

Beispiel 22: Trockenampullen oder Vialen, enthaltend 0,5 g 3-(1-Methyl-1H-tetrazol-5-ylthiomethyl)-7β-[(2S)-2-((2R)-2-amino-2-carboxyäthoxycarbonylamino)-2-(2-aminothiazol-4-yl)-acetamido]-3-cephem-4-carbonsäure-natriumsalz werden wie folgt hergestellt:

| | |
|---|---|
| 3-(1-Methyl-1H-tetrazol-5-ylthiomethyl)-7β-[(2S)-2-((2R)-2-amino-2-carboxyäthoxycarbonylamino)-2-(2-aminothiazol-4-yl)-acetamido]-3-cephem-4-carbonsäure-natriumsalz | 0,5 g |
| Mannit | 0,05 g |

Eine sterile wässrige Lösung von 3-(1-Methyl-1H-tetrazol-5-ylthiomethyl)-7β-[(2S)-2-((2R)-2-amino-2-carboxyäthoxycarbonylamino)-2-(2-aminothiazol-4-yl)-acetamido]-3-cephem-4-carbonsäure-natriumsalz und Mannit wird unter aseptischen Bedingungen in 5 ml Ampullen oder 5 ml Vialen verschlossen und geprüft

Patentansprüche (für alle benannten Länder ausser Oesterreich):

1. Verbindungen der Formel

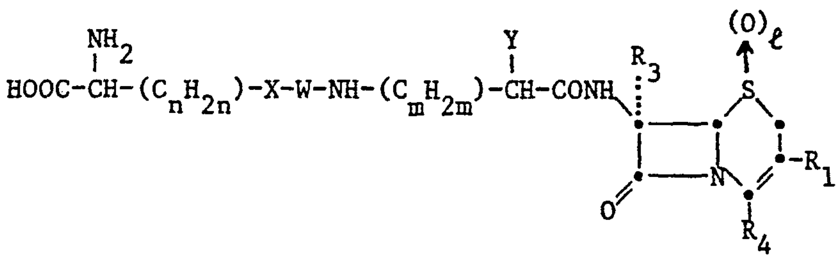

(I),

worin

$\ell$ eine ganze Zahl von 0 bis 2,

m eine ganze Zahl von 0 (direkte Bindung) bis 4,

n eine ganze Zahl von 1 bis 4,

X Sauerstoff, Schwefel oder die Gruppe -NH-,

W eine Gruppe -CO-, $-CONHSO_2-$ oder $-SO_2NHCO-$ oder X und W zusammen eine Gruppe -CO- oder $-CONHSO_2-$,

Y Wasserstoff oder einen unsubstituierten oder substituierten organischen Rest,

$R_1$ Wasserstoff, Niederalkyl, Niederalkoxy, Halogen oder eine Gruppe der Formel $-CH_2-R_2$, worin

$R_2$ eine freie, veresterte oder verätherte Hydroxyl- oder Mercaptogruppe oder eine quaternäre Ammoniumgruppe darstellt,

$R_3$ Wasserstoff oder Niederalkoxy und

$R_4$ Carboxyl oder geschütztes Carboxyl bedeuten,

Hydrate und Salze von Verbindungen der Formel I.

2. Verbindungen der Formel I, worin m 0 (direkte Bindung) bedeutet und l, n, X, W, Y, $R_1$, $R_2$, $R_3$ und $R_4$ die in Anspruch 1 genannten Bedeutungen haben, Hydrate und Salze von solchen Verbindungen.

3. Verbindungen der Formel I gemäss Anspruch 1, worin

$\ell$ eine ganze Zahl von 0 bis 2, m eine ganze Zahl von 0 (direkte Bindung) bis 4, n eine ganze Zahl von 1 bis 4, die Gruppen $-(C_mH_{2m})-$ und $-(C_nH_{2n})-$ unverzweigt sind, X Sauerstoff oder die Gruppe -NH-, W die Gruppe -CO- oder X-W- zusammen die Gruppe -CO-, Y Wasserstoff, Niederalkyl, z.B. Methyl, unsubstituiertes oder durch Amino substituiertes Furyl, Thienyl, Thiazolyl oder Thiadiazolyl, z.B. Furyl, Thienyl, Aminothiazolyl oder Aminothiadiazolyl, $R_1$ Wasserstoff, Niederalkoxy, z.B. Methoxy oder Aethoxy, Halogen, z.B. Chlor, oder eine Gruppe der Formel $-CH_2-R_2$, worin $R_2$ Niederalkanoyloxy, z.B. Acetyloxy, Carbamoyloxy, N-Niederalkylcarbamoyloxy, unsubstituiertes oder durch Niederalkyl, z.B. Methyl, Diniederalkylaminoniederalkyl, z.B. Dimethylaminomethyl, Carboxyniederalkyl, z.B. Carboxymethyl, Amino, Carboxyniederalkylamino, z.B. 2-Carboxyäthylamino oder Carbamoyl substituiertes Triazolylthio, z.B. 1H-1,2,3-Triazol-5-ylthio, Tetrazolylthio, z.B. 1H-Tetrazol-5-ylthio, Thiazolylthio, Thiadiazolylthio, z.B. 1,3,4-Thiadiazol-5-ylthio, Oxazolylthio, Oxadiazolylthio oder 5,6-Dioxotetrahydro-as-triazinylthio, z.B. 5,6-Dioxo-1,2,5,6-etrahydro-as-triazin-3-ylthio oder 5,6-Dioxo-1,4,5,6-tetrahydro-as-triazin-3-ylthio, 2-Niederalkyl-1-pyrazolium, z.B. 2-Methyl-1-pyrazolium, 2-Carboxyniederalkyl-1-pyrazolium, z.B. 2-Carboxymethyl-1-pyrazolium, 3-Niederalkyl-1-triazolium, z.B. 3-Methyl-1-triazolium, oder unsubstituiertes oder durch Hydroxyniederalkyl, z.B. Hydroxymethyl, Carboxy, Carboxyniederalkyl, z.B. Carboxymethyl, Halogen, z.B. Chlor oder Brom oder Carbamoyl substituiertes Pyridinium, z.B. 3- oder 4-Hydroxymethylpyridinium, 4-Carboxypyridinium, 3- oder 4-Carboxymethylpyridinium, 3- oder 4-Brompyridinium oder 3- oder 4-Carbamoylpyridinium, $R_3$ Wasserstoff oder Niederalkoxy, z.B. Methoxy, und $R_4$ Carboxyl, Pivaloyloxymethoxycarbonyl oder 1-Aethoxycarbonyloxyäthoxycarbonyl bedeuten, Hydrate und pharmazeutisch verwendbare Salze von solchen Verbindungen.

4. Verbindungen der Formel I gemäss Anspruch 2, worin m 0 (direkte Bindung) bedeutet und l, n, X, W, Y, $R_1$, $R_2$, $R_3$ und $R_4$ die in Anspruch 3 genannten Bedeutungen haben, Hydrate und pharmazeutisch verwendbare Salze von solchen Verbindungen.

5. Verbindungen der Formel I gemäss Anspruch 3, worin $\ell$ 0, m eine ganze Zahl von 1 bis 4, die Gruppen $-(C_mH_{2m})-$ und $-(C_nH_{2n})-$ unverzweigt sind, X Sauerstoff oder die Gruppe -NH-, W die Gruppe -CO- oder X-W zusammen die Gruppe -CO-, Y Wasserstoff, Niederalkyl, z.B. Methyl, Furyl, z.B. 2- oder 3-Furyl, Thienyl, z.B. 2- oder 3-Thienyl, Aminothiazolyl, z.B. 2-Aminothiazol-4-yl, oder Aminothiadiazolyl, z.B. 5-Amino-1,2,4-thiadiazol-3-yl, $R_1$ Wasserstoff, Niederalkoxy, z.B. Methoxy, Halogen, z.B. Chlor, oder eine Gruppe der Formel $-CH_2-R_2$, worin $R_2$ Niederalkanoyloxy, z.B. Acetyloxy, Carbamoyloxy, unsubstituiertes oder durch Niederalkyl, z.B. Methyl, Diniederalkylaminoniederalkyl, z.B. 2-Dimethylaminoäthyl, Sulfoniederalkyl, z.B. Sulfomethyl, Carboxyniederalkyl, z.B. Carboxymethyl, oder durch Carbamoyl substituiertes Triazolylthio, z.B. 1H-1,2,3-Triazol-5-ylthio, Tetrazolylthio, z.B. 1H-Tetrazol-5-ylthio, 1-Methyl-1H-tetrazol-5-ylthio, 1-Sulfomethyl-1H-tetrazol-5-ylthio, 1-Carboxymethyl-1H-tetrazol-5-ylthio oder 1-(2-Dimethylaminoäthyl)-1H-tetrazol-5-ylthio, Thiadiazolylthio, z.B. 1,3,4-Thiadiazol-5-ylthio oder 2-Methyl-1,3,4-thiadiazol-5-ylthio, 5,6-Dioxotetrahydrotriazin-3-ylthio, z.B. 2-Methyl-5,6-dioxo-1,2,5,6-tetrahydro-as-triazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, oder unsubstituiertes oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinium, z.B. 3- oder 4-Hydroxymethylpyridinium, 4-Carboxypyridinium, 3- oder 4-Carboxymethylpyridinium, 3- oder 4-Chlorpyridinium, 3- oder 4-Carboxymethylpyridinium, 3- oder 4-Chlorpyridinium, 3- oder 4-Brompyridinium oder 3- oder 4-Carbamoylpyridinium bedeutet, $R_3$ Wasserstoff oder Methoxy und $R_4$ Carboxyl be-

deuten, Hydrate und pharmazeutisch verwendbare Salze von solchen Verbindungen.

6. Verbindungen der Formel I gemäss Anspruch 4, worin m 0 (direkte Bindung) bedeutet und worin $\ell$, n, X, W, Y, $R_1$, $R_2$, $R_3$ und $R_4$ die in Anspruch 5 genannten Bedeutungen haben, Hydrate und Salze von solchen Verbindungen.

7. Verbindungen der Formel I gemäss Anspruch 5, worin $\ell$ 0, m eine ganze Zahl von 0 (direkte Bindung) bis 3, n 1 oder 2, X Sauerstoff, W die Gruppe -CO- oder X-W zusammen die Gruppe -CO-, Y Wasserstoff, Niederalkyl, z.B. Methyl, Furyl, z.B. 2- oder 3-Furyl, Aminothiazolyl, z.B. 2-Aminothiazol-4-yl, oder Aminothiadiazolyl, z.B. 5-Amino-1,2,4-thiadiazol-3-yl, $R_1$ Wasserstoff, Niederalkoxy, z.B. Methoxy, Halogen, z.B. Chlor, oder eine Gruppe der Formel $CH_2$-$R_2$, worin $R_2$ Niederalkanoyloxy, z.B. Acetyloxy, Carbamoyloxy, Tetrazolylthio, z.B. 1H-Tetrazol-5-ylthio, durch Niederalkyl, z.B. Methyl, Diniederalkylaminoniederalkyl, z.B. Dimethylaminoäthyl, Sulfoniederalkyl, z.B. Sulfomethyl, oder Carboxyniederalkyl, z.B. Carboxymethyl substituiertes Tetrazolylthio, z.B. 1-Methyl-1-tetrazol-5-ylthio, 1-(2-Dimethylaminoäthyl)-1H-tetrazol-5-ylthio, 1-Carboxymethyl-1H-tetrazol-5-ylthio, 1-Sulfomethyl-1H-tetrazol-5-ylthio, oder 1-Carboxymethyl-1H-tetrazol-5-ylthio, Thiadiazolylthio, z.B. 1,3,4-Thiadiazol-5-ylthio, oder durch Niederalkyl, z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxo-tetrahydro-as-triazinylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinium oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinium, z.B. 3- oder 4-Hydroxymethylpyridinium, 4-Carboxypyridinium, 3- oder 4-Carboxymethylpyridinium, 3- oder 4-Chlorpyridinium, 3- oder 4-Brompyridinium oder 3- oder 4-Carbamoylpyridinium bedeutet, $R_3$ Wasserstoff oder Methoxy und $R_4$

Carboxyl bedeuten, Hydrate und pharmazeutisch verwendbare Salze von solchen Verbindungen.

8. Verbindungen der Formel I gemäss Anspruch 6, worin m 0 (direkte Bindung) bedeutet, und worin $\ell$, n, X, W, Y, $R_1$, $R_2$, $R_3$ und $R_4$ die in Anspruch 7 genannten Bedeutungen haben, Hydrate und Salze von solchen Verbindungen.

9. 3-Acetoxymethyl-7α-methoxy-7β-[2-((2R)-2-amino-2-carboxyäthoxycarbonylamino)-acetylamino]-3-cephem-4-carbonsäure-natriumsalz gemäss Anspruch 7.

10. 3-(1-Methyl-1H-tetrazol-5-ylthiomethyl)-7α-methoxy-7β-[2-((2R)-2-amino-2-carboxyäthoxycarbonylamino)-acetylamino]-3-cephem-4-carbonsäurenatriumsalz gemäss Anspruch 7.

11. 3-(1-Methyl-1H-tetrazol-5-ylthiomethyl)-7β-[(2R,S)-2-((4R)-4-amino-4-carboxybutyramido)-2-(2-aminothiazol-4-yl)-acetamido]-3-cephem-4-carbonsäurenatriumsalz, gemäss Anspruch 7.

12. 3-Acetoxymethyl-7β-[(2R,S)-2-((2R)-2-amino-2-carboxyäthoxycarbonylamino)-2-(2-aminothiazol-4-yl)-acetamido]-3-cephem-4-carbonsäure-natriumsalz gemäss Anspruch 7.

13. 3-(1-Methyl-1H-tetrazol-5-ylthiomethyl)-7β-[(2R,S)-2-((2R)-2-amino-2-carboxyäthoxycarbonylamino)-2-(2-aminothiazol-4-yl)-acetamido]-3-cephem-4-carbonsäure-natriumsalz gemäss Anspruch 7.

14. 3-Acetoxymethyl-7β-[(2R)-2-((2R)-2-amino-2-carboxyäthoxycarbonylamino)-2-(2-aminothiazol-4-yl)-acetylamino]-3-cephem-4-carbonsäure-natriumsalz gemäss Anspruch 8.

15. 3-Acetoxymethyl-7β-[(2S)-2-((2R)-2-amino-2-carboxyäthoxycarbonyl-amino)-2-(2-aminothiazol-4-yl)-acetylamino]-3-cephem-4-carbonsäure-natriumsalz gemäss Anspruch 8.

16. 3-[1-(2-Dimethylaminoäthyl)-1H-tetrazol-5-ylthiomethyl]-7β-[(2R,S)-2-((2R)-2-amino-2-carboxyäthoxycarbonylamino)-2-(2-aminothiazol-4-yl)-acetylamino]-3-cephem-4-carbonsäure-natriumsalz gemäss Anspruch 8.

17. 3-(1-Carboxymethyl-1H-tetrazol-5-ylthiomethyl)-7β-[(2R,S)-2-((2R)-2-amino-2-carboxyäthoxycarbonylamino)-2-(2-aminothiazol-4-yl)-acetylamino]-3-cephem-4-carbonsäure-natriumsalz gemäss Anspruch 8.

18. 3-(1-Sulfomethyl-1H-tetrazol-5-ylthiomethyl)-7β-[(2R,S)-2-((2R)-2-amino-2-carboxyäthoxycarbonylamino)-2-(2-aminothiazol-4-yl)-acetylamino]-3-cephem-4-carbonsäure-natriumsalz gemäss Anspruch 8.

19. 3-(1-Methyl-1H-tetrazol-5-ylthiomethyl)-7β-[(2R,S)-2-((3R)-3-amino-3-carboxypropionylamino)-2-(2-aminothiazol-4-yl)-acetylamino]-3-cephem-4-carbonsäure-natriumsalz gemäss Anspruch 8.

20. 3-(1-Methyl-1H-tetrazol-5-ylthiomethyl)-7β-[(2R)-2-((2R)-2-amino-2-carboxyäthoxycarbonylamino)-2-(2-aminothiazol-4-yl)-acetylamino]-3-cephem-4-carbonsäure-natriumsalz gemäss Anspruch 8.

21. 3-(1-Methyl-1H-tetrazol-5-ylthiomethyl)-7β-[(2S)-2-((2R)-2-amino-2-carboxyäthoxycarbonylamino)-2-(2-aminothiazol-4-yl)-acetylamino]-3-cephem-4-carbonsäure-natriumsalz gemäss Anspruch 8.

22. 3-(4-Carbamoylpyridiniomethyl)-7β-[(2R,S)-2-((2R)-2-amino-2-carboxyäthoxycarbonylamino)-2-(2-aminothiazol-4-yl)-acetylamino]-3-cephem-4-carboxylat gemäss Anspruch 8.

23. 3-(1-Methyl-1H-tetrazol-5-ylthiomethyl)-7β-[4-((2R)-2-amino-2-carboxyäthoxycarbonylamino)-butyramido]-3-cephem-4-carbonsäure-natriumsalz gemäss Anspruch 8.

24. Pharmazeutische Präparate enthaltend Verbindungen der Formel I gemäss Anspruch 1, Hydrate oder pharmazeutisch verwendbare Salze von solchen Verbindungen.

25. Pharmazeutische Präparate enthaltend Verbindungen der Formel I gemäss Anspruch 2, Hydrate oder pharmazeutisch verwendbare Salze von solchen Verbindungen.

26. Verbindungen der Formel I gemäss Anspruch 1 zur Anwendung bei der Behandlung des menschlichen oder tierischen Körpers von bakteriellen Infektionen.

27. Verbindungen der Formel I gemäss Anspruch 2 zur Anwendung bei der Behandlung des menschlichen oder tierischen Körpers von bakteriellen Infektionen.

28. Verfahren zur Herstellung von Verbindungen der Formel I, worin $\ell$, m, n, X, W, Y, $R_1$, $R_2$, $R_3$ und $R_4$ die in Anspruch 1 genannten Bedeutungen haben und worin die Carboxylgruppen in freier Form vorliegen oder in physiologisch spaltbarer Form verestert sind, Hydraten und Salzen von solchen Verbindungen, dadurch gekennzeichnet, dass

a) in einer Verbindung der Formel

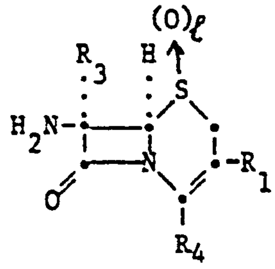

(II),

worin $\ell$, $R_1$, $R_3$ und $R_4$ die unter Formel I genannten Bedeutungen haben und die 7β-Aminogruppe gegebenenfalls durch eine die Acylierung erlaubende Gruppe geschützt ist, durch Umsetzung mit einer Carbonsäure der Formel

$$HOOC-\overset{NH_2}{\overset{|}{C}}H-(C_nH_{2n})-X-W-NH-(C_mH_{2m})-\overset{Y}{\overset{|}{C}}H-COOH \qquad (III),$$

worin m, n, X, W und Y die unter Formel I genannten Bedeutungen haben und die α-Aminocarbonsäuregruppierung $HOOC-CH(NH_2)-$ und in der Gruppe Y vorhandene funktionelle Gruppen in geschützter Form vorliegen, oder mit einem reaktionsfähigen funktionellen Säurederivat oder einem Salz davon acyliert, oder

b) in einer Verbindung der Formel

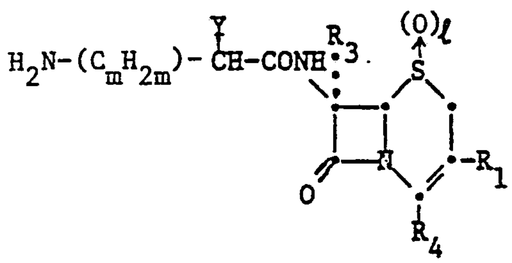

(IV),

worin $\ell$, m, Y, $R_1$ und $R_4$ die unter Formel I genannten Bedeutungen haben und die α-Aminogruppe gegebenenfalls durch eine die Acylierungsreaktion erlaubende Gruppe geschützt ist und in der Gruppe Y vorhandene funktionelle Gruppen gegebenenfalls in geschützter Form vorliegen, die Aminogruppe durch Umsetzung mit einem reaktionsfähigen funktionellen Derivat einer Säure der Formel

$$HOOC-\underset{NH_2}{\underset{|}{C}}H-(C_nH_{2n})-X-W-OH \qquad (V),$$

worin n, X und W die unter Formel I genannten Bedeutungen haben und die α-Aminocarbonsäuregruppierung $HOOC-CH(NH_2)-$ in geschützter Form vorliegt, oder, wenn X-W zusammen die Gruppe -CO- bedeuten, auch mit einer entsprechenden freien Säure oder mit einem Salz davon acyliert, oder

0059683



c) in einer Verbindung der Formel

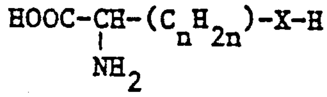 (VI),

worin n und X die unter Formel I genannten Bedeutungen haben und die α-Aminocarbonsäuregruppierung $HOOC-CH(NH_2)-$ in geschützter Form vorliegt, die Gruppe -X-H durch Umsetzung mit einem reaktionsfähigen funktionellen Derivat einer Säure der Formel

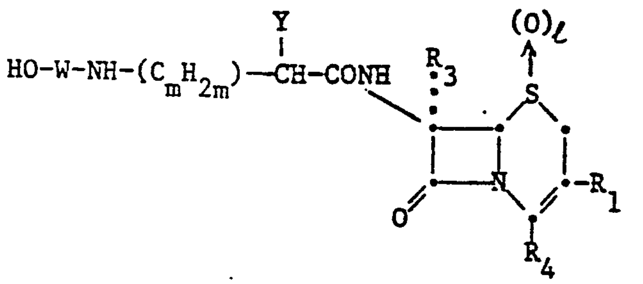 (VII),

worin $\ell$, m, W, Y, $R_1$, $R_3$ und $R_4$ die unter Formel I genannten Bedeutungen haben und in der Gruppe Y vorhandene funktionelle Gruppen gegebenenfalls in geschützter Form vorliegen, acyliert, oder

d) eine 2-Cephemverbindung der Formel

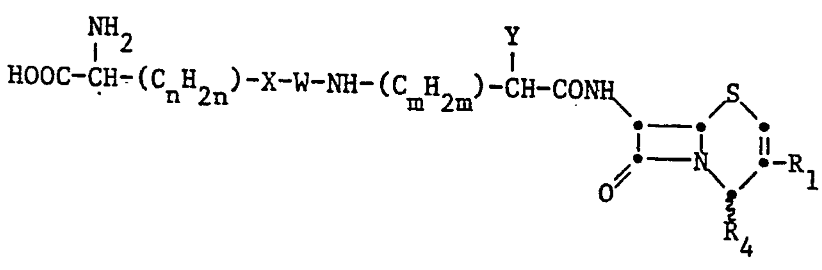 (VIII),

worin m, n, X, W, Y, $R_1$, $R_3$ und $R_4$ die unter Formel I genannten Bedeutungen haben und die Aminocarbonsäuregruppierung $HOOC-CH(NH_2)-$ und in der Gruppe Y vorhandene funktionelle Gruppen in geschützter Form vorliegen, zur entsprechenden 3-Cephemverbindung der Formel I isomerisiert und, wenn erwünscht, eine erfindungsgemäss erhältliche Verbindung der Formel I in eine andere Verbindung der Formel I umwandelt und/oder, wenn

erwünscht, eine erfindungsgemäss erhältliche Verbindung der Formel I, worin $\ell$ 0 bedeutet, in eine Verbindung der Formel I überführt, worin $\ell$ 1 oder 2 bedeutet, und/oder eine Verbindung der Formel I, worin $\ell$ 1 oder 2 bedeutet, in eine Verbindung der Formel I überführt, worin 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie Verbindung mit einer salzbildenden Gruppe in ein Salz überführt und/ oder ein erhältliches Gemisch von isomeren Verbindungen der Formel I in die einzelnen Isomeren auftrennt.

29. Verfahren zur Herstellung von Verbindungen der Formel I, worin m 0 (direkte Bindung) bedeutet und worin $\ell$, n, X, W, Y, $R_1$, $R_2$, $R_3$ und $R_4$ die in Anspruch 1 genannten Bedeutungen haben, und die Carboxylgruppen in freier Form vorliegen oder in physiologisch spaltbarer Form verestert sind, Hydraten und Salzen von solchen Verbindungen, dadurch gekennzeichnet, dass man

a) in einer Verbindung der Formel II, worin $\ell$, $R_1$, $R_3$ und $R_4$ die unter Formel I genannten Bedeutungen haben und die 7β-Aminogruppe gegebenenfalls durch eine die Acylierung erlaubende Gruppe geschützt ist, durch Umsetzung mit einer Carbonsäure der Formel III, worin die Indizes m und n und X, W und Y die unter Formel I genannten Bedeutungen haben und die α-Aminocarbonsäuregruppierung $HOOC-CH(NH_2)-$ und in der Gruppe Y vorhandene funktionelle Gruppen in geschützter Form vorliegen, oder mit einem reaktionsfähigen funktionellen Säurederivat oder einem Salz davon acyliert, oder

b) in einer Verbindung der Formel IV, worin m 0 (direkte Bindung) bedeutet, $\ell$, m, Y, $R_1$ und $R_4$ die unter Formel I genannten Bedeutungen haben und die α-Aminogruppe gegebenenfalls durch eine die Acylierungsreaktion erlaubende Gruppe geschützt ist und in

der Gruppe Y vorhandene funktionelle Gruppen gegebenenfalls in geschützter Form vorliegen, die Aminogruppe durch Umsetzung mit einem reaktionsfähigen funktionellen Derivat einer Säure der Formel (V), worin X und W die unter Formel I genannten Bedeutungen haben und die α-Aminocarbonsäuregruppierung HOOC-CH($NH_2$)- in geschützter Form vorliegt, oder, wenn X-W zusammen die Gruppe -CO- bedeuten, auch mit einer entsprechenden freien Säure oder mit einem Salz davon acyliert, oder

c) in einer Verbindung der Formel (VI), worin der Index n und X die unter Formel I genannten Bedeutungen haben und die α-Aminocarbonsäuregruppierung -X-H durch Umsetzung mit einem reaktionsfähigen funktionellen Derivat einer Säure der Formel VII, worin m 0 (direkte Bindung), $\ell$, m, W, Y, $R_1$, $R_3$ und $R_4$ die unter Formel I genannten Bedeutungen haben und in der Gruppe Y vorhandene funktionelle Gruppen gegebenenfalls in geschützter Form vorliegen, acyliert, oder

d) eine 2-Cephemverbindung der Formel VIII, worin m 0 (direkte Bindung) bedeutet und worin n, X, W, Y, $R_1$, $R_3$ und $R_4$ die Aminocarbonsäuregruppierung HOOC-CH($NH_2$)- und in der Gruppe Y vorhandene funktionelle Gruppen in geschützter Form vorliegen, zur entsprechenden 3-Cephemverbindung der Formel I isomerisiert und, wenn erwünscht, eine erfindungsgemäss erhältliche Verbindung der Formel I in eine andere Verbindung der Formel I umwandelt und/oder, wenn erwünscht, eine erfindungsgemäss erhältliche Verbindung der Formel I, worin $\ell$ 0 bedeutet, in eine Verbindung der Formel I überführt, worin $\ell$ 1 oder 2 bedeutet, und/oder eine Verbindung der Formel I, worin $\ell$ 1 oder 2 bedeutet, in eine Verbindung der Formel I überführt, worin $\ell$ 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie

Verbindung mit einer salzbildenden Gruppe in ein Salz überführt und/oder ein erhältliches Gemisch von isomeren Verbindungen der Formel I in die einzelnen Isomeren auftrennt.

30. Die nach dem Verfahren gemäss Anspruch 28 erhältlichen Verbindungen.

31. Die nach dem Verfahren gemäss Anspruch 29 erhältlichen Verbindungen.

32. Verbindungen der Formel

$$HOOC-\underset{|}{\overset{NH_2}{CH}}-(C_nH_{2n})-X-W-NH-(C_mH_{2m})-\underset{|}{\overset{Y}{CH}}-COOH \qquad (III),$$

worin m, n, X, W, und Y die in Anspruch 1 genannten Bedeutungen haben.

33. Verbindungen der Formel III, worin m 0 (direkte Bindung) bedeutet und worin m, n, X, W, Y, $R_1$, $R_2$, $R_3$ und $R_4$ die in Anspruch 1 genannten Bedeutungen haben.

34. Verfahren zur Herstellung von Verbindungen der Formel III, worin m, n, X, W und Y die in Anspruch 1 genannten Bedeutungen haben, und worin in der Gruppe Y vorhandene funktionelle Gruppen gegebenenfalls in geschützter Form vorliegen, dadurch gekennzeichnet, dass man eine Verbindung der Formel V, worin X und W die in Anspruch 1 unter Formel I genannten Bedeutungen haben und die α-Aminocarbonsäuregruppierung $HOOC-CH(NH_2)-$ in geschützter Form vorliegen, oder wenn X-W zusammen die Gruppe -CO- bedeuten, auch eine freie Säure oder ein reaktionsfähiges funktionelles Säurederivat oder ein Salz davon mit einer Säure der Formel

$$H_2N-(C_mH_{2m})-\underset{|}{\overset{Y}{CH}}-COOH \qquad (IX),$$

worin m und Y die in Anspruch 1 unter Formel I genannten Bedeutungen haben, und worin die Aminogruppe gegebenenfalls durch eine die Acylierungsreaktion erlaubende Gruppe geschützt ist und in der Gruppe Y vorhandene funktionelle Gruppe geschützt sind und die Carboxylgruppe intermediär geschützt ist, acyliert und, wenn erwünscht, eine erhältliche Verbindung der Formel III in eine andere Verbindung der Formel III mit entsprechend geschützten funktionellen Gruppen überführt.

35. Verfahren zur Herstellung von Verbindungen der Formel III, worin m 0 (direkte Bindung) und worin n, X, W und Y die in Anspruch 1 genannten Bedeutungen haben und in der Gruppe Y vorhandene funktionelle Gruppen gegebenenfalls in geschützter Form vorliegen, dadurch gekennzeichnet, dass man eine
Verbindung der Formel V, worin X und W die in Anspruch 1 unter Formel I genannten Bedeutungen haben und die α-Aminocarbonsäuregruppierung HOOC-CH($NH_2$)- in geschützter Form vorliegen, oder wenn X-W zusammen die Gruppe -CO- bedeuten, auch eine freie Säure oder ein reaktionsfähiges funktionelles Säurederivat oder ein Salz davon mit einer Säure der Formel

Y
|
$H_2$N-CH-COOH (IX')

worin Y die in Anspruch 1 unter Formel I genannte Bedeutung hat, und worin die Aminogruppe gegebenenfalls durch eine die Acylierungsreaktion erlaubende Gruppe geschützt ist und in der Gruppe Y vorhandene funktionelle Gruppe geschützt sind und die Carboxylgruppe intermediär geschützt ist, acyliert und, wenn erwünscht, eine erhältliche Verbindung der Formel III in eine andere Verbindung der Formel III mit entsprechend geschützten funktionellen Gruppen überführt.

Patentansprüche (für Oesterreich):

36. Verfahren zur Herstellung von Verbindungen der Formel I, worin $\ell$, m, n, X, W, Y, $R_1$, $R_2$, $R_3$ und $R_4$ die in Anspruch 1 genannten Bedeutungen haben, und worin die Carboxylgruppen in freier Form vorliegen oder in physiologisch spaltbarer Form verestert sind, Hydraten und Salzen von solchen Verbindungen, dadurch gekennzeichnet, dass man

a) in einer Verbindung der Formel II, worin $\ell$, $R_1$, $R_3$ und $R_4$ die unter Formel I genannten Bedeutungen haben und die 7β-Aminogruppe gegebenenfalls durch eine die Acylierung erlaubende Gruppe geschützt ist, durch Umsetzung mit einer Carbonsäure der Formel III, worin m, n, X, und Y die unter Formel I genannten Bedeutungen haben und die α-Aminocarbonsäuregruppierung $HOOC-CH(NH_2)-$ und in der Gruppe Y vorhandene funktionelle Gruppen in geschützter Form vorliegen, oder mit einem reaktionsfähigen funktionellen Säurederivat oder einem Salz davon acyliert, oder

b) in einer Verbindung der Formel IV, worin $\ell$, m, Y, $R_1$ und $R_4$ die unter Formel I genannten Bedeutungen haben und die α-Aminogruppe gegebenenfalls durch eine die Acylierungsreaktion erlaubende Gruppe geschützt ist und in der Gruppe Y vorhandene funktionelle Gruppen gegebenenfalls in geschützter Form vorliegen, die Aminogruppe durch Umsetzung mit einem reaktionsfähigen funktionellen Derivat einer Säure der Formel V, worin n, X und W die unter Formel I genannten Bedeutungen haben und die α-Aminocarbonsäuregruppierung $HOOC-CH(NH_2)-$ in geschützter Form vorliegt, oder, wenn X-W zusammen die Gruppe -CO- bedeuten, auch mit einer entsprechenden freien Säure oder mit einem Salz davon acyliert, oder

c) in einer Verbindung der Formel VI, worin n und X die unter Formel I genannten Bedeutungen haben und die α-Aminocarbonsäuregruppierung $HOOC-CH(NH_2)-$ in geschützter Form vorliegt, die Gruppe -X-H durch Umsetzung mit einem reaktionsfähigen funktionellen Derivat einer Säure der Formel VII, worin $\ell$, m, W, Y, $R_1$, $R_3$ und $R_4$ die unter Formel I genannten Bedeutungen haben und in der Gruppe Y vorhandene funktionelle Gruppen gegebenenfalls in geschützter Form vorliegen, acyliert, oder

d) eine 2-Cephemverbindung der Formel VIII, worin m, n, X, W, Y, $R_1$, $R_3$ und $R_4$ die unter Formel I genannten Bedeutungen haben und die Aminocarbonsäuregruppierung HOOC-CH($NH_2$)- und in der Gruppe Y vorhandene funktionelle Gruppen in geschützter Form vorliegen, zur entsprechenden 3-Cephemverbindung der Formel I isomerisiert und, wenn erwünscht, eine erfindungsgemäss erhältliche Verbindung der Formel I in eine andere Verbindung der Formel I umwandelt und/oder, wenn erwünscht, eine erfindungsgemäss erhältliche Verbindung der Formel I, worin $\ell$ 0 bedeutet, in eine Verbindung der Formel I überführt, worin $\ell$ 1 oder 2 bedeutet, und/oder eine Verbindung der Formel I, worin $\ell$ 1 oder 2 bedeutet, in eine Verbindung der Formel I überführt, worin $\ell$ 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie Verbindung mit einer salzbildenden Gruppe in ein Salz überführt und/oder ein erhältliches Gemisch von isomeren Verbindungen der Formel I in die einzelnen Isomeren auftrennt.

37. Verfahren zur Herstellung von Verbindungen der Formel I, worin m 0 (direkte Bindung) bedeutet und $\ell$, n, X, W, Y, $R_1$, $R_2$, $R_3$ und $R_4$ die in Anspruch 1 genannten Bedeutungen haben und die Carboxylgruppen in freier Form vorliegen oder in physiologisch spaltbarer Form verestert sind, Hydraten und Salzen von solchen Verbindungen, dadurch gekennzeichnet, dass man

a) in einer Verbindung der Formel II, worin $\ell$, $R_1$, $R_3$ und $R_4$ die unter Formel I genannten Bedeutungen haben und die 7β-Aminogruppe gegebenenfalls durch eine die Acylierung erlaubende Gruppe geschützt ist, durch Umsetzung mit einer Carbonsäure der Formel III, worin die Indizes m und n und X, W und Y die unter Formel I genannten Bedeutungen haben und die α-Aminocarbonsäuregruppierung $HOOC-CH(NH_2)-$ und in der Gruppe Y vorhandene funktionelle Gruppen in geschützter Form vorliegen, oder mit einem reaktionsfähigen funktionellen Säurederivat oder einem Salz davon acyliert, oder

b) in einer Verbindung der Formel IV, worin m 0 (direkte Bindung) bedeutet, $\ell$, m, Y, $R_1$ und $R_4$ die unter Formel I genannten Bedeutungen haben und die α-Aminogruppe gegebenenfalls durch eine die Acylierungsreaktion erlaubende Gruppe geschützt ist und in der Gruppe Y vorhandene funktionelle Gruppen gegebenenfalls in geschützter Form vorliegen, die Aminogruppe durch Umsetzung mit einem reaktionsfähigen funktionellen Derivat einer Säure der Formel (V), worin X und W die unter Formel I genannten Bedeutungen haben und die α-Aminocarbonsäuregruppierung $HOOC-CH(NH_2)-$ in geschützter Form vorliegt, oder, wenn X-W zusammen die Gruppe -CO- bedeuten, auch mit einer entsprechenden freien Säure oder mit einem Salz davon acyliert, oder

c) in einer Verbindung der Formel (VI), worin der Index n und X die unter Formel I genannten Bedeutungen haben und die α-Aminocarbonsäuregruppierung -X-H durch Umsetzung mit einem reaktionsfähigen funktionellen Derivat einer Säure der Formel VII, worin m 0 (direkte Bindung), $\ell$, m, W, Y, $R_1$, $R_3$ und $R_4$ die unter Formel I genannten Bedeutungen haben und in der Gruppe Y vorhandene funktionelle Gruppen gegebenenfalls in geschützter Form vorliegen, acyliert, oder

d) eine 2-Cephemverbindung der Formel VIII, worin m 0 (direkte Bindung) bedeutet und worin n, X, W, Y, $R_1$, $R_3$ und $R_4$ die

Aminocarbonsäuregruppierung HOOC-CH($NH_2$)- und in der Gruppe Y vorhandene funktionelle Gruppen in geschützter Form vorliegen, zur entsprechenden 3-Cephemverbindung der Formel I isomerisiert und, wenn erwünscht, eine erfindungsgemäss erhältliche Verbindung der Formel I in eine andere Verbindung der Formel I umwandelt und/oder, wenn erwünscht, eine erfindungsgemäss erhältliche Verbindung der Formel I, worin $\ell$ 0 bedeutet, in eine Verbindung der Formel I überführt, worin $\ell$ 1 oder 2 bedeutet, und/oder eine Verbindung der Formel I, worin $\ell$ 1 oder 2 bedeutet, in eine Verbindung der Formel I überführt, worin $\ell$ 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie Verbindung mit einer salzbildenden Gruppe in ein Salz überführt und/oder ein erhältliches Gemisch von isomeren Verbindungen der Formel I in die einzelnen Isomeren auftrennt.

38. Verfahren zur Herstellung von Verbindungen der Formel I gemäss Anspruch 36, worin $\ell$, m, n, X, W, Y, $R_1$, $R_2$, $R_3$ und $R_4$ die in Anspruch 3 genannten Bedeutungen haben, Hydraten und pharmazeutisch verwendbaren Salzen von solchen Verbindungen.

39. Verfahren zur Herstellung von Verbindungen der Formel I gemäss Anspruch 37, worin m 0 (direkte Bindung) bedeutet und worin $\ell$, n, X, W, Y, $R_1$, $R_2$, $R_3$ und $R_4$ die in Anspruch 3 genannten Bedeutungen haben, Hydraten und pharmazeutisch verwendbaren Salzen von solchen Verbindungen.

40. Verfahren zur Herstellung von Verbindungen der Formel I gemäss Anspruch 38, worin $\ell$, m, n, X, W, Y, $R_1$, $R_2$, $R_3$ und $R_4$ die in Anspruch 5 genannten Bedeutungen haben, Hydraten und Salzen von solchen Verbindungen.

41. Verfahren zur Herstellung von Verbindungen der Formel I gemäss Anspruch 39, worin m 0 (direkte Bindung) bedeutet und worin $\ell$, n, X, W, Y, $R_1$, $R_2$, $R_3$ und $R_4$ die in Anspruch 5 genannten Bedeutungen haben, Hydraten und Salzen von solchen Verbindungen.

42. Verfahren zur Herstellung von Verbindungen der Formel I gemäss Anspruch 40, worin $\ell$, m, n, X, W, Y, $R_1$, $R_2$, $R_3$ und $R_4$ die in Anspruch 7 genannten Bedeutungen haben, Hydraten und Salzen von solchen Verbindungen.

43. Verfahren zur Herstellung von Verbindungen der Formel I gemäss Anspruch 41, worin m 0 (direkt Bindung) bedeutet und $\ell$, n, X, W, Y, $R_1$, $R_2$, $R_3$ und $R_4$ die in Anspruch 7 genannten Bedeutungen haben, Hydraten und Salzen von solchen Verbindungen.

44. Verfahren zur Herstellung von 3-Acetoxymethyl-7α-methoxy-7β-[2-((2R)-2-amino-2-carboxyäthoxycarbonylamino)-acetylamino]-3-cephem-4-carbonsäure-natriumsalz gemäss Anspruch 42.

45. Verfahren zur Herstellung von 3-(1-Methyl-1H-tetrazol-5-ylthiomethyl)-7α-methoxy-7β-[2-((2R)-2-amino-2-carboxyäthoxycarbonylamino)-acetylamino]-3-cephem-4-carbonsäure-natriumsalz gemäss Anspruch 42.

46. Verfahren zur Herstellung von 3-(1-Methyl-1H-tetrazol-5-ylthiomethyl)-7β-[(2R,S)-2-((4R)-4-amino-4-carboxybutyramido)-2-(2-aminothiazol-4-yl)-acetamido]-3-cephem-4-carbonsäure-natriumsalz gemäss Anspruch 42.

47. Verfahren zur Herstellung von 3-Acetoxymethyl-7β-[(2R,S)-2-((2R)-2-amino-2-carboxyäthoxycarbonylamino)-2-(2-aminothiazol-4-yl)-acetamido]-3-cephem-4-carbonsäure-natriumsalz gemäss Anspruch 42.

48. Verfahren zur Herstellung von 3-(1-Methyl-1H-tetrazol-5-ylthiomethyl)-7β-[(2R,S)-2-((2R)-2-amino-2-carboxyäthoxycarbonylamino)-2-(2-aminothiazol-4-yl)-acetamido]-3-cephem-4-carbonsäure-natriumsalz gemäss Anspruch 42.

49. Verfahren zur Herstellung von 3-Acetoxymethyl-7β-[(2R)-2-((2R)-2-amino-2-carboxyäthoxycarbonylamino)-2-(2-aminothiazol-4-yl)-acetylamino]-3-cephem-4-carbonsäure-natriumsalz gemäss Anspruch 43.

50. Verfahren zur Herstellung von 3-Acetoxymethyl-7β-[(2S)-2-((2R)-2-amino-2-carboxyäthoxycarbonylamino)-2-(2-aminothiazol-4-yl)-acetylamino]-3-cephem-4-carbonsäure-natriumsalz gemäss Anspruch 43.

51. Verfahren zur Herstellung von 3-[1-(2-Dimethylaminoäthyl)-1H-tetrazol-5-ylthiomethyl]-7β-[(2R,S)-2-((2R)-2-amino-2-carboxyäthoxycarbonylamino)-2-(2-aminothiazol-4-yl)-acetylamino]-3-cephem-4-carbonsäure-natriumsalz gemäss Anspruch 43.

52. Verfahren zur Herstellung von 3-(1-Carboxymethyl-1H-tetrazol-5-ylthiomethyl)-7β-[(2R,S)-2-((2R)-2-amino-2-carboxyäthoxycarbonylamino)-2-(2-aminothiazol-4-yl)-acetylamino]-3-cephem-4-carbonsäure-natriumsalz gemäss Anspruch 43.

53. Verfahren zur Herstellung von 3-(1-Sulfomethyl-1H-tetrazol-5-ylthiomethyl)-7β-[(2R,S)-2-((2R)-2-amino-2-carboxyäthoxycarbonylamino)-2-(2-aminothiazol-4-yl)-acetylamino]-3-cephem-4-carbonsäure-natriumsalz gemäss Anspruch 43.

54. Verfahren zur Herstellung von 3-(1-Methyl-1H-tetrazol-5-ylthiomethyl)-7β-[(2R,S)-2-((3R)-3-amino-3-carboxypropionylamino)-2-(2-aminothiazol-4-yl)-acetylamino]-3-cephem-4-carbonsäure-natriumsalz gemäss Anspruch 43.

55. Verfahren zur Herstellung von 3-(1-Methyl-1H-tetrazol-5-ylthiomethyl)-7β-[(2R)-2-((2R)-2-amino-2-carboxyäthoxycarbonylamino)-2-(2-aminothiazol-4-yl)-acetylamino]-3-cephem-4-carbonsäure-natriumsalz gemäss Anspruch 43.

56. Verfahren zur Herstellung von 3-(1-Methyl-1H-tetrazol-5-ylthiomethyl)-7β-[(2S)-2-((2R)-2-amino-2-carboxyäthoxycarbonylamino)-2-(2-aminothiazol-4-yl)-acetylamino]-3-cephem-4-carbonsäure-natriumsalz gemäss Anspruch 43.

57. Verfahren zur Herstellung von 3-(4-Carbamoylpyridiniomethyl)-7β-[(2R,S)-2-((2R)-2-amino-2-carboxyäthoxycarbonylamino)-2-(2-aminothiazol-4-yl)-acetylamino]-3-cephem-4-carboxylat gemäss Anspruch 43.

58. Verfahren zur Herstellung von 3-(1-Methyl-1H-tetrazol-5-ylthiomethyl)-7α-methoxy-7β-[4-((2R)-2-amino-2-carboxyäthoxycarbonylamino)-butyramido]-3-cephem-4-carbonsäure-natriumsalz gemäss Anspruch 43.

59. Verfahren zur Herstellung von pharmazeutischen Präparaten enthaltend Verbindungen der Formel I gemäss Anspruch 36, Hydrate oder pharmazeutisch verwendbare Salze von solchen Verbindungen.

60. Verfahren zur Herstellung von pharmazeutischen Präparaten enthaltend Verbindungen der Formel I gemäss Anspruch 37, Hydrate oder pharmazeutisch verwendbare Salze von solchen Verbindungen.

61. Verfahren zur Herstellung von Verbindungen der Formel III, worin m, n, X, W und Y die in Anspruch 1 genannten Bedeutungen haben, und worin in der Gruppe Y vorhandene funktionelle Gruppen gegebenenfalls in geschützter Form vorliegen, dadurch gekennzeichnet, dass man eine

Verbindung der Formel V, worin X und W die in Anspruch 1 unter Formel I genannten Bedeutungen haben und die α-Aminocarbonsäuregruppierung $HOOC-CH(NH_2)-$ in geschützter Form vorliegen, oder wenn X-W zusammen die Gruppe -CO- bedeuten, auch eine freie Säure oder ein reaktionsfähiges funktionelles Säurederivat oder ein Salz davon mit einer Säure der Formel

$$H_2N-\overset{\overset{\displaystyle Y}{|}}{C}H-COOH \qquad (IX')$$

worin Y die in Anspruch 1 unter Formel I genannte Bedeutung hat, und worin die Aminogruppe gegebenenfalls durch eine die Acylierungsreaktion erlaubende Gruppe geschützt ist und in der Gruppe Y vorhandene funktionelle Gruppe geschützt sind und die Carboxylgruppe intermediär geschützt ist, acyliert und, wenn erwünscht, eine erhältliche Verbindung der Formel III in eine andere Verbindung der Formel III mit entsprechend geschützten funktionellen Gruppen überführt.

62. Verfahren zur Herstellung von Verbindungen der Formel III, worin m 0 (direkte Bindung) und worin n, X, W und Y die in Anspruch 1 genannten Bedeutungen haben und in der Gruppe Y vorhandene funktionelle Gruppen gegebenenfalls in geschützter Form vorliegen, dadurch gekennzeichnet, dass man eine

Verbindung der Formel V, worin X und W die in Anspruch 1 unter Formel I genannten Bedeutungen haben und die α-Aminocarbonsäuregruppierung $HOOC-CH(NH_2)-$ in geschützter Form vorliegen, oder wenn X-W zusammen die Gruppe -CO- bedeuten, auch eine freie Säure oder ein reaktionsfähiges funktionelles Säurederivat oder ein Salz davon mit einer Säure der Formel

$$H_2N-\overset{\overset{\displaystyle Y}{|}}{C}H-COOH \qquad (IX')$$

worin Y die in Anspruch 1 unter Formel I genannte Bedeutung hat, und worin die Aminogruppe gegebenenfalls durch eine die Acylierungsreaktion erlaubende Gruppe geschützt ist und in der Gruppe Y vorhandene funktionelle Gruppe geschützt sind und die Carboxylgruppe intermediär geschützt ist, acyliert und, wenn erwünscht, eine erhältliche Verbindung der Formel III in eine andere Verbindung der Formel III mit entsprechend geschützten funktionellen Gruppen überführt.